# EUROPEAN PATENT SPECIFICATION

(11) **EP 1 899 377 B1**
(45) Date of publication and mention of the grant of the patent: **22.08.2012**
(21) Application number: 06776123.9
(22) Date of filing: 03.07.2006
(51) Int. Cl.: C07K 16/22, A61K 39/395, A61P 25/28, A61P 19/10

(54) **HUMANISED ANTIBODIES SPECIFIC FOR NOGO-A AND PHRARMACEUTICAL USES THEREOF**
FÜR NOGO-A SPEZIFISCHE HUMANISIERTE ANTIKÖRPER UND IHRE PHARMAZEUTISCHE VERWENDUNG
ANTICORPS HUMANISES CONTRE NOGO-A ET LEUR UTILISATIONS PHARMACEUTIQUES

(30) Priority: 05.07.2005 GB 0513766; 14.12.2005 GB 0525448
(43) Date of publication of application: 19.03.2008
(73) Proprietor: GLAXO GROUP LIMITED, Greenford, Middlesex UB6 0NN (GB)
(72) Inventor: ELLIS, Jonathan Henry, GlaxoSmithKline, Stevenage, Hertfordshire SG1 2NY (GB); HAMBLIN, Paul Andrew, GlaxoSmithKline, Stevenage, Hertfordshire SG1 2NY (GB); HUSSAIN, Farhana, GlaxoSmithKline, Stevenage, Hertfordshire SG1 2NY (GB); LEWIS, Alan Peter, GlaxoSmithKline, Stevenage, Hertfordshire SG1 2NY (GB); MCADAM, Ruth, GlaxoSmithKline, Stevenage, Hertfordshire SG1 2NY (GB); PRINJHA, Rabinder, GlaxoSmithKline, Third Avenue, Harlow, Essex CM19 5AW (GB); WILSON, Paul, GlaxoSmithKline, Stevenage, Hertfordshire SG1 2NY (GB)
(74) Representative: Sayce, Alastair George
(86) International application number: PCT/EP2006/006535
(87) International publication number: WO 2007/003421

(56) References cited:
- WO-A-2004/052932
- WO-A-2005/028508
- WO-A-2005/061544
- WO-A-2005/061545
- PAPADOPOULOS C M ET AL: "FUNCTIONAL RECOVERY ANF NEUROANATOMICAL PLASTICITY FOLLOWING MIDDLE CEREBRAL ARTERY OCCLUSION AND IN-1 ANTIBODY TREATMENT IN THE ADULT RAT" ANNALS OF NEUROLOGY, BOSTON, US, vol. 51, no. 4, April 2002 (2002-04), pages 433-441, XP008034394 ISSN: 0364-5134
- BAREYRE F M ET AL: "Long-lasting sprouting and gene expression changes induced by the monoclonal antibody IN-1 in the adult spinal cord" JOURNAL OF NEUROSCIENCE, NEW YORK, NY, US, vol. 22, no. 16, 15 August 2002 (2002-08-15), pages 7097-7110, XP002330320 ISSN: 0270-6474
- WIESSNER C ET AL: "ANTI-NOGO-A ANTIBODY INFUSION 24 HOURS AFTER EXPERIMENTAL STROKE IMPROVED BEHAVIORAL OUTCOME AND CORTICOSPINAL PLASTICITY IN NORMOTENSIVE AND SPONTANEOUSLY HYPERTENSIVE RATS" JOURNAL OF CEREBRAL BLOOD FLOW AND METABOLISM, RAVEN PRESS, LTD., NEW YORK, NY, US, vol. 23, no. 2, February 2003 (2003-02), pages 154-165, XP008047583 ISSN: 0271-678X
- BROESAMLE C ET AL: "Regeneration of lesioned corticospinal tract fibers in the adult rat induced by a recombinant, humanized IN-1 antibody fragment" JOURNAL OF NEUROSCIENCE, NEW YORK, NY, US, vol. 20, no. 21, 1 November 2000 (2000-11-01), pages 8061-8068, XP002238449 ISSN: 0270-6474

## Description

### Field of the Invention

The present invention relates to immunoglobulins, particularly antibodies that bind to NOGO and neutralise the activity thereof, polynucleotides encoding such antibodies, pharmaceutical formulations containing said antibodies and to the use of such antibodies in the treatment and/or prophylaxis of neurological diseases.

### Background of the Invention

Stroke is a major cause of death and disability in the Western World. There is no approved therapy for the treatment of stroke other than tissue plasminogen (t-PA) which has to be administered within 3 hours of onset following a computer tomography (CT) scan to exclude haemorrhage. To date most therapeutic agents directed towards the treatment of acute stroke (i.e. neuroprotection), have predominantly involved targeting glutamate receptors and their down stream signalling pathways known to be involved in acute cell death. However to date these strategies have proved unsuccessful in clinical trials and are often associated with dose-limiting side effects (Hill & Hachinski, The Lancet, 352 : (suppl III) 10-14 (1998)). Therefore there is a need for novel approaches directed towards the amelioration of cell death following the cessation of blood flow. Neuroprotection is the ability of a treatment to prevent or ameliorate neuronal cell loss in response to an insult or disease process. This maybe achieved by targeting the neurons directly or indirectly by preventing glial (including oligodendrocyte) cell loss.

Following the onset of stroke, some degree of spontaneous functional recovery is observed in many patients, suggesting that the brain has the (albeit limited) ability to repair and/or remodel following injury. Agents that have the potential to enhance this recovery may therefore allow intervention to be made much later (potentially days) following the onset of cerebral ischaemia. Agents which are able to offer both acute neuroprotection and enhance functional recovery may provide significant advantages over current potential neuroprotective strategies.

The mechanisms underlying functional recovery are currently unknown. The sprouting of injured or non-injured axons has been proposed as one possible mechanism. However, although in vivo studies have shown that treatment of spinal cord injury or stroke with neurotrophic factors results in enhanced functional recovery and a degree of axonal sprouting, these do not prove a direct link between the degree of axonal sprouting and extent of functional recovery (Jakeman, et al. 1998, Exp. Neurol. 154: 170-184, Kawamata et al. 1997, Proc Natl Acad. Sci. USA., 94:8179-8184, Ribotta, et al. 2000, J Neurosci. 20: 5144-5152). Furthermore, axonal sprouting requires a viable neuron. In diseases such as stroke which is associated with extensive cell death, enhancement of functional recovery offered by a given agent post stroke may therefore be through mechanisms other than axonal sprouting such as differentiation of endogenous stem cells, activation of redundant pathways, changes in receptor distribution or excitability of neurons or glia (Fawcett & Asher, 1999, Brain Res. Bulletin, 49: 377-391, Homer & Gage, 2000, Nature 407 963-970).

The limited ability of the central nervous system (CNS) to repair following injury may be due to molecules within the CNS environment that have an inhibitory effect on axonal sprouting (neurite outgrowth) such as the myelin protein NOGO (Sato S. et al (1989) Biochem. Biophys. Res. Comm.163,1473-1480; McKerracher L et al (1994) Neuron 13, 805-811; Mukhopadhyay G et al (1994) Neuron 13, 757-767; Torigoe K and Lundborg G (1997) Exp. Neurology 150, 254-262; Schafer et al (1996) Neuron 16, 1107-1113; WO9522344; WO9701352; Prinjha R et al (2000) Nature 403, 383-384; Chen MS et al (2000) Nature 403, 434-439; GrandPre T et al (2000) Nature 403, 439-444; US005250414A; WO200005364A1; WO0031235).

Three forms of human NOGO have been identified: NOGO-A having 1192 amino acid residues (GenBank accession no. AJ251383); NOGO-B, a splice variant which lacks residues 186 to 1004 in the putative extracellular domain (GenBank accession no. AJ251384) and a shorter splice variant, NOGO-C, which also lacks residues 186 to 1004 and also has smaller, alternative amino terminal domain (GenBank accession no. AJ251385) (Prinjha et al (2000) supra).

Inhibition of the CNS inhibitory proteins such as NOGO may provide a therapeutic means to ameliorate neuronal damage and promote neuronal repair and growth thereby potentially assisting recovery from neuronal injury such as that sustained in stroke. Examples of such NOGO inhibitors may include small molecules, peptides and antibodies.

Antibodies typically comprise two heavy chains linked together by disulphide bonds and two light chains. Each light chain is linked to a respective heavy chain by disulphide bonds. Each heavy chain has at one end a variable domain followed by a number of constant domains. Each light chain has a variable domain at one end and a constant domain at its other end. The light chain variable domain is aligned with the variable domain of the heavy chain. The light chain constant domain is aligned with the first constant domain of the heavy chain. The constant domains in the light and heavy chains are not involved directly in binding the antibody to antigen.

The variable domains of each pair of light and heavy chains form the antigen binding site. The variable domains on the light and heavy chains have the same general structure and each domain comprises four framework regions, whose sequences are relatively conserved, connected by three complementarity determining regions (CDRs) often referred to as hypervariable regions. The four framework regions largely adopt a beta-sheet conformation and the CDRs form loops connecting, and in some cases forming part of, the beta-sheet structure. The CDRs are held in close proximity by the framework regions and, with the CDRs from the other domain, contribute to the formation of the antigen binding site. CDRs and framework regions of antibodies may be determined by reference to Kabat et al ("Sequences of proteins of immunological interest" US Dept. of Health and Human Services, US Government Printing Office, 1987).

It has been reported that a murine monoclonal antibody, IN-1, that was raised against NI-220/250, a myelin protein which is a potent inhibitor of neurite growth (and subsequently shown to be fragment of NOGO-A), promotes axonal regeneration (Caroni, P and Schwab, ME (1988) Neuron 1 85-96; Schnell, L and Schwab, ME (1990) Nature 343 269-272; Bregman, BS et al (1995) Nature 378 498-501 and Thallmair, M et al (1998) Nature Neuroscience 1 124-131). It has also been reported that NOGO-A is the target for IN-1 (Chen et al (2000) Nature 403 434-439). Administration of IN-1 Fab fragment or humanised IN-1 to rats that have undergone spinal cord transection, enhanced recovery (Fiedler, M et al (2002) Protein Eng 15 931-941; Brosamle, C et al (2000) J. Neuroscience 20 8061-8068).

Monoclonal antibodies which bind to NOGO are described in WO 04/052932 and WO2005/028508. WO 04/052932 discloses a murine antibody 11C7 which binds to certain forms of human NOGO with high affinity.

It is desirable to isolate and develop further therapeutically useful monoclonal antibodies that bind to, and inhibit the activity of, human NOGO. The process of neurodegeneration underlies many neurological diseases/disorders including, but not limited to, acute diseases such as stroke (ischemic or haemorrhagic), traumatic brain injury and spinal cord injury as well as chronic diseases including Alzheimer's disease, fronto-temporal dementias (tauopathies), peripheral neuropathy, Parkinson's disease, Creutzfeldt-Jakob disease (CJD), Schizophrenia, amyotrophic lateral sclerosis (ALS), multiple sclerosis, Huntington's disease, multiple sclerosis and inclusion body myositis. Consequently an anti-NOGO monoclonal antibody may be useful in the treatment of these diseases/disorders. Such antibodies for the treatment of the above mentioned disease/disorders are provided by the present invention and described in detail below.

WO 2005/061544 discloses high affinity monoclonal antibodies, including a murine monoclonal antibody 2A10, and a humanised variant thereof H1L11.

### Summary of the Invention

The present invention provides a number of monoclonal antibodies that bind to human NOGO. The present application gives reference to many SEQ ID numbers, which are summarised in Table 12, with the actual sequences following that table towards the end of this document.

The murine antibody 2A10 binds to human NOGO with high affinity, and binds to the form of NOGO which is expressed by human cell lines with high affinity. 2A10 has been humanised successfully in the past (H1L11 which comprises the heavy chain variable region H1 (SEQ ID NO. 77) and the light chain variable region L11 (SEQ ID NO. 78)). The present invention provides additional humanised monoclonal antibodies which retain a high proportion of the affinity of, or have equivalent affinity to, the donor antibody (2A10) to human NOGO. In particular the antibodies of the present invention have a high degree of binding to human NOGO both in recombinant form as expressed in bacterial cells (such as E.Coli), and also in the form that it is expressed by a human neuroblastoma cell line (for example the human neuroblastoma cell line IMR32).

For the purposes of providing humanised variants of 2A10, the present inventors have identified a number of key amino acid residues in the framework sequence of the 2A10 variable regions which are believed to be important in optimal retention of binding affinity to human NOGO. The 2A10 heavy chain variable region (VH) is provided in SEQ ID NO. 7; the 2A10 light chain variable region (VL) is provided in SEQ ID NO. 8. Chimeric heavy and light chains comprising the murine variable regions and human constant regions are provided in SEQ ID NOs 9 and 10 respectively (the combination of the two chimeric chains is termed HcLc). The skilled reader will understand that SEQ ID NOs 9 and 10 represent the heavy chain or light chains prior to any processing (e.g. host cell mediated processing) for removal of a signal sequence. Typically the processed forms of the antibody chains will begin at position 20 (after the removal of the signal sequence) for SEQ ID NO.9; and will begin at position 21 for SEQ ID NO. 10. Within the context of the present invention all full length heavy and light chain sequences will be provided with the signal sequence included (which for all other full length sequences, which are not SEQ ID NOs 9 and 10, corresponds to SEQ ID NO.75). It will be apparent to the skilled man that the antibody produced by a cell line transfected with a gene encoding the any of these full length heavy and light chains, should not comprise this signal sequence.

**Table 1, CDRs of the 2A10 heavy chain are:**

| CDR | *According to Kabat* |
|---|---|
| H1 | SYWMH (SEQ ID NO:1) |
| H2 | NINPSNGGTNYNEKFKS (SEQ ID NO:2) |
| H3 | GQGY (SEQ ID NO:3) |

**Table 2, CDRs of 2A10 light chain:**

| CDR | According to Kabat |
|---|---|
| L1 | RSSKSLLYKDGKTYLN (SEQ ID NO:4) |
| L2 | LMSTRAS (SEQ ID NO:5) |
| L3 | QQLVEYPLT (SEQ ID NO:6) |

The CDRs were identified according to Kabat (Kabat et al. (1991) "Sequences of proteins of immunological interest"; Fifth Edition; US Department of Health and Human Services; NIH publication No 91-3242). CDRs preferably are as defined by Kabat but following the principles of protein structure and folding as defined by Chothia and Lesk, (Chothia et al., (1989) "Conformations of immunoglobulin hypervariable regions"; Nature 342, p877-883) it will be appreciated that additional residues may also be considered to be part of the antigen binding region and are thus encompassed by the present invention.

The VH and VL domains H1 and L11 have been previously described in WO 2005/061544 and are a result of the CDRs mentioned in tables 1 and 2 being grafted into human variable regions with high homology to the 2A10 donor antibody, each grafted construct further comprising back mutations in kabat positions 93 and 94 (for the H1 VH) or 4, 45 and 46 (for L11 VL).

The 2A10 antibody is capable of binding to human NOGO, and also binds to Marmoset and Rat NOGO, and it is believed that the new humanised antibodies of the present invention will retain that property. For example a monoclonal antibody comprising the H20 and L16 variable regions (see below for details) binds to Marmoset NOGO as well as Rat, Cynomolgus and Squirrel monkey NOGO. The sequence of Marmoset NOGO-A fragment is given in SEQ ID NO. 113.

Antibodies comprising the CDRs in Table 1 and 2 are provided by the present invention. Also encompassed within the present invention are antibodies comprising analogs of these CDRs.

In a first aspect, the invention provides as humanised antibody as claimed in claim 1.

Unless specifically stated otherwise, when a numerical position of an amino acid residue found within a specific sequence is mentioned in this document, for example "position 12", it is intended that the skilled reader assigns the first amino acid in the sequence the position "1" and counts from position one and identifies the amino acid which is in the desired position, in this example the twelfth amino acid residue in the sequence. The skilled reader will notice that this numbering system does not correspond with the Kabat numbering system which is often used for amino acid positions within antibody sequences. The following table (Table 3) illustrates the substitutions/back-mutations of the present invention and gives their numerical positions and the Kabat number for that numerical position:

**Table 3,**

| Human framework residue of H1 (SEQ ID NO 77) | Numerical Position | Kabat number of that numerical position | Corresponding residue in murine 2A10 (SEQ ID NO 7) |
|---|---|---|---|
| K | 12 | 12 | V |
| V | 20 | 20 | L |
| R | 38 | 38 | K |
| A | 40 | 40 | R |
| M | 48 | 48 | I |
| R | 67 | 66 | K |
| V | 68 | 67 | A |
| M | 70 | 69 | L |
| R | 72 | 71 | V |
| T | 74 | 73 | K |
| T | 76 | 75 | S |
| V | 79 | 78 | A |
| T | 91 | 87 | S |

The following table includes details of 20 different heavy chain variable (VH) regions based on the H1 VH (SEQ ID NO. 77) further comprising the substitutions mentioned in the table (Table 4) where the H1 residue at the relevant position is substituted with the 2A10 residue at that position (in the table, "-" means that there is no substitution in that position, and so the residue remains as in the sequence of H1):

**Table 4,**

| New VH (SEQ ID NO. X) | Numerical Residue No. | 12 | 20 | 38 | 40 | 48 | 67 | 68 | 70 | 72 | 74 | 76 | 79 | 91 |
|---|---|---|---|---|---|---|---|---|---|---|---|---|---|---|
| | KabatNo. | 12 | 20 | 38 | 40 | 48 | 66 | 67 | 69 | 71 | 73 | 75 | 78 | 87 |
| | *2A10* | *V* | *L* | *K* | *R* | *I* | *K* | *A* | *L* | *V* | *K* | *S* | *A* | *S* |
| | *H1* | *K* | *V* | *R* | *A* | *M* | *R* | *V* | M | *M* | *T* | *T* | *V* | *T* |
| H5 (11) | | - | - | - | - | - | - | - | - | - | - | - | A | - |
| H6 (12) | | - | - | - | - | I | - | A | - | - | - | - | A | - |
| H700 (13) | | - | - | - | | I | | A | L | V | K | - | - | - |
| H8 | | - | | - | - | I | - | A | L | V | - | - | A | - |
| H9 | | - | - | | - | I | - | A | L | V | K | - | A | - |
| H10 | | - | - | K | - | I | - | A | L | V | K | A | A | - |
| H12 | | - | - | - | - | I | - | A | - | - | K | - | - | - |
| H13 | | - | - | - | - | I | - | A | L | V | - | K | - | - |
| H14 (14) | | V | L | K | - | I | - | A | L | - | - | - | A | - |
| H15 (15) | | V | L | K | R | I | K | A | L | V - | - | S | A | S |
| H16 (16) | | - | - | K | R | I | K | A | L | V | K | - | A | - |
| H17 (17) | | V | L | K | R | I | K | A | L | V | K | - | A | - |
| H18 (18) | | V | L | K | R | I | K | A | L | V | K | S | A | S |
| H19 (85) | | - | - | - | R | I | - | A | - | - | - | - | A | - |
| H20 (86) | | - | - | - | - | I | K | - | - | - | - | | A | - |
| H21 (87) | | - | - | - | R | I | K | A | - | - | - | - | A | - |
| H22 (88) | | - | - | K | R | I | K | A | - | - | - | - | A | - |
| H23 (89 | | - | - | K | R | I | K | A | - | V | - | - | - | - |
| H24 (90) | | - | - | K | R | I | K | A | L | V | - | - | - | - |
| H25 (91) | | - | - | - | R | - | - | - | - | - | - | - | A | - |

The following table (Table 5) illustrates light chain substitutions/back-mutations and gives their numerical positions and the Kabat number for that numerical position:

**Table 5,**

| Human framework residue of L13 (SEQ ID NO 20) | Numerical Position | Kabat number | Corresponding residue in murine 2A10 (SEQ ID NO 8) |
|---|---|---|---|
| M | 4 | 4 | I |
| S | 7 | 7 | D |
| L | 11 | 11 | N |
| A | 19 | 19 | V |
| Q | 42 | 37 | L |
| P | 64 | 59 | S |
| G | 70 | 65 | S |

The following table (Table 6) includes details of seven different light chain variable (VL) regions based on, the L13 VL (SEQ ID NO. 20) optionally further comprising the substitutions mentioned in the table where the L13 residue at the relevant position is substituted with the 2A10 residue at that position (in the table, "-" means that there is no substitution in that position, and so the residue remains as in the sequence of L13):

**Table 6,**

| New VL (SEQ ID NO.X) | Numerical Residue No. | 4 | 7 | 11 | 19 | 42 | 64 | 70 |
|---|---|---|---|---|---|---|---|---|
| | Kabat No. | 4 | 7 | 11 | 19 | 37 | 59 | 65 |
| | *210* | *I* | *D* | *N* | *V* | *L* | *S* | *S* |
| | *L13* | *M* | *S* | *L* | *A* | *Q* | *P* | *G* |
| L11 (78) | | I | - | - | - | - | - | - |
| L13 (20) | | - | - | - | - | - | - | - |
| L14 (21) | | - | - | - | - | L | - | - |
| L15 (22) | | - | - | - | - | L | S | - |
| L16 (23) | | - | - | N | V | L | - | - |
| L17 (24) | | - | D | - | - | - | S | S |
| L18 (25) | | - | D | N | V | L | S | S |

Particular embodiments are antibodies comprising the following combinations of heavy and light chain variable regions: H20L16 (SEQ ID 86 + SEQ ID 23), H22L16 (SEQ ID 88 + SEQ ID 23), H23L16 (SEQ ID 89 + SEQ ID 23), H24L16 (SEQ ID 90 + SEQ ID 23), H20L18 (SEQ ID 86 + SEQ ID 25), H22L18 (SEQ ID 88 + SEQ ID 25), H23L18 (SEQ ID 89 + SEQ ID 25), H24L18 (SEQ ID 90 + SEQ ID 25). In one embodiment the antibody comprises H20L16 or variant thereof comprising an analog of the naturally occurring CDRs.

The humanised antibodies of the present invention bind to human NOGO with a comparable affinity to that of the murine donor antibody 2A10 or chimeric version thereof (HcLc). In one embodiment the binding of the antibody of the present invention to NOGO has an affinity constant (KD, as measured by BiaCore techniques) within 10 fold of 2A10, and in another embodiment the affinity constant is within three or two fold of 2A10, in another embodiment the affinity constant is not different from 2A10 (or 2A10 HcLc). In another embodiment the affinity constant is within three or two fold of that of 2A10 and the off-rate (kd) is within 10 fold, or three fold, or two fold of 2A10, in another embodiment the off-rate is not different from 2A10 (or 2A10 HcLc). The method of measuring the affinity constant and the off-rate of the antibody should be clear to the skilled reader, however the kinetic analysis BiaCore method given in Example 5 of this document is illustrative in this regard. For example, the affinity constant and the off-rate of 2A10 as measured by BiaCore kinetic analysis is commonly in the region of 1nM and 1.84 x10⁻³ (kd 1/s) respectively; in the same assay the antibodies of one embodiment of the present invention will have an affinity constant of less than 8-10nM and 1.84x10⁻². In a similar manner the analysis of the chimeric 2A10 antibody, HcLc, will have an affinity constant of around 1 to 2nM and 2 x10⁻³ to 4 x10⁻³ (kd 1/s) respectively

In typical embodiments, the antibodies of the invention are of the IgG class, more typically human IgG1 or IgG4, with a κ type human light chain.

A further aspect of the invention provides a pharmaceutical composition comprising an anti-NOGO antibody of the present invention or functional fragment thereof together with a pharmaceutically acceptable diluent or carrier. The antibodies of the present invention may be used in a method of treatment or prophylaxis of stroke (particularly ischemic stroke) and other neurological diseases, in particular Alzheimer's disease or Multiple sclerosis, in a human which comprises administering to said human in need thereof an effective amount of an anti-NOGO antibody of the invention or functional fragments thereof.

The antibodies of the present invention may be used in a method of inhibiting neurodegeneration and/or promoting functional recovery in a human patient afflicted with, or at risk of developing, a stroke (particularly ischemic stroke) or other neurological disease, in particular Alzheimer's disease or Multiple sclerosis, which comprises administering to said human in need thereof an effective amount of an anti-NOGO antibody of the invention or a functional fragment thereof.

The antibodies of the present invention may be used in a method of treatment, or in the manufacture of a medicament for the treatment, of traumatic nerve injury, such as spinal chord injury or other traumatic injury to the nervous system.

Other aspects and advantages of the present invention are described further in the detailed description and the preferred embodiments thereof.

Full length antibodies described herein include those comprising the light chains of SEQ ID NOs 34-40 and the heavy chains of SEQ ID NOs 92-98. It will be apparent to those skilled in the art that all of the sequences given for the full length antibody chains in Table 7 and 12 (and appended sequences) represent the heavy chain or light chains prior to any processing (e.g. host cell mediated processing) for removal of a signal sequence. Typically the processed forms of the antibody chains will begin at position 20 (after the removal of the signal sequence (residues 1 - 19) which corresponds to SEQ ID NO. 75). The present invention provides the antibodies having the polypeptide sequences listed (after removal of the first 19 amino acids of the signal sequence), and also provides the antibodies in the form in which they are produced and purified from host cells expressing the polynucleotides encoding the heavy and light chain.

**Table 7, Specific full length antibodies include:**

| Antibody | Light Chain | Heavy Chain |
|---|---|---|
| H20L16 FL | SEQ ID NO. 38 | SEQ ID NO. 93 |
| H20L18 FL | SEQ ID NO. 40 | SEQ ID NO. 93 |

In one embodiment the antibody is H20L16 FL, or variant thereof comprising a CDR analog.

In one embodiment of the present invention the antibodies are not lytic, in that the constant region are not capable of binding to complement and/or mediating ADCC (antibody dependent cell cytotoxicity). The constant regions of the full length antibodies are therefore either based on constant domains that are naturally non lytic, such as human IgG4, or comprise inactivating substitutions at positions 235 and 237 within a human IgG1 constant region (see EP0307434 for more details).

### Description of the Figures

Figure 1A and B, ELISA data for monoclonal antibody supernatants binding to recombinant human NOGO.
Figure 2A and B, ELISA data for purified monoclonal antibody binding to recombinant human NOGO.
Figure 3A and B, ELISA data for monoclonal antibody supernatants binding to recombinant human NOGO.
Figure 4A and B, ELISA data for monoclonal antibody supernatants binding to recombinant human NOGO.
Figure 5A and B, ELISA data for monoclonal antibody supernatants binding to recombinant human NOGO.
Figure 6, A to F, FACS data for purified antibody binding to human NOGO expressed by human neuroblastoma cells.
Figure 7, Competition ELISA results
Figure 8, ELISA data for monoclonal antibodies binding to recombinant human NOGO.
Figure 9, ELISA data for monoclonal antibodies binding to recombinant human NOGO.
Figure 10, ELISA data for monoclonal antibodies binding to recombinant human NOGO.
Figure 11, ELISA data for monoclonal antibodies binding to recombinant human NOGO.
Figure 12A and B, ELISA data for monoclonal antibodies binding to recombinant human NOGO.
Figure 13, FACS data for purified antibody binding to human NOGO expressed by human neuroblastoma cells.
Figure 14A to C, FACS data for purified antibody binding to human NOGO expressed by human neuroblastoma cells.

### Detailed Description of the Invention

Antibodies of the invention typically have the structure of a natural antibody or functional fragment thereof. The antibody may therefore comprise a full length antibody, a (Fab)₂ fragment, a Fab fragment, a light chain dimer or a heavy chain dimer. The antibody may be an IgG1, IgG2, IgG3, or IgG4; or IgM; IgA, IgE or IgD or a modified variant thereof. The constant domain of the antibody heavy chain may be selected accordingly. The light chain constant domain may be a kappa or lambda constant domain. Furthermore, the antibody may comprise modifications of all classes eg. IgG dimers, Fc mutants that no longer bind Fc receptors or mediate Clq binding. The antibody may also be a chimeric antibody of the type described in WO86/01533 which comprises an antigen binding region and a non-immunoglobulin region. The antigen binding region is an antibody light chain variable domain or heavy chain variable domain. Typically the antigen binding region comprises both light and heavy chain variable domains. The non-immunoglobulin region is fused at its C terminus to the antigen binding region. The non-immunoglobulin region is typically a non-immunoglobulin protein and may be an enzyme, a toxin or protein having known binding specificity. The two regions of this type of chimeric antibody may be connected via a cleavable linker sequence. Immunoadhesins having the CDRs as hereinbefore described are also contemplated in the present invention.

The constant region is selected according to the functionality required. Normally an IgG1 will demonstrate lytic ability through binding to complement and/or will mediate ADCC (antibody dependent cell cytotoxicity). An IgG4 will be preferred if a non-cytotoxic blocking antibody is required. However, IgG4 antibodies can demonstrate instability in production and therefore it may be more preferable to modify the generally more stable IgG1. Suggested modifications are described in EP0307434 preferred modifications include at positions 235 and 237. The invention therefore provides a lytic or a non-lytic form of an antibody according to the invention.

In one embodiment the antibody of the invention is a full length (i.e. a tetramer comprising two heavy and two light chains) non-lytic IgG1 antibody having the VH or VL sequences described supra.

"NOGO" refers to any NOGO polypeptide, including variant forms. This includes, but is not limited to, NOGO-A having 1192 amino acid residues (GenBank accession no. AJ251383); NOGO-B, a splice variant which lacks residues 186 to 1004 in the putative extracellular domain (GenBank accession no. AJ251384) and a shorter splice variant, NOGO-C, which also lacks residues 186 to 1004 and also has smaller, alternative amino terminal domain (GenBank accession no. AJ251385) (Prinjha et al (2000) supra). All references to "NOGO" herein is understood to include any and all variant forms of NOGO such as NOGO-A and the splice variants described, unless a specific form is indicated.

"Neutralising" and grammatical variations thereof refers to inhibition, either total or partial, of NOGO function including its binding to neurones and inhibition of neurite growth.

The terms Fv, Fc, Fd, Fab, or F(ab)₂ are used with their standard meanings (see, e.g., Harlow et al., Antibodies A Laboratory Manual, Cold Spring Harbor Laboratory, (1988)).

A "chimeric antibody" refers to a type of engineered antibody which contains a naturally-occurring variable region (light chain and heavy chains) derived from a donor antibody in association with light and heavy chain constant regions derived from an acceptor antibody.

A "humanized antibody" refers to a type of engineered antibody having its CDRs derived from a non-human donor immunoglobulin, the remaining immunoglobulin-derived parts of the molecule being derived from one (or more) human immunoglobulin(s). In addition, framework support residues may be altered to preserve binding affinity (see, e.g., Queen et al., Proc, Natl Acad Sci USA, 86:10029-10032 (1989), Hodgson et al., Bio/Technology, 9:421 (1991)). A suitable human acceptor antibody may be one selected from a conventional database, e.g., the KABAT® database, Los Alamos database, and Swiss Protein database, by homology to the nucleotide and amino acid sequences of the donor antibody. A human antibody characterized by a homology to the framework regions of the donor antibody (on an amino acid basis) may be suitable to provide a heavy chain constant region and/or a heavy chain variable framework region for insertion of the donor CDRs. A suitable acceptor antibody capable of donating light chain constant or variable framework regions may be selected in a similar manner. It should be noted that the acceptor antibody heavy and light chains are not required to originate from the same acceptor antibody. The prior art describes several ways of producing such humanised antibodies - see for example EP-A-0239400 and EP-A-054951

The term "donor antibody" refers to an antibody (monoclonal, and/or recombinant) which contributes the amino acid sequences of its variable regions, CDRs, or other functional fragments or analogs thereof to a first immunoglobulin partner, so as to provide the altered immunoglobulin coding region and resulting expressed altered antibody with the antigenic specificity and neutralizing activity characteristic of the donor antibody.

The term "acceptor antibody" refers to an antibody (monoclonal and/or recombinant) heterologous to the donor antibody, which contributes all (or any portion, but preferably all) of the amino acid sequences encoding its heavy and/or light chain framework regions and/or its heavy and/or light chain constant regions to the first immunoglobulin partner. Preferably a human antibody is the acceptor antibody.

"CDRs" are defined as the complementarity determining region amino acid sequences of an antibody which are the hypervariable regions of immunoglobulin heavy and light chains. See, e.g., Kabat et al., Sequences of Proteins of Immunological Interest, 4th Ed., U.S. Department of Health and Human Services, National Institutes of Health (1987). There are three heavy chain and three light chain CDRs (or CDR regions) in the variable portion of an immunoglobulin. Thus, "CDRs" as used herein refers to all three heavy chain CDRs, or all three light chain CDRs (or both all heavy and all light chain CDRs, if appropriate).
The structure and protein folding of the antibody may mean that other residues are considered part of the antigen binding region and would be understood to be so by a skilled person. See for example Chothia et al., (1989) Conformations of immunoglobulin hypervariable regions; Nature 342, p877-883.

CDRs provide the majority of contact residues for the binding of the antibody to the antigen or epitope. CDRs of interest in this invention are derived from donor antibody variable heavy and light chain sequences, and include analogs of the naturally occurring CDRs, which analogs also share or retain the same antigen binding specificity and/or neutralizing ability as the donor antibody from which they were derived.

A "functional fragment" is a partial heavy or light chain variable sequence (e.g., minor deletions at the amino or carboxy terminus of the immunoglobulin variable region) which retains the same antigen binding specificity and the same or similar neutralizing ability as the antibody from which the fragment was derived.

An "analog" is an amino acid sequence modified by at least one amino acid, wherein said modification can be chemical or a substitution or a rearrangement of a few amino acids (i.e., no more than 10), which modification permits the amino acid sequence to retain the biological characteristics, e.g., antigen specificity and high affinity, of the unmodified sequence.

The present invention also includes the use of Fab fragments or F(ab')₂ fragments derived from mAbs of the present invention directed against NOGO. A Fab fragment contains the entire light chain and amino terminal portion of the heavy chain; and an F(ab')₂ fragment is the fragment formed by two Fab fragments bound by disulfide bonds. Fab fragments and F(ab')₂ fragments can be obtained by conventional means, e.g., cleavage of mAb with the appropriate proteolytic enzymes, papain and/or pepsin, or by recombinant methods. The Fab and F(ab')₂ fragments are useful themselves as therapeutic or prophylactic, and as donors of sequences including the variable regions and CDR sequences useful in the formation of recombinant or humanized antibodies as described herein.

In still a further embodiment, the antibody of the invention may have attached to it an additional agent. For example, the procedure of recombinant DNA technology may be used to produce an engineered antibody of the invention in which the Fc fragment or CH2-CH3 domain of a full length antibody molecule has been replaced by an enzyme or other detectable molecule (i.e., a polypeptide effector or reporter molecule).

The antibodies of the present invention may be produced by making a conventional expression vector or recombinant plasmid by placing these coding sequences for the antibody in operative association with conventional regulatory control sequences capable of controlling the replication and expression in, and/or secretion from, a host cell. Regulatory sequences include promoter sequences, e.g., CMV promoter, and signal sequences, which can be derived from other known antibodies. Similarly, a second expression vector can be produced having a DNA sequence which encodes a complementary antibody light or heavy chain. Preferably this second expression vector is identical to the first except insofar as the coding sequences and selectable markers are concerned, so to ensure as far as possible that each polypeptide chain is functionally expressed. Alternatively, the heavy and light chain coding sequences for the altered antibody may reside on a single vector.

A selected host cell is co-transfected by conventional techniques with both the first and second vectors (or simply transfected by a single vector) to create the transfected host cell of the invention comprising both the recombinant or synthetic light and heavy chains. The transfected cell is then cultured by conventional techniques to produce the engineered antibody of the invention. The antibody which includes the association of both the recombinant heavy chain and/or light chain is screened from culture by appropriate assay, such as ELISA or RIA. Similar conventional techniques may be employed to construct other altered antibodies and molecules.

Suitable vectors for the cloning and subcloning steps employed in the methods and construction of the compositions of this invention may be selected by one of skill in the art. For example, the conventional pUC series of cloning vectors may be used. One vector, pUC19, is commercially available from supply houses, such as Amersham (Buckinghamshire, United Kingdom) or Pharmacia (Uppsala, Sweden). Additionally, any vector which is capable of replicating readily, has an abundance of cloning sites and selectable genes (e.g., antibiotic resistance), and is easily manipulated may be used for cloning.

Similarly, the vectors employed for expression of the antibodies may be selected by one of skill in the art from any conventional vector. The vectors also contain selected regulatory sequences (such as CMV or RSV promoters) which direct the replication and expression of heterologous DNA sequences in selected host cells. These vectors contain the above described DNA sequences which code for the antibody or altered immunoglobulin coding region. In addition, the vectors may incorporate the selected immunoglobulin sequences modified by the insertion of desirable restriction sites for ready manipulation.

The expression vectors may also be characterized by genes suitable for amplifying expression of the heterologous DNA sequences, e.g., the mammalian dihydrofolate reductase gene (DHFR). Other preferable vector sequences include a poly A signal sequence, such as from bovine growth hormone (BGH) and the betaglobin promoter sequence (betaglopro). The expression vectors useful herein may be synthesized by techniques well known to those skilled in this art.

The components of such vectors, e.g. replicons, selection genes, enhancers, promoters, signal sequences and the like, may be obtained from commercial or natural sources or synthesized by known procedures for use in directing the expression and/or secretion of the product of the recombinant DNA in a selected host. Other appropriate expression vectors of which numerous types are known in the art for mammalian, bacterial, insect, yeast, and fungal expression may also be selected for this purpose.

Also described herein is a cell line transfected with a recombinant plasmid containing the coding sequences of the antibodies or altered immunoglobulin molecules thereof. Host cells useful for the cloning and other manipulations of these cloning vectors are also conventional. However, most desirably, cells from various strains of E. coli are used for replication of the cloning vectors and other steps in the construction of altered antibodies.

Suitable host cells or cell lines for the expression of the antibody of the invention are preferably mammalian cells such as NS0, Sp2/0, CHO (e.g. DG44), COS, a fibroblast cell (e.g., 3T3), and myeloma cells, and more preferably a CHO or a myeloma cell. Human cells may be used, thus enabling the molecule to be modified with human glycosylation patterns. Alternatively, other eukaryotic cell lines may be employed. The selection of suitable mammalian host cells and methods for transformation, culture, amplification, screening and product production and purification are known in the art. See, e.g., Sambrook et al., cited above.

Bacterial cells may prove useful as host cells suitable for the expression of the recombinant Fabs of the present invention (see, e.g., Plückthun, A., Immunol. Rev., 130:151-188 (1992)). However, due to the tendency of proteins expressed in bacterial cells to be in an unfolded or improperly folded form or in a nonglycosylated form, any recombinant Fab produced in a bacterial cell would have to be screened for retention of antigen binding ability. If the molecule expressed by the bacterial cell was produced in a properly folded form, that bacterial cell would be a desirable host. For example, various strains of E. coli used for expression are well-known as host cells in the field of biotechnology: Various strains of B. subtilis, Streptomyces, other bacilli and the like may also be employed in this method.

Where desired, strains of yeast cells known to those skilled in the art are also available as host cells, as well as insect cells, e.g. Drosophila and Lepidoptera and viral expression systems. See, e.g. Miller et al., Genetic Engineering, 8:277-298, Plenum Press (1986) and references cited therein.

The general methods by which the vectors may be constructed, the transfection methods required to produce the host cells of the invention, and culture methods necessary to produce the antibody of the invention from such host cell are all conventional techniques. Typically, the culture method of the present invention is a serum-free culture method, usually by culturing cells serum-free in suspension. Likewise, once produced, the antibodies of the invention may be purified from the cell culture contents according to standard procedures of the art, including ammonium sulfate precipitation, affinity columns, column chromatography, gel electrophoresis and the like. Such techniques are within the skill of the art and do not limit this invention. For example, preparation of altered antibodies are described in WO 99/58679 and WO 96/16990.

Yet another method of expression of the antibodies may utilize expression in a transgenic animal, such as described in U. S. Patent No. 4,873,316. This relates to an expression system using the animal's casein promoter which when transgenically incorporated into a mammal permits the female to produce the desired recombinant protein in its milk.

Once expressed by the desired method, the antibody is then examined for in vitro activity by use of an appropriate assay. Presently conventional ELISA assay formats are employed to assess qualitative and quantitative binding of the antibody to NOGO. Additionally, other in vitro assays may also be used to verify neutralizing efficacy prior to subsequent human clinical studies performed to evaluate the persistence of the antibody in the body despite the usual clearance mechanisms.

The therapeutic agents of this invention may be administered as a prophylactic or following the stroke event/on-set of clinical symptoms, or as otherwise needed. The dose and duration of treatment relates to the relative duration of the molecules of the present invention in the human circulation, and can be adjusted by one of skill in the art depending upon the condition being treated and the general health of the patient. It is envisaged that repeated dosing (e.g. once a week or once every two weeks) over an extended time period (e.g. four to six months) maybe required to achieve maximal therapeutic efficacy.

The mode of administration of the therapeutic agent of the invention may be any suitable route which delivers the agent to the host. The antibodies and pharmaceutical compositions of the invention are parlicularly useful for parenteral administration, i.e., subcutaneously, intrathecally, intraperitoneally, intramuscularly, intravenously, or intranasally, of which intravenously is particularly preferred.

Therapeutic agents of the invention may be prepared as pharmaceutical compositions containing an effective amount of the antibody of the invention as an active ingredient in a pharmaceutically acceptable carrier. In the prophylactic agent of the invention, an aqueous suspension or solution containing the engineered antibody, preferably buffered at physiological pH, in a form ready for injection is preferred. The compositions for parenteral administration will commonly comprise a solution of the antibody of the invention or a cocktail thereof dissolved in a pharmaceutically acceptable carrier, preferably an aqueous carrier. A variety of aqueous carriers may be employed, e.g., 0.9% saline, 0.3% glycine, and the like. These solutions are sterile and generally free of particulate matter. These solutions may be sterilized by conventional, well known sterilization techniques (e.g., filtration). The compositions may contain pharmaceutically acceptable auxiliary substances as required to approximate physiological conditions such as pH adjusting and buffering agents, etc. The concentration of the antibody of the invention in such pharmaceutical formulation can vary widely, i.e., from less than about 0.5%, usually at or at least about 1% to as much as 15 or 20% by weight and will be selected primarily based on fluid volumes, viscosities, etc., according to the particular mode of administration selected.

Thus, a pharmaceutical composition of the invention for intramuscular injection could be prepared to contain 1 mL sterile buffered water, and between about 1 ng to about 100 mg, e.g. about 50 ng to about 30 mg or more preferably, about 5 mg to about 25 mg, of an antibody of the invention. Similarly, a pharmaceutical composition of the invention for intravenous infusion could be made up to contain about 250 ml of sterile Ringer's solution, and about 1 to about 30 and preferably 5 mg to about 25 mg of an engineered antibody of the invention. Actual methods for preparing parenterally administrable compositions are well known or will be apparent to those skilled in the art and are described in more detail in, for example, Remington's Pharmaceutical Science, 15th ed., Mack Publishing Company, Easton, Pennsylvania. For the preparation of intravenously administrable antibody formulations of the invention see Lasmar U and Parkins D "The formulation of Biopharmaceutical products", Pharma. Sci.Tech.today, page 129-137, Vol.3 (3rd April 2000), Wang, W "Instability, stabilisation and formulation of liquid protein pharmaceuticals", Int. J. Pharm 185 (1999) 129-188, Stability of Protein Pharmaceuticals Part A and B ed Ahern T.J., Manning M.C., New York, NY: Plenum Press (1992), Akers,M.J. "Excipient-Drug interactions in Parenteral Formulations", J.Pharm Sci 91 (2002) 2283-2300, Imamura, K et al "Effects of types of sugar on stabilization of Protein in the dried state", J Pharm Sci 92 (2003) 266-274, Izutsu, Kkojima, S. "Excipient crystalinity and its protein-structure-stabilizing effect during freeze-drying", J Pharm. Pharmacol, 54 (2002) 1033-1039, Johnson, R, "Mannitol-sucrose mixtures-versatile formulations for protein lyophilization", J. Pharm. Sci, 91 (2002) 914-922. Ha,E Wang W, Wang Y.j. "Peroxide formation in polysorbate 80 and protein stability". J. Pharm Sci, 91,2252-2264,(2002) .

It is preferred that the therapeutic agent of the invention, when in a pharmaceutical preparation, be present in unit dose forms. The appropriate therapeutically effective dose can be determined readily by those of skill in the art. To effectively treat stroke and other neurological diseases in a human, one dose of up to 700 mg per 70 kg body weight of an antagonist or antibody of this invention should be administered parenterally, preferably i.v. or i.m. (intramuscularly). Such dose may, if necessary, be repeated at appropriate time intervals selected as appropriate by a physician. As disclosed in the examples, the present inventors have been able to demonstrate a positive effect on functional recovery in the rat model therein when antibodies of the invention were administered intravenously.

The antibodies described herein can be lyophilized for storage and reconstituted in a suitable carrier prior to use. This technique has been shown to be effective with conventional immunoglobulins and art-known lyophilization and reconstitution techniques can be employed.

Antibodies of the invention may also be used in combination (i.e. simultaneously, sequentially or separately) with a neurotrophic factor such as nerve growth factor (NGF), for example brain derived neurotrophic factor (BDNF), anti-inflammatory agents such as corticosteroids, and/or tPA. Combinations of a NOGO antibody of the invention and e.g. tPA maybe assessed in the MCAO model set forth in the examples below.

In another aspect, the invention provides a pharmaceutical composition comprising anti-NOGO antibody of the present invention or a functional fragment thereof and a pharmaceutically acceptable carrier for treatment or prophylaxis of stroke and other neurological diseases.

In a yet further aspect, the invention provides a pharmaceutical composition comprising the anti-NOGO antibody of the present invention or a functional fragment thereof and a pharmaceutically acceptable carrier for inhibiting neurodegeneration and/or promoting functional recovery in a human patient suffering, or at risk of developing, a stroke or other neurological disease.

Antibodies of the invention may be used in methods of treatment to slow or halt the progression and/or onset of Alzheimer's disease in addition to (or as an alternative to) treating established disease in a human patient.

Neurological diseases or disorders as used hereinabove includes, but is not limited to traumatic brain injury, spinal cord injury, fronto-temporal dementias (tauopathies), peripheral neuropathy, Parkinson's disease, Huntington's disease, multiple sclerosis and in particular Alzheimer's disease.

The antibodies of invention may also be used in a method of promoting axonal sprouting comprising the step of contacting a human axon with an anti-NOGO antibody. This method may be performed in-vitro or in-vivo, preferably the method is performed in-vivo.

As used herein, the term "functional recovery" refers to a motor and/or sensory and/or behavioural improvement in a subject following e.g. an ischemic event or injury or on-set of clinical symptoms. Functional recovery in humans may be evaluated by instruments designed to measure elemental neurological functions such as motor strength, sensation and coordination, cognitive functions such as memory, language and the ability to follow directions, and functional capacities such as basic activities of daily living or instrumental activities. Recovery of elemental neurological function can be measured with instruments such as the NIH Stroke Scale (NIHSS), recovery of cognitive function can be measured with neuropsychological tests such as Boston Naming Test, Trail-making Tests, and California Verbal Learning Test, and activities of daily living may be measured with instruments such as the ADCS/ADL (Alzheimer's Disease Clinical Studies/Activities of Daily Living) scale or the Bristol Activities of Daily Living Scale, all tests and scales known in the art.

### Example 1, Construction and expression of humanised anti-NOGO antibodies

Murine and Humanised V_{H} and V_{L} constructs were prepared de novo by build up of overlapping oligonucleotides including restriction sites for cloning into Rld and Rln mammalian expression vectors as well as a human signal sequence. Hind III and Spell restriction sites were introduced to frame the V_{H} domain containing the CAMPATH-1 H signal sequence for cloning into Rld containing the human γ1 mutated constant region. Hind III and BsiWI restriction sites were introduced to frame the V_{L} domain containing the CAMPATH-1 H signal sequence for cloning into Rln containing the human kappa constant region.
CAMPATH-1H signal sequence: MGWSCIILFLVATATGVHS (SEQ.I.D.NO:82)
In parallel a chimeric version of 11C7 was generated (see WO04/052932). The variable heavy domain sequence (derived from WO04/052932 Seq ID 43) and variable light domain sequence (derived from WO04/052932 Seq ID 44) were prepared de novo by build up of overlapping oligonucleotides. Hind III and Spel restriction sites were introduced to frame the V_{H} domain for cloning into Rld containing the human γ1 mutated constant region. HindIII and BsiWI restriction sites were introduced to frame the V_{L} domain for cloning into Rln containing the human kappa constant region.

### Example 2, Antibody expression in CHO cells

Rld and Rln plasmids encoding the heavy and light chains respectively were transiently co-transfected into CHO cells and expressed at small scale or large scale to produce antibody. Alternatively the same plasmids were co-transfected into CHO cells by electroporation and a stable polyclonal population of cells expressing the appropriate antibody were selected using a nucleoside-free media. In some instances the antibody contained in the supernatant was assayed, in other cases the recombinant antibody was recovered and purified by affinity chromatography on Protein A sepharose.

### Example 3, Humanised anti-NOGO antibody binds to NOGO

GST-human NOGO-A56 (SEQ ID: 76) at 1µg/ml in PBS was coated onto Nunc Immunosorp plates (100µl per well) at 4°C overnight. Wells were rinsed once with TBS + 0.05% Tween (TBST) then incubated with 2% BSA in TBST to block non-specific binding sites at room temperature for 1 hour. Antibodies were diluted in TBST + 2% BSA to 10µg/ml and 1/2 dilutions made from this. Antibodies were added to wells in duplicate and incubated at room temperature for 1 hour. Wells were washed three times with TBST then incubated with anti-human kappa peroxidase conjugate (1:2000) for 1 hour. The wells were washed three times with TBST and then incubated with 100µl OPD peroxidase substrate (Sigma) per well for 10 minutes. The colour reaction was stopped by the addition of 25µl concentrated H₂SO₄. Optical density at 490nm was measured using a plate reader. Background values read from wells with no antibody were subtracted.

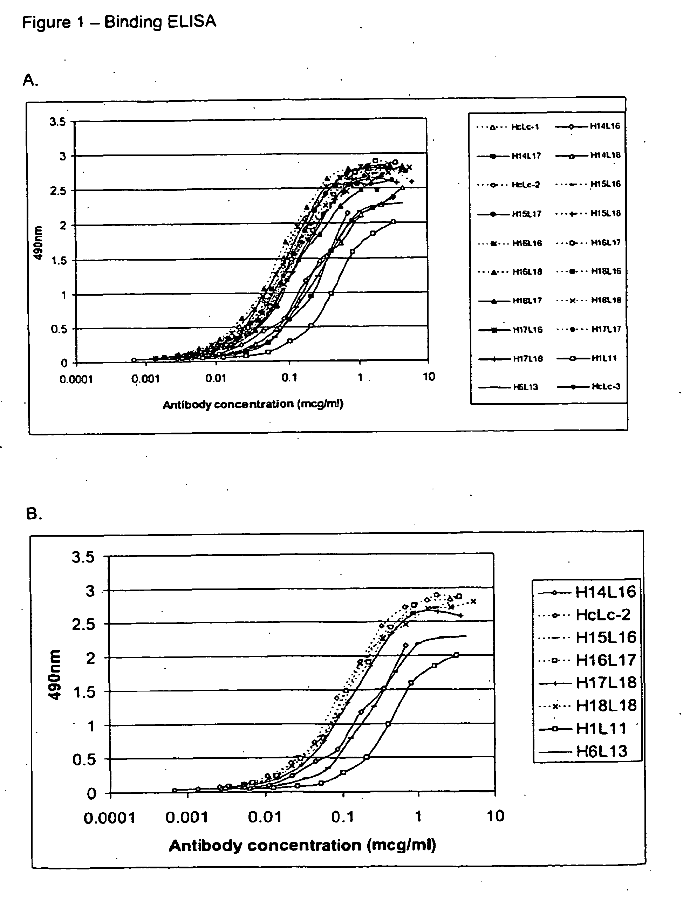

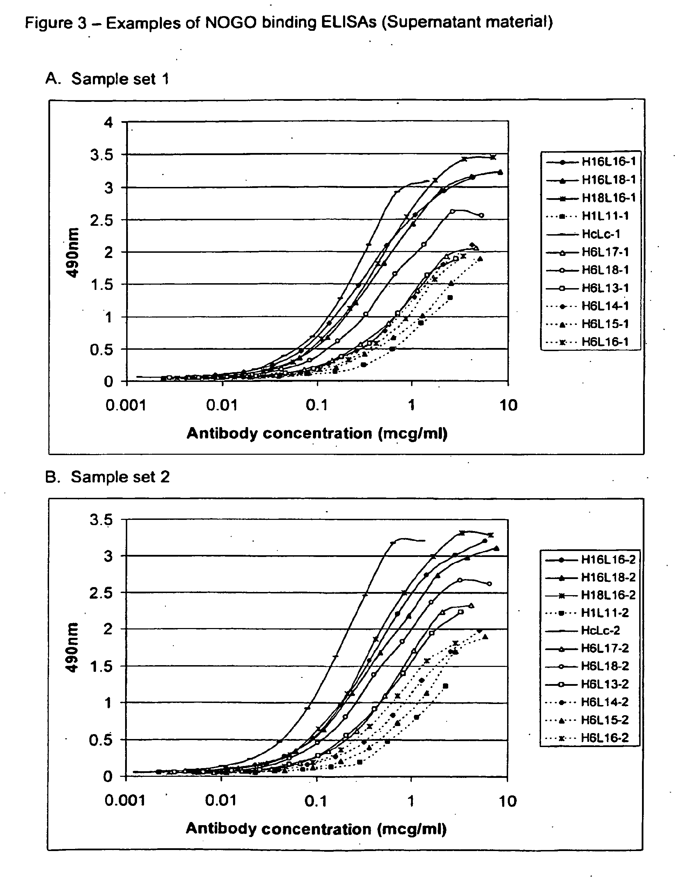

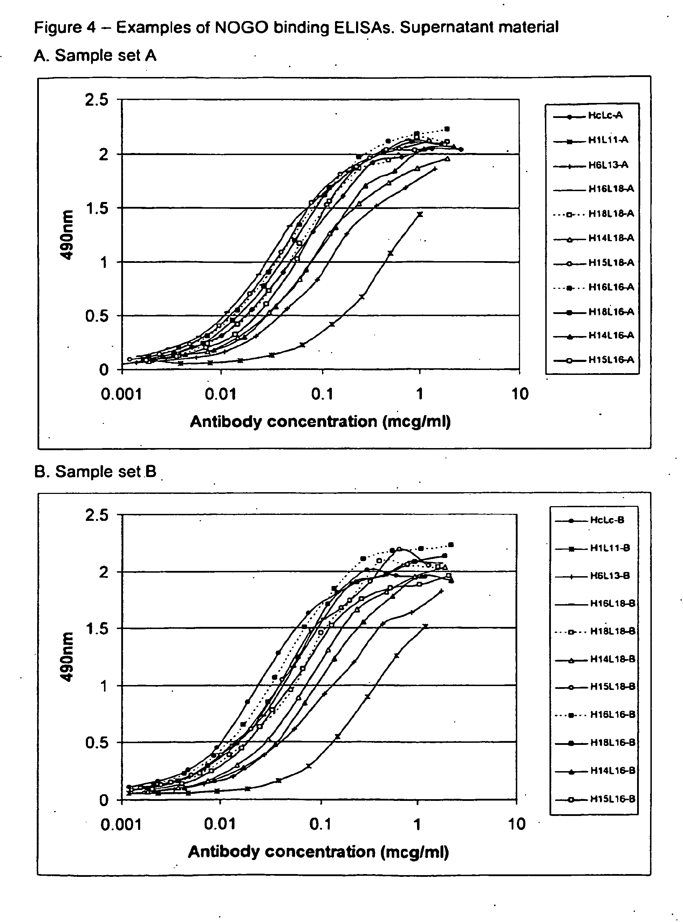

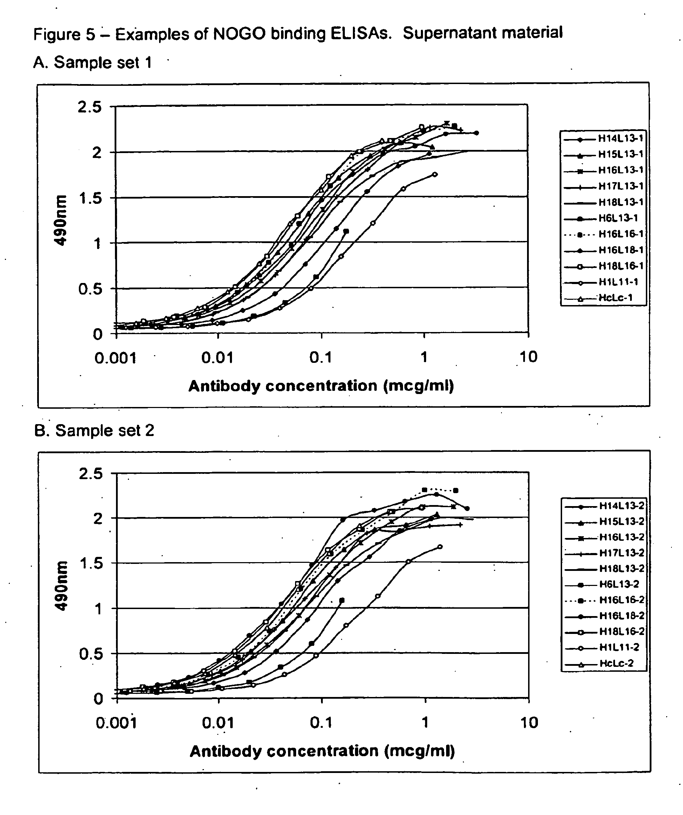

Figures 1-5 illustrate the dose-dependent binding of humanised antibodies in comparison with the chimera (termed HcLc which is the chimera of 2A10 (comprising the 2A10 murine VH (SEQ ID NO. 7) and VL (SEQ ID NO.8) and human l9G constant regions)) to human NOGO-A56 (see Example 6 for details) in an ELISA assay. The Y-axis shows the measured optical density (OD) at 490nm, a quantitative measure of antibody captured in the wells. The X-axis shows the concentration of antibody used (mcg/ml) per well at each data point.

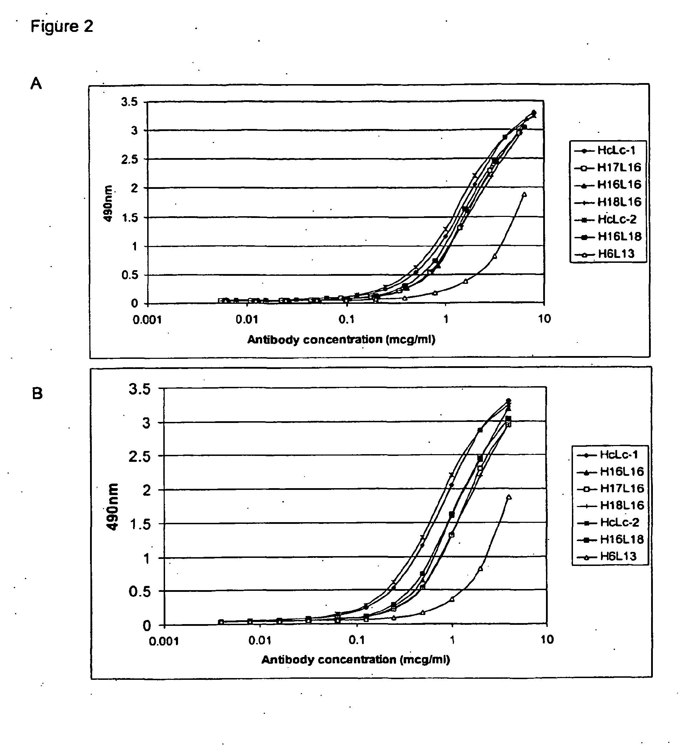

The antibody material used in Figures 1, 3, 4 and 5 was generated from small scale transient transfections. Human IgG levels in the supernatant are quantified by ELISA (Example 4). For figure 2, the material used was purified antibody (see example 2) generated by either the polyclonal expression system or large scale transient transfections. In these cases, IgG levels were quantified by ELISA and optical density.

In another experiment, antibody material (in the form of CHO cell culture supernatant) was generated from small scale transient transfections (in triplicate) for the following humanised antibodies: H16L16, H17L16, H18L16, H16L18 and the chimeric antibody HcLc. The results from this experiment are consistent with the data presented in Figures 1-5 with the exception of H17L16 which performed less well than shown in Figures 1A and Figure 2. Whilst this observation cannot be explained it should be noted that the conclusions are contradicted by another experiment with supernatant material (see Figure 1A) and an experiment using purified H17L16 material (Figure 2), both experiments indicated that H17L16 shows comparable binding to the other optimised variants.

### Example 4, Antibody quantification protocol

Nunc Immunosorp plates were coated with capture antibody H 19 (goat anti-human IgG chain, Sigma #l3382) at 2µg/ml in Bicarbonate buffer (Sigma #C3041) and incubated overnight at 4°C. The plates were washed twice with TBS containing 0.05% Tween20 (TBST) and blocked with 200µl TBST containing 2% BSA (block buffer) for 1 hr at room temperature. The plates were washed twice with TBST. Tissue culture supernatants containing antibody were titrated across the plate in 2-fold dilution steps into block buffer and incubated at room temperature for 1 hr. The plates were washed three times with TBST. HRP conjugated antibody H23 (goat anti-human kappa chain, Sigma #A7164) was diluted 1:2000 in TBST and 100µl added to each well. The plates were incubated at room temperature for 1 hr. The plates were washed three times with TBST and developed with 100µl of Fast-OPD substrate (Sigma #P9187). Colour was allowed to develop for 5-10mins after which time the ELISA was stopped with 25µl 3M H₂SO₄. The absorbance at 490nM was read plate and antibody concentration determined by reference to a standard curve.

### Example 5, Antibody competition ELISA protocol

GST-human NOGO-A56 (SEQ ID: 76) at 0.1-1.0µg/m) in PBS was coated onto Nunc Immunosorp plates (100µl per well) at 4°C overnight or at 37°C for 1 hour. Wells were rinsed three times with PBS then incubated with 1% BSA in PBS (block buffer) to block non-specific binding sites at room temperature for 2 hours. In parallel, a 50:50 mix of antibodies was made. Murine antibody 2A10 was added to a final concentration of either 0.5 or 1.0 mcg/ml in block buffer. Chimeric antibodies (mouse variable regions cloned onto a human IgG1 Fc mutated constant region) were added to a final concentration of 0-25mcg/ml in block buffer. The block buffer was removed from the plates and 100µl of the 50:50 mix of antibodies was added for 1 hour at room temperature. Wells were washed three times with PBS then incubated with 100µl of rabbit polyclonal antimouse immunoglobulins peroxidase conjugate (diluted 1:2000 in block buffer, DakoCytomation #P0260) for 1 hour at room temperature. The wells were washed three times with PBS and then incubated with 100µl OPD peroxidase substrate (Sigma #P9187) or TMB substrate (Sigma #T8665) per well for 10-30 minutes. The colour reaction was stopped by the addition of 25µl concentrated H₂SO₄. Optical density at 490nm (OPD) or 450nm (TMB) was measured using a plate reader.

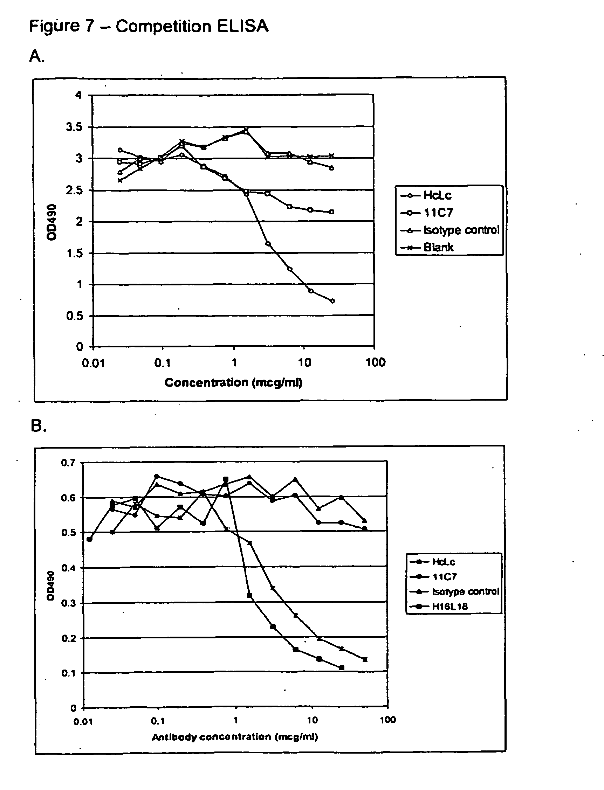

In the first experiment (Figure 7A), plates were coated with GST-human NOGO-A56 at 0.5µg/ml in PBS overnight at 4°C and the plates were developed with TMB substrate. In this experiment, the murine antibody 2A10 was assessed in combination with HcLc (the chimeric form of 2A10), 11C7, an isotype control Chimeric antibody and a blank control. In the second experiment (Figure 7B), plates were coated with GST-human NOGO-A56 at 0.5µg/ml in PBS at 37°C for 1 hour and the plates were developed with OPD substrate. In this experiment, the murine antibody 2A10 was assessed in combination with HcLc, 11C7, an isotype control and purified H16L18 antibody.

### Example 6, Production of NOGO-A Fragment (NOGO-A56, SEQ.I.D.NI:76)

A cDNA sequence encoding amino acids 586-785 (MQESLYPAAQLCPSFEESEATPSPVLPDIVMEAPLNSAVPSAGASVIQPSSSPL EASSVNYESIKHEPENPPPYEEAMSVSLKKVSGIKEEIKEPENINAALQETEAPYI SIACDLIKETKLSAEPAPDFSDYSEMAKVEQPVPDHSELVEDSSPDSEPVDLFS DDSIPDVPQKQDETVMLVKESLTETSFESMIEYENKE - SEQ.I.D.NO:76) of human NOGO-A was cloned into the BamHI-Xhol sites of pGEX-6P1 to generate a GST-tagged fusion protein designated NOGO-A56. Plasmid was expressed in BL21 cells in 2XTY medium with 100µg/ml ampicillin following induction with IPTG to 0.5mM at 37C for 3hours. Cell pellets were lysed by sonication and the fusion protein purified using Glutathione-sepharose (Amersham Pharmacia) following manufacturers instructions. Purified protein was eluted using reduced glutathione and extensively dialysed against PBS, quantitated using BSA standards and a BioRad coomassie based protein assay and then stored in aliquots at -80C.

### Example 7, BiaCore Analysis of humanised Anti NOGO Monoclonal Antibodies

The binding kinetics of the purified anti-NOGO monoclonal antibodies (mAb) to recombinantly expressed human NOGO-A (GST-human NOGO-A56) was analysed using the Biacore3000 biosensor. The hNOGO-A chip was prepared as follows:

### Method

hNOGO (GST-human NOGO-A56) was immobilised to a CM5 chip by primary amine coupling using the Biacore Wizard program designed for targeted immobilisation levels. The CM5 sensor surface was activated by passing a solution of 50mM N-hydroxy-succinimide (NHS) and 200mM N-ethyl-N'-dimethylaminopropyl carbonide (EDC). Then hNOGO in sodium acetate buffer, pH5.0 or pH 4.5, was passed over the chip and immobilised. After immobilisation was complete any still activated esters were blocked by an injection of 1M ethanolamine hydrochloride, pH8.5.

The anti-NOGO mAbs were diluted down in HBS-EP (10mM HEPES, pH 7.4, 150mM NaCl, 3mM EDTA, and 0.005% P-20 surfactant) and binding studies were carried out at range of defined antibody concentrations. All runs were referenced against a blanked sensor surface (one that had been activated and blocked as described earlier but had no addition of ligand). Analysis of binding was carried out using the BIAevaluation kinetic analysis software version 4.1. Biacore analysis of other antibodies of the invention essentially followed the same protocol as described herein.

### Results - Table 8

Mean (+/- standard deviation) of four separate experiments

| Antibody | ka (1/Ms) | kd (1/s.) | KD (nM) |
|---|---|---|---|
| HcLc | 2.70e6 (2.7e5) | 3.78e-3 (7.0e-4) | 1.41 (0.3) |
| H6L13 | 1.82e6 (3.5e5) | 1.37e-2(2.0e-3) | 7.68(1.2) |
| H16L16 | 4.37e6 (4.5e5) | 5.54e-3 (1.4e-3) | 1.26 (0.2) |
| H16L18 | 4.18e6 (4.1e5) | 5.52e-3 (9.0e-4) | 1.33 (0.2) |
| H17L16 | 3.38e6 (1.3e5) | 6.10e-3(1.3e-3) | 1.82(0.4) |
| H18L16 | 3.64e6 (3.5e5) | 5.86e-3 (9.0e-4) | 1.62 (0.3) |
| H1L11 | 1.73e6 (1.7e5) | 3.14 e-2 (4.2e-3) | 18.6(3.7) |

### Results - Table 9

Results shown are from a single experiment with the exception of HcLc and H6Lc where the values shown are the mean (+/- standard deviation) of two separate experiments

| Antibody (number of independent runs) | ka (1/Ms) | kd (1/s) | KD (nM) |
|---|---|---|---|
| HcLc (2) | 3.52e6 (2.8e5) | 3.46e-3 (7.9e-4) | 0.995 (0.3) |
| HcL11 | 3.78e6 | 1.34e-2 | 3.55 |
| H6Lc (2) | 2.17e6 (3.8e5) | 2.84e-3 (1.5e-3) | 2.21 (0.3) |
| HcL13 | 4.8e6 | 9.38e-3 | 1.98 |
| H6L13 | 2.95e6 | 2.33e-2 | 7.9 |
| H6L6 | 1.2e6 | 2.54e-2 | 21.4 |
| H10L13 | 1.64e6 | 2.12e-2 | 12.95 |
| H700L11 | 1.19e6 | 2.33e-2 | 19.45 |
| H5L6 | 1.5e6 | 4.7e-2 | 30.3 |
| 11C7 | 1.44e6 | 8.25e-5 | 0.057 |

### Results - Table 10

Results shown are from a single experiment

| Antibody | ka (1/Ms) | kd (1/s) | KD (nM) |
|---|---|---|---|
| H6L13 | 1.77e6 | 1.3e-2 | 7.3 |
| H16L16 | 5.47e6 | 5.69e-3 | 1.0 |
| H14L16 | 2.05e6 | 7.39e-3 | 3.6 |
| H14L18 | 2.41 e6 | 8.69e-3 | 3.6 |
| H1L11 | 1.33e6 | 2.84e-2 | 21.3 |
| HcLc | 2.62e6 | 4.12e-3 | 1.6 |

### Example 8. BiaCore Analysis of humanised Anti NOGO Monoclonal Antibodies using off-rate ranking

The hNOGO chip was prepared as for kinetic analysis. Cell supernatants where taken directly from transient transfections of CHO-K1 cells. These were passed directly over the sensor surface and the interaction measured. A mock transfected cell supernatant was used for double referencing to remove any artefacts due to the tissue culture media. All runs were referenced against a blanked sensor surface (one that had been activated and blocked as described earlier but had no addition of ligand). Analysis of binding was carried out using the BIAevaluation kinetic analysis software version 4.1.

### Results - Table 11

Results shown are from a single experiment with the exception of H6L13, H16L16, H16L18, H1L11, HcLc and H18L16 where the values shown are the mean (+/- standard deviation) of two or three separate experiments

| Antibody (number of independent runs) | kd (1/s) |
|---|---|
| H14L13 | 1.38e-2 |
| H15L13 | 9.65e-3 |
| H16L13 | 9.07e-3 |
| H17L13 | 9.31e-3 |
| H18L13 | 9.07e-3 |
| H6L13 (x3) | 1.73e-2 (4.8e-3) |
| H16L16 (x3) | 6.64e-3 (9.2e-4) |
| H16L18 (x3) | 6.09e-3 (7.4e-4) |
| H1L11(x3) | 4.03e-2 (1.8e-2) |
| HcLc (x2) | 3.76e-3 (7.1 e-4) |
| H15L16 | 6.04e-3 |
| H14L16 | 8.9e-3 |
| H18L16 (x2) | 6.87e-3 (7.5e-4) |
| H14L18 | 8.35e-3 |
| H15L18 | 5.94e-3 |
| H18L18 | 5.8e-3 |
| H6L17 | 1.58e-2 |
| H6L18 | 1.06e-2 |
| H6L14 | 4.57e-2 |
| H6L15 | 2.11 e-2 |
| H6L16 | 1.14e-2 |

**Example 9,** *FACS Analysis of humanised Anti NOGO Monoclonal Antibodies*

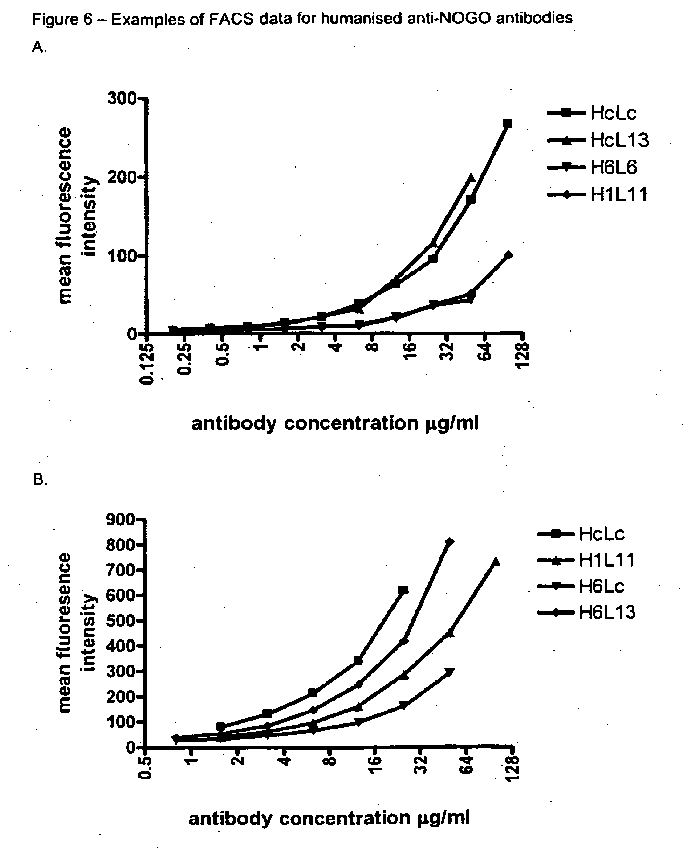

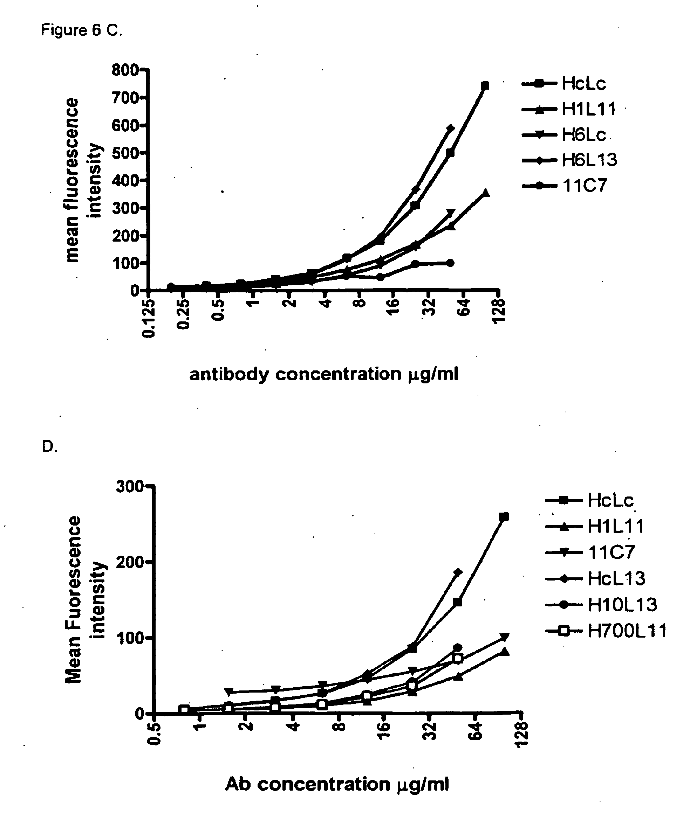

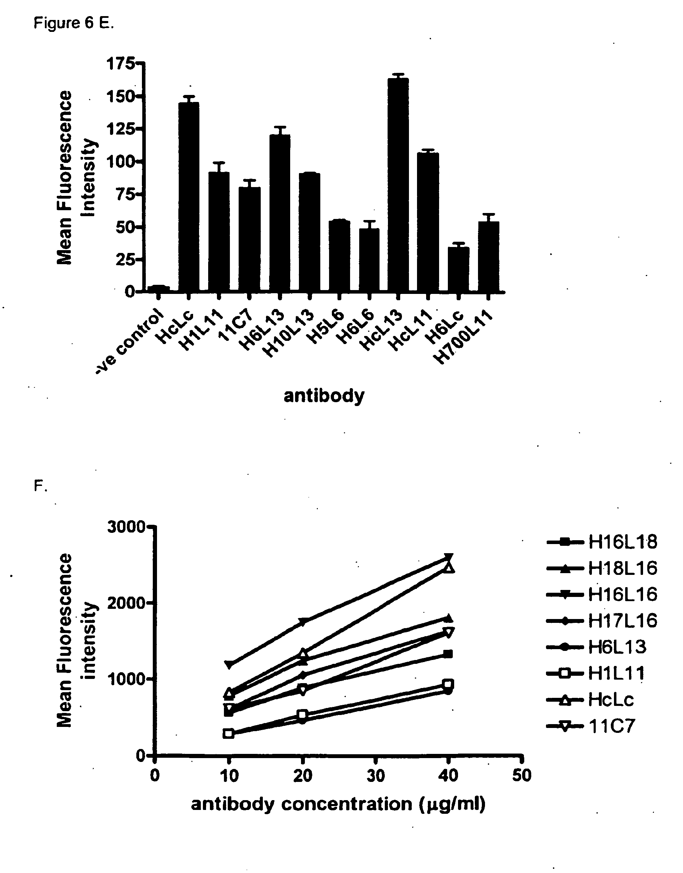

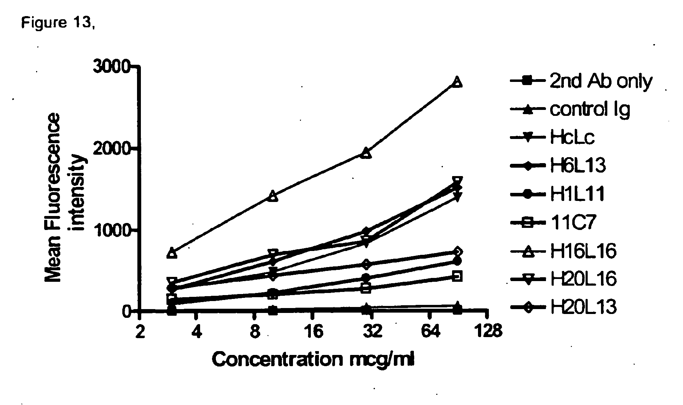

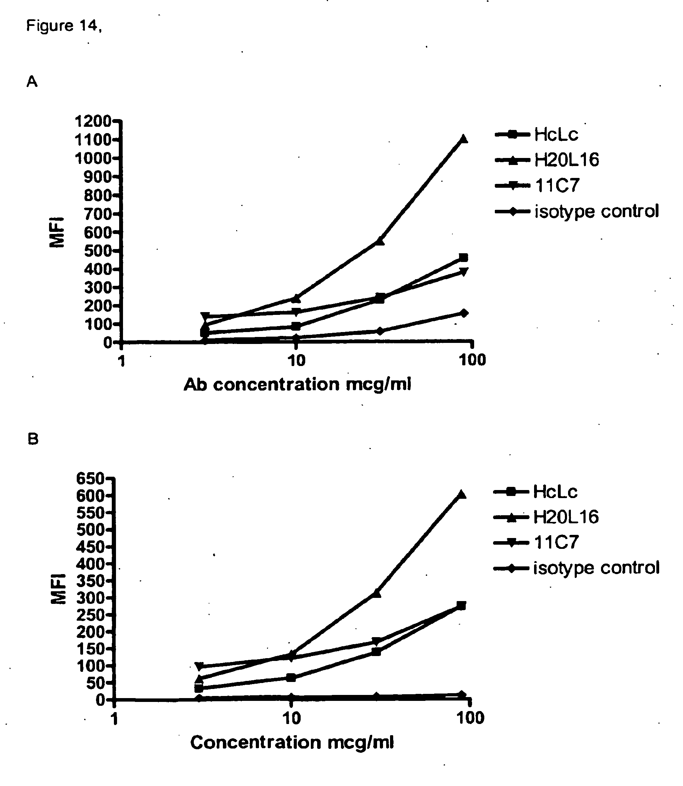

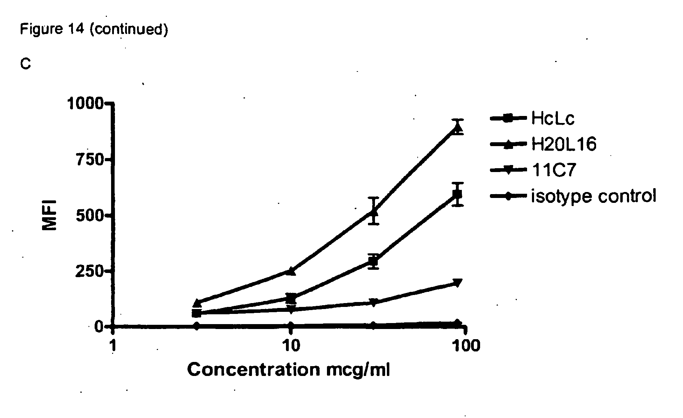

IMR32 human neuroblastoma cells were re-suspended in FACS staining buffer (PBS + 4% heat inactivated FCS) at a density of 10⁶ cells per ml. 100µl of this suspension was transferred to wells of a 96 well round bottomed microplate. 100µl of "Fix & Perm" Medium A (Caltag Laboratories, GAS001S-100) was added to each well and the plate incubated at room temperature for 15 mins. The cells were pelleted was washed twice in FACS staining buffer. Following washing, the cells were re-suspended in 50µl of a solution of the purified anti-NOGO antibodies or an isotype matched control antibody at a concentration of 2X the final concentration (0-200 µg/ml in FACS staining buffer). 50µl of "Fix & Perm" Medium B (Caltag Laboratories GAS002S-100) was added and the plate incubated on ice for 1 hour. Cells were washed twice in FACS staining buffer before being re-suspended in 100µl of a solution of a PE conjugated anti human γ1 specific goat F(ab')2 (Sigma P-8047) at a dilution of 1/50 or 1/100. Cells incubated for 1 hour on ice. The cells were pelleted and washed 3 times in FACs staining buffer and cells re-suspended in 100µl the same buffer. 100µl of "Fix & Perm" Medium B was added to fix the cells for the experiments shown in Figures 6 and 13. 100µl of BD CellFIX was added to fix the cells for the experiments shown in Figure 14. The degree of staining was determined by flow cytometry using a Becton Dickinson FACScan flow cytometer. The isotype matched controls were used as reference.

Results are shown in Figure 6A to F. The totality of the data shown illustrates that the HcLc antibody (of 2A10) gives a strong signal in this FACS assay, which indicates strong binding to human cell expressed NOGO. The data also shows that humanised versions of this chimera can retain this property. The H20L16 antibody consistently outperforms 11C7 in this assay, whilst the 2A10 chimeric antibody, and other humanised versions thereof, consistently either outperform 11 C7 in this assay or are comparable thereto.

### Example 10, Additional humanised anti-NOGO monoclonal antibodies

Following the techniques described in Examples 1 and 2, a number of additional monoclonal antibodies were produced. The binding activity of these antibodies were assayed by BIAcore, ELISA and FACS.

### 10.1 BIAcore

Based on the methodologies described in Example 7, data on the additional anti-NOGO antibodies is shown in tables 13, 14 , 15 and 17 below (purified antibody material) and table 16 (supernatant material).

### Results - Table 13

Results shown are from a single experiment. In this experiment, the value obtained for H16L16 was not typical of values obtained in other experiments (typically exhibiting a KD(nM) around 1 (see tables 8 and 10)).

| Antibody | *ka(1*/*Ms)* | *kd (1*/*s)* | KD (nM) |
|---|---|---|---|
| HcLc | 2.6e6 | 4.4e-3 | 1.7 |
| H20L16 | 4.4e6 | 7.3e-3 | 1.7 |
| H22L16 | 4.9e6 | 6.9e-3 | 1.47 |
| H23L16 | 1.9e6 | 6.4e-3 | 3.8 |
| H24L16 | 1.9e6 | 6.9e-3 | 3.7 |
| H6L13 | 2.2e6 | 1.4e-2 | 6.4 |
| H1L11 | 1.4e6 | 3.1 e-2 | 22.3 |
| H16L16 | 1.2e6 | 4.0e-3 | 3.4 |

### Results - Table 14

Results shown are from a single experiment.

| Antibody | ka (1/Ms) | kd (1/s) | KD (nM) |
|---|---|---|---|
| H1L11 | 1.34e6 | 2.82e-2 | 21.0 |
| H6L13 | 2.02e6 | 1.57e-2 | 7.76 |
| HcLc | 2.46e6 | 3.5e-3 | 1.42 |
| H20L16 | 5.24e6 | 8.47e-3 | 1.61 |
| H22L16 | 5.83e6 | 7.67e-3 | 1.32 |
| H23L16 | 2.95e6 | 5.87e-3 | 1.99 |
| H24L16 | 2.3e6 | 5.72e-3 | 2.49 |

### Results - Table 15

Results shown are from a single experiment.

| Antibody | ka (1/Ms) | kd (1/s) | KD (nM) |
|---|---|---|---|
| H1L11 | 1.19E6 | 2.19E-2 | 18.4 |
| H6L13 | 1.91 E6 | 1.55E-2 | 8.1 |
| HcLc | 2.32E6 | 3.69E-3 | 1.59 |
| H22L16 | 5.69E6 | 7.59E-3 | 1.33 |
| H16L13 | 4.76E6 | 1.21 E-2 | 2.53 |
| H20L13 | 5.25E6 | 1.6E-2 | 3.06 |

### Results - Table 16

Results shown are from a single experiment. Data is off-rate ranking based on supernant material from small-scale transient transfection

| Antibody | Off-rate: kd (1/s) |
|---|---|
| H23Lc | 3.42E-03 |
| H24Lc | 3.83E-03 |
| HcLc | 3.92E-03 |
| H22Lc | 4.67E-03 |
| H20Lc | 5.07E-03 |
| H16L18 | 5.66E-03 |
| H21Lc | 5.69E-03 |
| H16L16 | 5.77E-03 |
| H24L16 | 5.85E-03 |
| H19Lc | 5.95E-03 |
| H23L16 | 6.06E-03 |
| H20L16 | 7.18E-03 |
| H22L16 | 7.78E-03 |
| H19L16 | 8.73E-03 |
| H23L13 | 9.56E-03 |
| H24L13 | 9.96E-03 |
| H20L 13 | 1.20E-02 |
| H21 L13 | 1.20E-02 |
| H19L13 | 1.30E-02 |
| H16L13 | 1.38E-02 |
| H1L11 | 1.96E-02 |

**Table 17 Results are from 12 independent runs. Data shown is the mean and standard deviation of the 12 runs**

| **Antibody** | ***Ka*** | ***kd*** | **KD (nM)** |
|---|---|---|---|
| H20L16 | 5.43e6 (1.77e5) | 1.02e-2 (1.56e-3) | 1.86(0.16) |
| HcLc | 3.92e6(4.74e5) | 7.54e-3(1.90e-3) | 1.90(0.18) |

### 10.2 ELISA

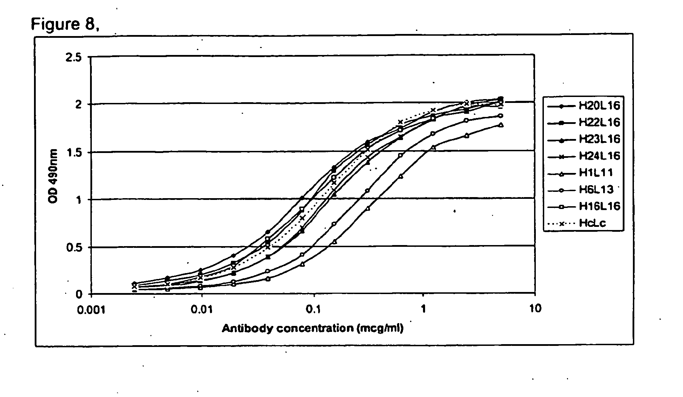

Based on the methodologies described in Example 3, additional data on the NOGO antibodies is shown in Figures 8-11. In some of these experiments, different concentrations of coating antigen were used (0.1-1.0mcg/ml)

Results - Figure 8. NOGO antigen coated at 1.0mcg/ml

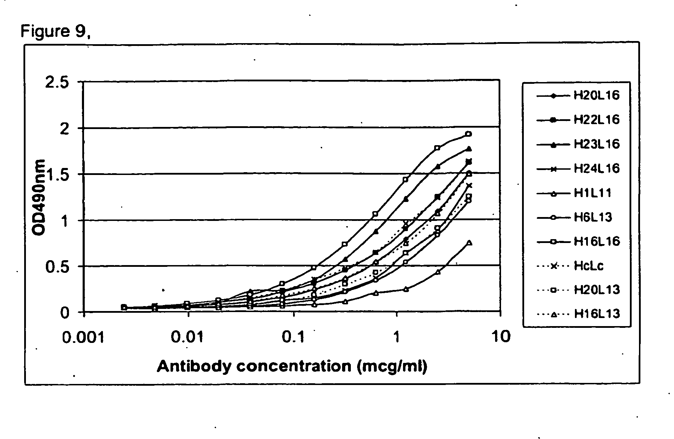

Results - Figure 9. NOGO 5+6 antigen coated at 0.1 mcg/ml

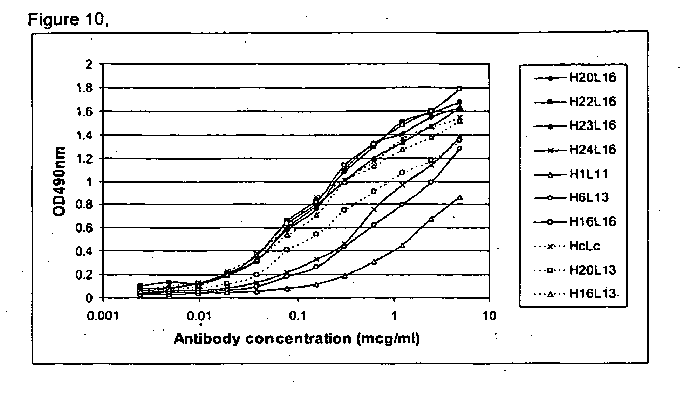

Results - Figure 10. NOGO antigen coated at 0.5mcg/ml

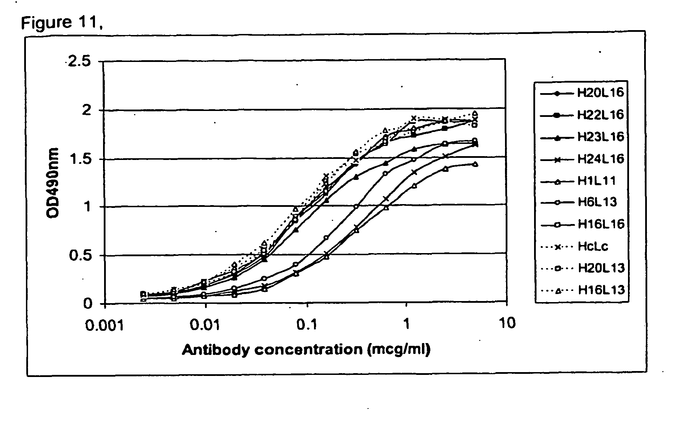

Results - Figure 11. NOGO antigen coated at 1.0mcg/ml

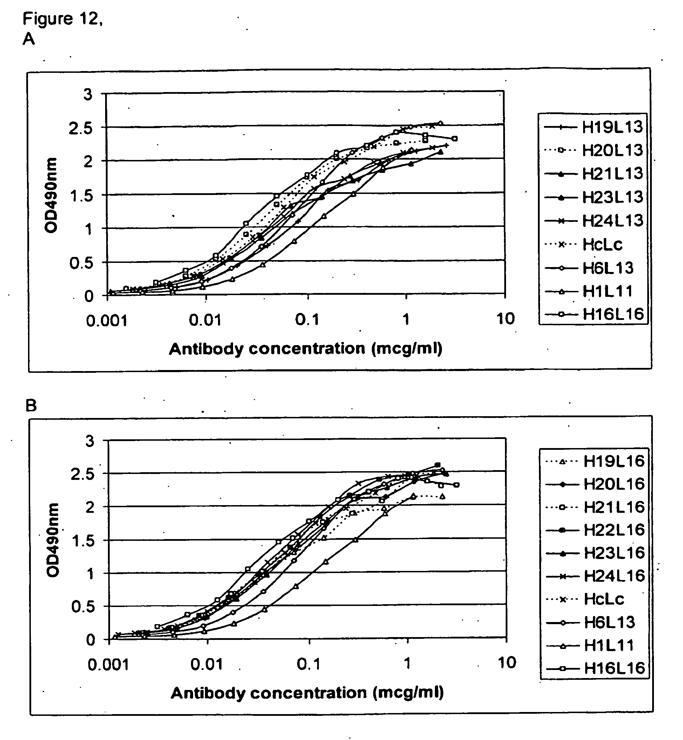

Results: Figure 12A and B, GST-human NOGO A56 antigen coated at 1.0mcg/ml. Binding ELISA of supernatant material from small scale transient transfection

### 10.3 Intracellular FACS

Based on the methodologies described in Example 9, additional FACS data is shown in Figure 13. Additional data is shown in Figure 14A to C. Figure 14A represents a single data point for each sample. Figures 14A and 14B represent the average of duplicate samples.

### Example 11, Reverse format BiaCore Analysis of humanised Anti NOGO Monoclonal Antibodies

The binding kinetics of the purified anti-NOGO monoclonal antibodies (mAb) to recombinantly expressed human NOGO-A (GST-human NOGO-A56) was analysed using the BiacoreT100. The chip was prepared as follows:

### Method

Biacore^{™} kinetic analysis of the humanised NOGO antbodies was carried out using Protein A capture of the candidate antibodies. Briefly, Protein A was immobilised on a CM5 chip by primary amine coupling, using standard coupling procedures, to densities around 3000-4000 resonance units (RU's) using the immobilisation wizard inherent in the machines software. Humanised antibody was then passed over the Protein A surface and captured to levels of around 200-400 RU's, after a period of stabilisation, human NOGO 5+6 GST was passed over the captured antibody surface at defined concentrations and binding sensorgrams were obtained. Acidic regeneration (100mM H₃PO₄ or 10mM Glycine pH 1.5) resulted in total removal of the captured antibody from the Protein A surface, and did not significantly reduce the surfaces binding capacity. All curves were double referenced against a buffer injection instead of human NOGO 5+6 GST and the data was fitted to the 1:1 binding model using the global fit parameters in Biacore evaluation software T100 v1.1. All experiments carried out on a T100 instrument.

**Table 18 - Results shown are the mean and standard deviation of 5 or 6 independent runs**

| **Antibody** | ***Ka*** | ***kd*** | **KD (nM)** |
|---|---|---|---|
| H20L16 | 1.01e6(1.35e5) | 3.13e-4(2.79e-5) | 0.31(0.036) |
| HcLc | 1.38e6(3.70e5) | 5.69e-4(1.54e-4) | 0.41(0.062) |
| 11C7 | 2.85e5(4.06e4) | 1.17e-4(1.22e-5) | 0.42(0.065) |

**NOGO antibody sequences Summary (Table 12)**

| Description | Sequence identifier (SEQ.I.D.NO) | |
|---|---|---|
| | amino acid sequence | Polynucleotide sequence |
| 2A10, CDR-H1 | 1 | - |
| 2A10, CDR-H2 | 2 | - |
| 2A10, CDR-H3 | 13 | - |
| 2A10, CDR-L1 | 4 | - |
| 2A10, CDR-L2 | 5 | - |
| 2A10, CDR-L3 | 6 | - |
| 2A10, VH (murine) | 7 | 41 |
| 2A10, VL (murine) | 8 | 42 |
| Chimeric heavy chain Hc | 9 | 43 |
| Chimeric light chain Lc | 10 | 44 |
| 2A10 VH humanised construct H5 | 11 | 45 |
| 2A10 VH humanised construct H6 | 12 | 46 |
| 2A10 VH humanised construct H700 | 13 | 47 |
| 2A10 VH humanised construct H14 | 14 | 48 |
| 2A10 VH humanised construct H15 | 15 | 49 |
| 2A10 VH humanised construct H16 | 16 | 50 |
| 2A10 VH humanised construct H17 | 17 | 51 |
| 2A10 VH humanised construct H18 | 18 | 52 |
| 2A10 VL humanised construct L6 | 19 | 53 |
| 2A10 VL humanised construct L13 | 20 | 54 |
| 2A10 VL humanised construct L14 | 21 | 55 |
| 2A10 VL humanised construct L15 | 22 | 56 |
| 2A10 VL humanised construct L16 | 23 | 57 |
| 2A10 VL humanised construct L17 | 24 | 58 |
| 2A10 VL humanised construct L18 | 25 | 59 |
| 2A10 heavy chain humanised construct H5 | 26 | 60 |
| 2A10 heavy chain humanised construct H6 | 27 | 61 |
| 2A10 heavy chain humanised construct H700 | 28 | 62 |
| 2A10 heavy chain humanised construct H14 | 29 | 63 |
| 2A10 heavy chain humanised construct H15 | 30 | 64 |
| 2A10 heavy chain humanised construct H16 | 31 | 65 |
| 2A10 heavy chain humanised construct H17 | 32 | 66 |
| 2A10 heavy chain humanised construct H18 | 33 | 67 |
| 2A10 light chain humanised construct L6 | 34 | 68 |
| 2A10 light chain humanised construct L13 | 35 | 69 |
| 2A10 liqht chain humanised construct L14 | 36 | 70 |
| 2A10 light chain humanised construct L15 | 37 | 71 |
| 2A10 light chain humanised construct L16 | 38 | 72 |
| 2A10 light chain humanised construct L17 | 39 | 73 |
| 2A10 light chain humanised construct L18 | 40 | 74 |
| Campath leader sequence | 75 | - |
| Amino acids 586-785 of human NOGO A (NOGO-A56) | 76 | - |
| 2A10 VH humanised construct H1 | 77 | 81 |
| 2A10 VL humanised construct L11 | 78 | 82 |
| 2A10 heavy chain humanised construct H1 | 79 | 83 |
| 2A10 light chain humanised construct L11 | 80 | 84 |
| 2A10 VH humanised construct H19 | 85 | 99 |
| 2A10 VH humanised construct H20 | 86 | 100 |
| 2A10 VH humanised construct H21 | 87 | 101 |
| 2A10 VH humanised construct H22 | 88 | 102 |
| 2A10 VH humanised construct H23 | 89 | 103 |
| 2A10 VH humanised construct H24 | 90 | 104 |
| 2A10 VH humanised construct H25 | 91 | 105 |
| 2A10 heavy chain humanized construct H19 | 92 | 106 |
| 2A10 heavy chain humanised construct H20 | 93 | 107 |
| 2A10 heavy chain humanised construct H21 | 94 | 108 |
| 2A10 heavy chain humanised construct H22 | 95 | 109 |
| 2A10 heavy chain humanised construct H23 | 96 | 110 |
| 2A10 heavy chain humanised construct H24 | 97 | 111 |
| 2A10 heavy chain humanised construct H25 | 98 | 112 |

### Sequences

SEQ ID 1: 2A10 CDR-H1
SYWMH

SEQ ID 2: 2A10 CDR-H2
NINPSNGGTNYNEKFKS

SEQ ID 3: 2A10 CDR-H3
GQGY

SEQ ID 4: 2A10 CDR-L1
RSSKSLLYKDGKTYLN

SEQ ID 5: 2A10 CDR-L2
LMSTRAS

SEQ ID 6: 2A10 CDR-L3
QQLVEYPLT

SEQ ID 7: 2A10, VH (murine)
QVQLQQPGTELVKPGASVKLSCKASGYTFTSYWMHWVKQRPGQGLEWIGNIN
PSNGGTNYNEKFKSKATLTVDKSSSTAYMQLSSLTSEDSAVYYCELGQGYWG
QGTTLTVSS

SEQ ID 8: 2A10, VL (murine)
DIVITQDELSNPVTSGESVSISCRSSKSLLYKDGKTYLNWFLQRPGQSPQLLIYL
MSTRASGVSDRFSGSGSGTDFTLEISRVKAEDVGVYYCQQLVEYPLTFGAGTK
LELK

SEQ ID 9: Chimeric heavy chain Hc
MGWSCIILFLVAAATGVHSQVQLQQPGTELVKPGASVKLSCKASGYTFTSYWM
HWVKQRPGQGLEWIGNINPSNGGTNYNEKFKSKATLTVDKSSSTAYMQLSSLT
SEDSAVYYCELGQGYWGQGTLVTVSSASTKGPSVFPLAPSSKSTSGGTAALG
CLVKDYFPEPVTVSWNSGALTSGVHTFPAVLQSSGLYSLSSVVTVPSSSLGTQ

TYICNVNHKPSNTKVDKKVEPKSCDKTHTCPPCPAPELAGAPSVFLFPPKPKDT
LMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLT
VDKSRWQQGNVFSCSVMHEALHNHYTQKSLSLSPGK

SEQ ID 10: Chimeric light chain Lc
MRCSLQFLGVLMFWISGVSGDIVITQDELSNPVTSGESVSISCRSSKSLLYKDG
KTYLNWFLQRPGQSPQLLIYLMSTRASGVSDRFSGSGSGTDFTLEISRVKAEDV
GVYYCQQLVEYPLTFGAGTKLELKRTVAAPSVFIFPPSDEQLKSGTASVVCLLN
NFYPREAKVQWKVDNALQSGNSQESVTEQDSKDSTYSLSSTLTLSKADYEKHK
VYACEVTHQGLSSPVTKSFNRGEC

SEQ ID 11: 2A10 VH humanised construct H5
QVQLVQSGAEVKKPGASVKVSCKASGYTFTSYWMHWVRQAPGQGLEWMGN
INPSNGGTNYNEKFKSRVTMTRDTSTSTAYMELSSLRSEDTAVYYCELGQGY
WGQGTLVTVSS

SEQ ID 12: 2A10 VH humanised construct H6
QVQLVQSGAEVKKPGASVKVSCKASGYTFTSYWMHWVRQAPGQGLEWIGNI
NPSNGGTNYNEKFKSRATMTRDTSTSTAYMELSSLRSEDTAVYYCELGQGY
WGQGTLVTVSS

SEQ ID 13: 2A10 VH humanised construct H700
QVQLVQSGAEVKKPGASVKVSCKASGYTFTSYWMHWVRQAPGQGLEWIGNI
NPSNGGTNYNEKFKSRATLTVDKSTSTAYMELSSLRSEDTAVYYCELGQGYW
GQGTLVTVSS

SEQ ID 14: 2A10 VH humanised construct H14
QVQLVQSGAEVVKPGASVKLSCKASGYTFTSYWMHWVKQAPGQGLEWIGNI
NPSNGGTNYNEKFKSRATLTRDTSTSTAYMELSSLRSEDTAVYYCELGQGYW
GQGTLVTVSS

SEQ ID 15: 2A10 VH humanised construct H15
QVQLVQSGAEVVKPGASVKLSCKASGYTFTSYWMHWVKQRPGQGLEWIGNIN
PSNGGTNYNEKFKSKATLTVDTSSSTAYMELSSLRSEDSAVYYCELGQGYWG
QGTLVTVSS

SEQ ID 16: 2A10 VH humanised construct H16

QVQLVQSGAEVKKPGASVKVSCKASGYTFTSYWMHWVKQRPGQGLEWIGNIN
PSNGGTNYNEKFKSKATLTVDKSTSTAYMELSSLRSEDTAVYYCELGQGYWG
QGTLVTVSS

SEQ ID 17: 2A10 VH humanised construct H17
QVQLVQSGAEVVKPGASVKLSCKASGYTFTSYWMHWVKQRPGQGLEWIGNIN
PSNGGTNYNEKFKSKATLTVDKSTSTAYMELSSLRSEDTAVYYCELGQGYWG
QGTLVTVSS

SEQ ID 18: 2A10 VH humanised construct H18
QVQLVQSGAEVVKPGASVKLSCKASGYTFTSYWMHWVKQRPGQGLEWIGNIN
PSNGGTNYNEKFKSKATLTVDKSSSTAYMELSSLRSEDSAVYYCELGQGYWG
QGTLVTVSS

SEQ ID 19: 2A10 VL humanised construct L6
DIVMTQSPLSLPVTPGEPASISCRSSKSLLYKDGKTYLNWFLQRPGQSPQLLIYL
MSTRASGVPDRFSGSGSGTDFTLKISRVEAEDVGVYYCQQLVEYPLTFGGGTK
VEIK

SEQ ID 20: 2A10 VL humanised construct L13
DIVMTQSPLSLPVTLGQPASISCRSSKSLLYKDGKTYLNWFQQRPGQSPQLLIY
LMSTRASGVPDRFSGGGSGTDFTLKISRVEAEDVGVYYCQQLVEYPLTFGQGT
KLEIK

SEQ ID 21: 2A10 VL humanised construct L14
DIVMTQSPLSLPVTLGQPASISCRSSKSLLYKDGKTYLNWFLQRPGQSPQLLIYL
MSTRASGVPDRFSGGGSGTDFTLKISRVEAEDVGVYYCQQLVEYPLTFGQGTK
LEIK

SEQ ID 22: 2A10 VL humanised construct L15
DIVMTQSPLSLPVTLGQPASISCRSSKSLLYKDGKTYLNWFLQRPGQSPQLLIYL
MSTRASGVSDRFSGGGSGTDFTLKISRVEAEDVGVYYCQQLVEYPLTFGQGTK
LEIK

SEQ ID 23: 2A10 VL humanised construct L16




SEQ ID 24: 2A10 VL humanised construct L17
DIVMTQDPLSLPVTLGQPASISCRSSKSLLYKDGKTYLNWFQQRPGQSPQLLIY
LMSTRASGVSDRFSGSGSGTDFTLKISRVEAEDVGVYYCQQLVEYPLTFGQGT
KLEIK

SEQ ID 25: 2A10 VL humanised construct L18
DIVMTQDPLSNPVTLGQPVSISCRSSKSLLYKDGKTYLNWFLQRPGQSPQLLIY
LMSTRASGVSDRFSGSGSGTDFTLKISRVEAEDVGVYYCQQLVEYPLTFGQGT
KLEIK

SEQ ID 26: 2A10 heavy chain humanised construct H5
MGWSCIILFLVATATGVHSQVQLVQSGAEVKKPGASVKVSCKASGYTFTSYWM
HWVRQAPGQGLEWMGNINPSNGGTNYNEKFKSRVTMTRDTSTSTAYMELSSL
RSEDTAVYYCELGQGYWGQGTLVTVSSASTKGPSVFPLAPSSKSTSGGTAAL
GCLVKDYFPEPVTVSWNSGALTSGVHTFPAVLQSSGLYSLSSVVTVPSSSLGT
QTYICNVNHKPSNTKVDKKVEPKSCDKTHTCPPCPAPELAGAPSVFLFPPKPKD
TLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKL
TVDKSRWQQGNVFSCSVMHEALHNHYTQKSLSLSPGK

SEQ ID 27: 2A10 heavy chain humanised construct H6
MGWSCIILFLVATATGVHSQVQLVQSGAEVKKPGASVKVSCKASGYTFTSYWM
HWVRQAPGQGLEWIGNINPSNGGTNYNEKFKSRATMTRDTSTSTAYMELSSL
RSEDTAVYYCELGQGYWGQGTLVTVSSASTKGPSVFPLAPSSKSTSGGTAAL
GCLVKDYFPEPVTVSWNSGALTSGVHTFPAVLQSSGLYSLSSVVTVPSSSLGT
QTYICNVNHKPSNTKVDKKVEPKSCDKTHTCPPCPAPELAGAPSVFLFPPKPKD
TLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKL
TVDKSRWQQGNVFSCSVMHEALHNHYTQKSLSLSPGK

SEQ ID 28: 2A10 heavy chain humanised construct H700
MGWSCIILFLVATATGVHSQVQLVQSGAEVKKPGASVKVSCKASGYTFTSYWM
HWVRQAPGQGLEWIGNINPSNGGTNYNEKFKSRATLTVDKSTSTAYMELSSLR
SEDTAVYYCELGQGYWGQGTLVTVSSASTKGPSVFPLAPSSKSTSGGTAALG
CLVKDYFPEPVTVSWNSGALTSGVHTFPAVLQSSGLYSLSSVVTVPSSSLGTQ
TYICNVNHKPSNTKVDKKVEPKSCDKTHTCPPCPAPELAGAPSVFLFPPKPKDT
LMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLT
VDKSRWQQGNVFSCSVMHEALHNHYTQKSLSLSPGK

SEQ ID 29: 2A10 heavy chain humanised construct H14
MGWSCIILFLVATATGVHSQVQLVQSGAEVVKPGASVKLSCKASGYTFTSYWM
HWVKQAPGQGLEWIGNINPSNGGTNYNEKFKSRATLTRDTSTSTAYMELSSLR
SEDTAVYYCELGQGYWGQGTLVTVSSASTKGPSVFPLAPSSKSTSGGTAALG
CLVKDYFPEPVTVSWNSGALTSGVHTFPAVLQSSGLYSLSSVVTVPSSSLGTQ
TYICNVNHKPSNTKVDKKVEPKSCDKTHTCPPCPAPELAGAPSVFLFPPKPKDT
LMISRTPEVTCVVVDVSHEDPEVKFNWYVDGVEVHNAKTKPREEQYNSTYRVV

SVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLT
VDKSRWQQGNVFSCSVMHEALHNHYTQKSLSLSPGK

SEQ ID 30: 2A10 heavy chain humanised construct H15
MGWSCIILFLVATATGVHSQVQLVQSGAEVVKPGASVKLSCKASGYTFTSYWM
HWVKQRPGQGLEWIGNINPSNGGTNYNEKFKSKATLTVDTSSSTAYMELSSLR
SEDSAVYYCELGQGYWGQGTLVTVSSASTKGPSVFPLAPSSKSTSGGTAALG
CLVKDYFPEPVTVSWNSGALTSGVHTFPAVLQSSGLYSLSSVVTVPSSSLGTQ
TYICNVNHKPSNTKVDKKVEPKSCDKTHTCPPCPAPELAGAPSVFLFPPKPKDT
LMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLT
VDKSRWQQGNVFSCSVMHEALHNHYTQKSLSLSPGK

SEQ ID 31: 2A10 heavy chain humanised construct H16
MGWSCIILFLVATATGVHSQVQLVQSGAEVKKPGASVKVSCKASGYTFTSYWM
HWVKQRPGQGLEWIGNINPSNGGTNYNEKFKSKATLTVDKSTSTAYMELSSLR
SEDTAVYYCELGQGYWGQGTLVTVSSASTKGPSVFPLAPSSKSTSGGTAALG
CLVKDYFPEPVTVSWNSGALTSGVHTFPAVLQSSGLYSLSSVVTVPSSSLGTQ
TYICNVNHKPSNTKVDKKVEPKSCDKTHTCPPCPAPELAGAPSVFLFPPKPKDT
LMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLT
VDKSRWQQGNVFSCSVMHEALHNHYTQKSLSLSPGK

SEQ ID 32: 2A10 heavy chain humanised construct H17
MGWSCIILFLVATATGVHSQVQLVQSGAEVVKPGASVKLSCKASGYTFTSYWM
HWVKQRPGQGLEWIGNINPSNGGTNYNEKFKSKATLTVDKSTSTAYMELSSLR
SEDTAVYYCELGQGYWGQGTLVTVSSASTKGPSVFPLAPSSKSTSGGTAALG
CLVKDYFPEPVTVSWNSGALTSGVHTFPAVLQSSGLYSLSSVVTVPSSSLGTQ
TYICNVNHKPSNTKVDKKVEPKSCDKTHTCPPCPAPELAGAPSVFLFPPKPKDT
LMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLT
VDKSRWQQGNVFSCSVMHEALHNHYTQKSLSLSPGK

SEQ ID 33: 2A10 heavy chain humanised construct H18
MGWSCIILFLVATATGVHSQVQLVQSGAEVVKPGASVKLSCKASGYTFTSYWM
HWVKQRPGQGLEWIGNINPSNGGTNYNEKFKSKATLTVDKSSSTAYMELSSLR
SEDSAVYYCELGQGYWGQGTLVTVSSASTKGPSVFPLAPSSKSTSGGTAALG
CLVKDYFPEPVTVSWNSGALTSGVHTFPAVLQSSGLYSLSSVVTVPSSSLGTQ
TYICNVNHKPSNTKVDKKVEPKSCDKTHTCPPCPAPELAGAPSVFLFPPKPKDT
LMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDE

LTKNQVSLTCLVKGFYPSDIAVEWESNGQPENNYKTTPPVLDSDGSFFLYSKLT
VDKSRWQQGNVFSCSVMHEALHNHYTQKSLSLSPGK

SEQ ID 34: 2A10 light chain humanised construct L6
MGWSCIILFLVATATGVHSDIVMTQSPLSLPVTPGEPASISCRSSKSLLYKDGKT
YLNWFLQRPGQSPQLLIYLMSTRASGVPDRFSGSGSGTDFTLKISRVEAEDVG
VYYCQQLVEYPLTFGGGTKVEIKRTVAAPSVFIFPPSDEQLKSGTASVVCLLNNF
YPREAKVQWKVDNALQSGNSQESVTEQDSKDSTYSLSSTLTLSKADYEKHKVY
ACEVTHQGLSSPVTKSFNRGEC

SEQ ID 35: 2A10 light chain humanised construct L13
MGWSCIILFLVATATGVHSDIVMTQSPLSLPVTLGQPASISCRSSKSLLYKDGKT
YLNWFQQRPGQSPQLLIYLMSTRASGVPDRFSGGGSGTDFTLKISRVEAEDVG
VYYCQQLVEYPLTFGQGTKLEIKRTVAAPSVFIFPPSDEQLKSGTASVVCLLNNF
YPREAKVQWKVDNALQSGNSQESVTEQDSKDSTYSLSSTLTLSKADYEKHKVY
ACEVTHQGLSSPVTKSFNRGEC

SEQ ID 36: 2A10 light chain humanised construct L14
MGWSCIILFLVATATGVHSDIVMTQSPLSLPVTLGQPASISCRSSKSLLYKDGKT
YLNWFLQRPGQSPQLLIYLMSTRASGVPDRFSGGGSGTDFTLKISRVEAEDVG
VYYCQQLVEYPLTFGQGTKLEIKRTVAAPSVFIFPPSDEQLKSGTASVVCLLNNF
YPREAKVQWKVDNALQSGNSQESVTEQDSKDSTYSLSSTLTLSKADYEKHKVY
ACEVTHQGLSSPVTKSFNRGEC

SEQ ID 37: 2A10 light chain humanised construct L15
MGWSCIILFLVATATGVHSDIVMTQSPLSLPVTLGQPASISCRSSKSLLYKDGKT
YLNWFLQRPGQSPQLLIYLMSTRASGVSDRFSGGGSGTDFTLKISRVEAEDVG
VYYCQQLVEYPLTFGQGTKLEIKRTVAAPSVFIFPPSDEQLKSGTASVVCLLNNF
YPREAKVQWKVDNALQSGNSQESVTEQDSKDSTYSLSSTLTLSKADYEKHKVY
ACEVTHQGLSSPVTKSFNRGEC

SEQ ID 38: 2A10 light chain humanised construct L16
MGWSCIILFLVATATGVHSDIVMTQSPLSNPVTLGQPVSISCRSSKSLLYKDGKT
YLNWFLQRPGQSPQLLIYLMSTRASGVPDRFSGGGSGTDFTLKISRVEAEDVG
VYYCQQLVEYPLTFGQGTKLEIKRTVAAPSVFIFPPSDEQLKSGTASVVCLLNNF
YPREAKVQWKVDNALQSGNSQESVTEQDSKDSTYSLSSTLTLSKADYEKHKVY
ACEVTHQGLSSPVTKSFNRGEC

SEQ ID 39: 2A10 light chain humanised construct L17
MGWSCIILFLVATATGVHSDIVMTQDPLSLPVTLGQPASISCRSSKSLLYKDGKT
YLNWFQQRPGQSPQLLIYLMSTRASGVSDRFSGSGSGTDFTLKISRVEAEDVG
VYYCQQLVEYPLTFGQGTKLEIKRTVAAPSVFIFPPSDEQLKSGTASVVCLLNNF
YPREAKVQWKVDNALQSGNSQESVTEQDSKDSTYSLSSTLTLSKADYEKHKVY
ACEVTHQGLSSPVTKSFNRGEC

SEQ ID 40: 2A10 light chain humanised construct L18
MGWSCIILFLVATATGVHSDIVMTQDPLSNPVTLGQPVSISCRSSKSLLYKDGKT
YLNWFLQRPGQSPQLLIYLMSTRASGVSDRFSGSGSGTDFTLKISRVEAEDVG
VYYCQQLVEYPLTFGQGTKLEIKRTVAAPSVFIFPPSDEQLKSGTASVVCLLNNF
YPREAKVQWKVDNALQSGNSQESVTEQDSKDSTYSLSSTLTLSKADYEKHKVY
ACEVTHQGLSSPVTKSFNRGEC

SEQ ID 41: PN encoding 2A10, VH (murine) SEQ ID: 7
CAGGTCCAACTGCAGCAGCCTGGGACTGAACTGGTGAAGCCTGGGGCTTC
AGTGAAGCTGTCCTGCAAGGCTTCTGGCTACACCTTCACCAGCTACTGGAT
GCACTGGGTGAAGCAGAGGCCTGGACAAGGCCTTGAGTGGATTGGAAATAT
TAATCCTAGCAATGGTGGTACTAACTACAATGAGAAGTTCAAGAGCAAGGCC
ACACTGACTGTAGACAAATCCTCCAGCACAGCCTACATGCAGCTCAGCAGC
CTGACATCTGAGGACTCTGCGGTCTATTATTGTGAACTGGGACAGGGCTAC
TGGGGCCAAGGCACCACTCTCACAGTCTCCTCA

SEQ ID 42: PN encoding 2A10, VL (murine) SEQ ID: 8
GATATTGTGATAACCCAGGATGAACTCTCCAATCCTGTCACTTCTGGAGAAT
CAGTTTCCATCTCCTGCAGGTCTAGTAAGAGTCTCCTATATAAGGATGGGAA
GACATACTTGAATTGGTTTCTGCAGAGACCAGGACAATCTCCTCAGCTCCTG
ATCTATTTGATGTCCACCCGTGCATCAGGAGTCTCAGACCGGTTTAGTGGCA
GTGGGTCAGGAACAGATTTCACCCTGGAAATCAGTAGAGTGAAGGCTGAGG
ATGTGGGTGTGTATTACTGTCAACAACTTGTAGAGTATCCGCTCACGTTCGG
TGCTGGGACCAAGCTGGAGCTGAAA

SEQ ID 43: PN encoding Chimeric heavy chain Hc SEQ ID: 9
ATGGGATGGAGCTGTATCATCCTCTTTTTGGTAGCAGCAGCTACAGGTGTCC
ACTCCCAGGTCCAACTGCAGCAGCCTGGGACTGAACTGGTGAAGCCTGGG
GCTTCAGTGAAGCTGTCCTGCAAGGCTTCTGGCTACACCTTCACCAGCTACT
GGATGCACTGGGTGAAGCAGAGGCCTGGACAAGGCCTTGAGTGGATTGGA
AATATTAATCCTAGCAATGGTGGTACTAACTACAATGAGAAGTTCAAGAGCA
AGGCCACACTGACTGTAGACAAATCCTCCAGCACAGCCTACATGCAGCTCA
GCAGCCTGACATCTGAGGACTCTGCGGTCTATTATTGTGAACTGGGACAGG
GCTACTGGGGCCAAGGCACACTAGTCACAGTCTCCTCAGCCTCCACCAAGG
GCCCATCGGTCTTCCCCCTGGCACCCTCCTCCAAGAGCACCTCTGGGGGC
ACAGCGGCCCTGGGCTGCCTGGTCAAGGACTACTTCCCCGAACCGGTGAC
GGTGTCGTGGAACTCAGGCGCCCTGACCAGCGGCGTGCACACCTTCCCGG
CTGTCCTACAGTCCTCAGGACTCTACTCCCTCAGCAGCGTGGTGACCGTGC
CCTCCAGCAGCTTGGGCACCCAGACCTACATCTGCAACGTGAATCACAAGC
CCAGCAACACCAAGGTGGACAAGAAAGTTGAGCCCAAATCTTGTGACAAAA
CTCACACATGCCCACCGTGCCCAGCACCTGAACTCGCGGGGGCACCGTCA
GTCTTCCTCTTCCCCCCAAAACCCAAGGACACCCTCATGATCTCCCGGACC
CCTGAGGTCACATGCGTGGTGGTGGACGTGAGCCACGAAGACCCTGAGGT
CAAGTTCAACTGGTACGTGGACGGCGTGGAGGTGCATAATGCCAAGACAAA
GCCGCGGGAGGAGCAGTACAACAGCACGTACCGTGTGGTCAGCGTCCTCA
CCGTCCTGCACCAGGACTGGCTGAATGGCAAGGAGTACAAGTGCAAGGTCT
CCAACAAAGCCCTCCCAGCCCCCATCGAGAAAACCATCTCCAAAGCCAAAG

GGCAGCCCCGAGAACCACAGGTGTACACCCTGCCCCCATCCCGGGATGAG
CTGACCAAGAACCAGGTCAGCCTGACCTGCCTGGTCAAAGGCTTCTATCCC
AGCGACATCGCCGTGGAGTGGGAGAGCAATGGGCAGCCGGAGAACAACTA
CAAGACCACGCCTCCCGTGCTGGACTCCGACGGCTCCTTCTTCCTCTACAG
CAAGCTCACCGTGGACAAGAGCAGGTGGCAGCAGGGGAACGTCTTCTCAT
GCTCCGTGATGCATGAGGCTCTGCACAACCACTACACGCAGAAGAGCCTCT
CCCTGTCTCCGGGTAAATGA

SEQ ID 44: PN encoding Chimeric light chain Lc SEQ ID: 10
ATGAGGTGCTCTCTTCAGTTTCTGGGGGTGCTTATGTTCTGGATCTCTGGAG
TCAGTGGGGATATTGTGATAACCCAGGATGAACTCTCCAATCCTGTCACTTC
TGGAGAATCAGTTTCCATCTCCTGCAGGTCTAGTAAGAGTCTCCTATATAAG
GATGGGAAGACATACTTGAATTGGTTTCTGCAGAGACCAGGACAATCTCCTC
AGCTCCTGATCTATTTGATGTCCACCCGTGCATCAGGAGTCTCAGACCGGTT
TAGTGGCAGTGGGTCAGGAACAGATTTCACCCTGGAAATCAGTAGAGTGAA
GGCTGAGGATGTGGGTGTGTATTACTGTCAACAACTTGTAGAGTATCCGCTC
ACGTTCGGTGCTGGGACCAAGCTGGAGCTGAAACGTACGGTGGCTGCACC
ATCTGTCTTCATCTTCCCGCCATCTGATGAGCAGTTGAAATCTGGAACTGCC
TCTGTTGTGTGCCTGCTGAATAACTTCTATCCCAGAGAGGCCAAAGTACAGT
GGAAGGTGGACAACGCCCTCCAATCGGGTAACTCCCAGGAGAGTGTCACA
GAGCAGGACAGCAAGGACAGCACCTACAGCCTCAGCAGCACCCTGACGCT
GAGCAAAGCAGACTACGAGAAACACAAAGTCTACGCCTGCGAAGTCACCCA
TCAGGGCCTGAGCTCGCCCGTCACAAAGAGCTTCAACAGGGGAGAGTGTTA
G

SEQ ID 45: PN encoding 2A10 VH humanised construct H5 SEQ ID: 11
CAGGTGCAGCTGGTGCAGTCTGGGGCTGAGGTGAAGAAGCCTGGGGCCTC
AGTGAAGGTTTCCTGCAAGGCATCTGGATACACCTTCACCAGCTACTGGAT
GCACTGGGTGCGACAGGCCCCTGGACAAGGGCTTGAGTGGATGGGAAATA
TTAATCCTAGCAATGGTGGTACTAACTACAATGAGAAGTTCAAGAGCAGAGT
CACCATGACCAGGGACACGTCCACGAGCACAGCCTACATGGAGCTGAGCA
GCCTGAGATCTGAGGACACGGCCGTGTATTACTGTGAACTGGGACAGGGCT
ACTGGGGCCAGGGAACACTAGTCACAGTCTCCTCA

SEQ ID 46: PN encoding 2A10 VH humanised construct H6 SEQ ID: 12
CAGGTGCAGCTGGTGCAGTCTGGGGCTGAGGTGAAGAAGCCTGGGGCCTC
AGTGAAGGTTTCCTGCAAGGCATCTGGATACACCTTCACCAGCTACTGGAT
GCACTGGGTGCGACAGGCCCCTGGACAAGGGCTTGAGTGGATCGGAAATA
TTAATCCTAGCAATGGTGGTACTAACTACAATGAGAAGTTCAAGAGCAGAGC
CACCATGACCAGGGACACGTCCACGAGCACAGCCTACATGGAGCTGAGCA
GCCTGAGATCTGAGGACACGGCCGTGTATTACTGTGAACTGGGACAGGGCT
ACTGGGGCCAGGGAACACTAGTCACAGTCTCCTCA

SEQ ID 47: PN encoding 2A10 VH humanised construct H700 SEQ ID: 13

CAGGTGCAGCTGGTGCAGTCTGGGGCTGAGGTGAAGAAGCCTGGGGCCTC
AGTGAAGGTTTCCTGCAAGGCATCTGGATACACCTTCACCAGCTACTGGAT
GCACTGGGTGCGACAGGCCCCTGGACAAGGGCTTGAGTGGATCGGAAATA
TTAATCCTAGCAATGGTGGTACTAACTACAATGAGAAGTTCAAGAGCAGAGC
CACCCTGACCGTAGACAAATCCACGAGCACAGCCTACATGGAGCTGAGCAG
CCTGAGATCTGAGGACACGGCCGTGTATTACTGTGAACTGGGACAGGGCTA
CTGGGGCCAGGGAACACTAGTCACAGTCTCCTCA

SEQ ID 48: PN encoding 2A10 VH humanised construct H14 SEQ ID: 14
CAGGTGCAGCTGGTGCAGTCTGGGGCTGAGGTGGTCAAGCCTGGGGCCTC
AGTGAAGCTCTCCTGCAAGGCATCTGGATACACCTTCACCAGCTACTGGAT
GCACTGGGTGAAACAGGCCCCTGGACAAGGGCTTGAGTGGATCGGAAATAT
TAATCCTAGCAATGGTGGTACTAACTACAATGAGAAGTTCAAGAGCAGAGCC
ACCCTCACCAGGGACACGTCCACGAGCACAGCCTACATGGAGCTGAGCAG
CCTGAGATCTGAGGACACGGCCGTGTATTACTGTGAACTGGGACAGGGCTA
CTGGGGCCAGGGAACACTAGTCACAGTCTCCTCA

SEQ ID 49: PN encoding 2A10 VH humanised construct H15 SEQ ID: 15
CAGGTGCAGCTGGTGCAGTCTGGGGCTGAGGTGGTCAAGCCTGGGGCCTC
AGTGAAGCTCTCCTGCAAGGCATCTGGATACACCTTCACCAGCTACTGGAT
GCACTGGGTGAAACAGCGACCTGGACAAGGGCTTGAGTGGATCGGAAATAT
TAATCCTAGCAATGGTGGTACTAACTACAATGAGAAGTTCAAGAGCAAAGCC
ACCCTCACCGTCGACACGTCCAGCAGCACAGCCTACATGGAGCTGAGCAG
CCTGAGATCTGAGGACAGCGCCGTGTATTACTGTGAACTGGGACAGGGCTA
CTGGGGCCAGGGAACACTAGTCACAGTCTCCTCA

SEQ ID 50: PN encoding 2A10 VH humanised construct H16 SEQ ID: 16
CAGGTGCAGCTGGTGCAGTCTGGGGCTGAGGTGAAGAAGCCTGGGGCCTC
AGTGAAGGTTTCCTGCAAGGCATCTGGATACACCTTCACCAGCTACTGGAT
GCACTGGGTGAAACAGCGACCTGGACAAGGGCTTGAGTGGATCGGAAATAT
TAATCCTAGCAATGGTGGTACTAACTACAATGAGAAGTTCAAGAGCAAAGCC
ACCCTCACCGTCGACAAATCCACGAGCACAGCCTACATGGAGCTGAGCAGC
CTGAGATCTGAGGACACGGCCGTGTATTACTGTGAACTGGGACAGGGCTAC
TGGGGCCAGGGAACACTAGTCACAGTCTCCTCA

SEQ ID 51: PN encoding 2A10 VH humanised construct H17 SEQ ID: 17
CAGGTGCAGCTGGTGCAGTCTGGGGCTGAGGTGGTCAAGCCTGGGGCCTC
AGTGAAGCTCTCCTGCAAGGCATCTGGATACACCTTCACCAGCTACTGGAT
GCACTGGGTGAAACAGCGACCTGGACAAGGGCTTGAGTGGATCGGAAATAT
TAATCCTAGCAATGGTGGTACTAACTACAATGAGAAGTTCAAGAGCAAAGCC
ACCCTCACCGTCGACAAATCCACGAGCACAGCCTACATGGAGCTGAGCAGC
CTGAGATCTGAGGACACGGCCGTGTATTACTGTGAACTGGGACAGGGCTAC
TGGGGCCAGGGAACACTAGTCACAGTCTCCTCA

SEQ ID 52: PN encoding 2A10 VH humanised construct H18 SEQ ID: 18

CAGGTGCAGCTGGTGCAGTCTGGGGCTGAGGTGGTCAAGCCTGGGGCCTC
AGTGAAGCTCTCCTGCAAGGCATCTGGATACACCTTCACCAGCTACTGGAT
GCACTGGGTGAAACAGCGACCTGGACAAGGGCTTGAGTGGATCGGAAATAT
TAATCCTAGCAATGGTGGTACTAACTACAATGAGAAGTTCAAGAGCAAAGCC
ACCCTCACCGTCGACAAATCCAGCAGCACAGCCTACATGGAGCTGAGCAGC
CTGAGATCTGAGGACAGCGCCGTGTATTACTGTGAACTGGGACAGGGCTAC
TGGGGCCAGGGAACACTAGTCACAGTCTCCTCA

SEQ ID 53: PN encoding 2A10 VL humanised construct L6 SEQ ID: 19
GATATTGTGATGACTCAGTCTCCACTCTCCCTGCCCGTCACCCCTGGAGAG
CCGGCCTCCATCTCCTGCAGGTCTAGTAAGAGTCTCCTATATAAGGATGGG
AAGACATACTTGAATTGGTTCCTGCAGCGACCAGGGCAGTCTCCACAGCTC
CTGATCTATTTGATGTCCACCCGTGCATCAGGGGTCCCTGACAGGTTCAGT
GGCAGTGGATCAGGCACAGATTTTACACTGAAAATCAGCAGAGTGGAGGCT
GAGGATGTTGGGGTTTATTACTGCCAACAACTTGTAGAGTATCCGCTCACGT
TCGGCGGAGGGACCAAGGTGGAGATCAAA

SEQ ID 54: PN encoding 2A10 VL humanised construct L13 SEQ ID: 20
GATATTGTGATGACCCAGTCTCCACTCTCCCTGCCCGTCACCCTTGGACAG
CCGGCCTCCATCTCCTGCAGGTCTAGTAAGAGTCTCCTATATAAGGATGGG
AAGACATACTTGAATTGGTTTCAGCAGAGGCCAGGCCAATCTCCACAGCTC
CTAATTTATTTGATGTCCACCCGTGCATCTGGGGTCCCAGACAGATTCAGCG
GCGGTGGGTCAGGCACTGATTTCACACTGAAAATCAGCAGGGTGGAGGCT
GAGGATGTTGGGGTTTATTACTGCCAACAACTTGTAGAGTATCCGCTCACGT
TTGGCCAGGGGACCAAGCTGGAGATCAAA

SEQ ID 55: PN encoding 2A10 VL humanised construct L14 SEQ ID: 21
GATATTGTGATGACCCAGTCTCCACTCTCCCTGCCCGTCACCCTTGGACAG
CCGGCCTCCATCTCCTGCAGGTCTAGTAAGAGTCTCCTATATAAGGATGGG
AAGACATACTTGAATTGGTTTCTCCAGAGGCCAGGCCAATCTCCACAGCTCC
TAATTTATTTGATGTCCACCCGTGCATCTGGGGTCCCAGACAGATTCAGCGG
CGGTGGGTCAGGCACTGATTTCACACTGAAAATCAGCAGGGTGGAGGCTGA
GGATGTTGGGGTTTATTACTGCCAACAACTTGTAGAGTATCCGCTCACGTTT
GGCCAGGGGACCAAGCTGGAGATCAAA

SEQ ID 56: PN encoding 2A10 VL humanised construct L15 SEQ ID: 22
GATATTGTGATGACCCAGTCTCCACTCTCCCTGCCCGTCACCCTTGGACAG
CCGGCCTCCATCTCCTGCAGGTCTAGTAAGAGTCTCCTATATAAGGATGGG
AAGACATACTTGAATTGGTTTCTCCAGAGGCCAGGCCAATCTCCACAGCTCC
TAATTTATTTGATGTCCACCCGTGCATCTGGGGTCAGCGACAGATTCAGCGG
CGGTGGGTCAGGCACTGATTTCACACTGAAAATCAGCAGGGTGGAGGCTGA
GGATGTTGGGGTTTATTACTGCCAACAACTTGTAGAGTATCCGCTCACGTTT
GGCCAGGGGACCAAGCTGGAGATCAAA

SEQ ID 57: PN encoding 2A10 VL humanised construct L16 SEQ ID: 23

GATATTGTGATGACCCAGTCTCCACTCTCCAACCCCGTCACCCTTGGACAG
CCGGTCTCCATCTCCTGCAGGTCTAGTAAGAGTCTCCTATATAAGGATGGGA
AGACATACTTGAATTGGTTTCTCCAGAGGCCAGGCCAATCTCCACAGCTCCT
AATTTATTTGATGTCCACCCGTGCATCTGGGGTCCCAGACAGATTCAGCGG
CGGTGGGTCAGGCACTGATTTCACACTGAAAATCAGCAGGGTGGAGGCTGA
GGATGTTGGGGTTTATTACTGCCAACAACTTGTAGAGTATCCGCTCACGTTT
GGCCAGGGGACCAAGCTGGAGATCAAA

SEQ ID 58: PN encoding 2A10 VL humanised construct L17 SEQ ID: 24
GATATTGTGATGACCCAGGACCCACTCTCCCTGCCCGTCACCCTTGGACAG
CCGGCCTCCATCTCCTGCAGGTCTAGTAAGAGTCTCCTATATAAGGATGGG
AAGACATACTTGAATTGGTTTCAGCAGAGGCCAGGCCAATCTCCACAGCTC
CTAATTTATTTGATGTCCACCCGTGCATCTGGGGTCAGCGACAGATTCAGCG
GCAGCGGGTCAGGCACTGATTTCACACTGAAAATCAGCAGGGTGGAGGCT
GAGGATGTTGGGGTTTATTACTGCCAACAACTTGTAGAGTATCCGCTCACGT
TTGGCCAGGGGACCAAGCTGGAGATCAAA

SEQ ID 59: PN encoding 2A10 VL humanised construct L18 SEQ ID: 25
GATATTGTGATGACCCAGGACCCACTCTCCAACCCCGTCACCCTTGGACAG
CCGGTCTCCATCTCCTGCAGGTCTAGTAAGAGTCTCCTATATAAGGATGGGA
AGACATACTTGAATTGGTTTCTCCAGAGGCCAGGCCAATCTCCACAGCTCCT
AATTTATTTGATGTCCACCCGTGCATCTGGGGTCAGCGACAGATTCAGCGG
CAGCGGGTCAGGCACTGATTTCACACTGAAAATCAGCAGGGTGGAGGCTGA
GGATGTTGGGGTTTATTACTGCCAACAACTTGTAGAGTATCCGCTCACGTTT
GGCCAGGGGACCAAGCTGGAGATCAAA

SEQ ID 60: PN encoding 2A10 heavy chain humanised construct H5 SEQ ID:
26
ATGGGATGGAGCTGTATCATCCTCTTCTTGGTAGCAACAGCTACAGGTGTCC
ACTCCCAGGTGCAGCTGGTGCAGTCTGGGGCTGAGGTGAAGAAGCCTGGG
GCCTCAGTGAAGGTTTCCTGCAAGGCATCTGGATACACCTTCACCAGCTACT
GGATGCACTGGGTGCGACAGGCCCCTGGACAAGGGCTTGAGTGGATGGGA
AATATTAATCCTAGCAATGGTGGTACTAACTACAATGAGAAGTTCAAGAGCA
GAGTCACCATGACCAGGGACACGTCCACGAGCACAGCCTACATGGAGCTG
AGCAGCCTGAGATCTGAGGACACGGCCGTGTATTACTGTGAACTGGGACAG
GGCTACTGGGGCCAGGGAACACTAGTCACAGTCTCCTCAGCCTCCACCAAG
GGCCCATCGGTCTTCCCCCTGGCACCCTCCTCCAAGAGCACCTCTGGGGG
CACAGCGGCCCTGGGCTGCCTGGTCAAGGACTACTTCCCCGAACCGGTGA
CGGTGTCGTGGAACTCAGGCGCCCTGACCAGCGGCGTGCACACCTTCCCG
GCTGTCCTACAGTCCTCAGGACTCTACTCCCTCAGCAGCGTGGTGACCGTG
CCCTCCAGCAGCTTGGGCACCCAGACCTACATCTGCAACGTGAATCACAAG
CCCAGCAACACCAAGGTGGACAAGAAAGTTGAGCCCAAATCTTGTGACAAA
ACTCACACATGCCCACCGTGCCCAGCACCTGAACTCGCGGGGGCACCGTC
AGTCTTCCTCTTCCCCCCAAAACCCAAGGACACCCTCATGATCTCCCGGAC
CCCTGAGGTCACATGCGTGGTGGTGGACGTGAGCCACGAAGACCCTGAGG
TCAAGTTCAACTGGTACGTGGACGGCGTGGAGGTGCATAATGCCAAGACAA
AGCCGCGGGAGGAGCAGTACAACAGCACGTACCGTGTGGTCAGCGTCCTC

ACCGTCCTGCACCAGGACTGGCTGAATGGCAAGGAGTACAAGTGCAAGGTC
TCCAACAAAGCCCTCCCAGCCCCCATCGAGAAAACCATCTCCAAAGCCAAA
GGGCAGCCCCGAGAACCACAGGTGTACACCCTGCCCCCATCCCGGGATGA
GCTGACCAAGAACCAGGTCAGCCTGACCTGCCTGGTCAAAGGCTTCTATCC
CAGCGACATCGCCGTGGAGTGGGAGAGCAATGGGCAGCCGGAGAACAACT
ACAAGACCACGCCTCCCGTGCTGGACTCCGACGGCTCCTTCTTCCTCTACA
GCAAGCTCACCGTGGACAAGAGCAGGTGGCAGCAGGGGAACGTCTTCTCA
TGCTCCGTGATGCATGAGGCTCTGCACAACCACTACACGCAGAAGAGCCTC
TCCCTGTCTCCGGGTAAATGA

SEQ ID 61: PN encoding 2A10 heavy chain humanised construct H6 SEQ ID:
27
ATGGGATGGAGCTGTATCATCCTCTTCTTGGTAGCAACAGCTACAGGTGTCC
ACTCCCAGGTGCAGCTGGTGCAGTCTGGGGCTGAGGTGAAGAAGCCTGGG
GCCTCAGTGAAGGTTTCCTGCAAGGCATCTGGATACACCTTCACCAGCTACT
GGATGCACTGGGTGCGACAGGCCCCTGGACAAGGGCTTGAGTGGATCGGA
AATATTAATCCTAGCAATGGTGGTACTAACTACAATGAGAAGTTCAAGAGCA
GAGCCACCATGACCAGGGACACGTCCACGAGCACAGCCTACATGGAGCTG
AGCAGCCTGAGATCTGAGGACACGGCCGTGTATTACTGTGAACTGGGACAG
GGCTACTGGGGCCAGGGAACACTAGTCACAGTCTCCTCAGCCTCCACCAAG
GGCCCATCGGTCTTCCCCCTGGCACCCTCCTCCAAGAGCACCTCTGGGGG
CACAGCGGCCCTGGGCTGCCTGGTCAAGGACTACTTCCCCGAACCGGTGA
CGGTGTCGTGGAACTCAGGCGCCCTGACCAGCGGCGTGCACACCTTCCCG
GCTGTCCTACAGTCCTCAGGACTCTACTCCCTCAGCAGCGTGGTGACCGTG
CCCTCCAGCAGCTTGGGCACCCAGACCTACATCTGCAACGTGAATCACAAG
CCCAGCAACACCAAGGTGGACAAGAAAGTTGAGCCCAAATCTTGTGACAAA
ACTCACACATGCCCACCGTGCCCAGCACCTGAACTCGCGGGGGCACCGTC
AGTCTTCCTCTTCCCCCCAAAACCCAAGGACACCCTCATGATCTCCCGGAC
CCCTGAGGTCACATGCGTGGTGGTGGACGTGAGCCACGAAGACCCTGAGG
TCAAGTTCAACTGGTACGTGGACGGCGTGGAGGTGCATAATGCCAAGACAA
AGCCGCGGGAGGAGCAGTACAACAGCACGTACCGTGTGGTCAGCGTCCTC
ACCGTCCTGCACCAGGACTGGCTGAATGGCAAGGAGTACAAGTGCAAGGTC
TCCAACAAAGCCCTCCCAGCCCCCATCGAGAAAACCATCTCCAAAGCCAAA
GGGCAGCCCCGAGAACCACAGGTGTACACCCTGCCCCCATCCCGGGATGA
GCTGACCAAGAACCAGGTCAGCCTGACCTGCCTGGTCAAAGGCTTCTATCC
CAGCGACATCGCCGTGGAGTGGGAGAGCAATGGGCAGCCGGAGAACAACT
ACAAGACCACGCCTCCCGTGCTGGACTCCGACGGCTCCTTCTTCCTCTACA
GCAAGCTCACCGTGGACAAGAGCAGGTGGCAGCAGGGGAACGTCTTCTCA
TGCTCCGTGATGCATGAGGCTCTGCACAACCACTACACGCAGAAGAGCCTC
TCCCTGTCTCCGGGTAAATGA

SEQ ID 62: PN encoding 2A10 heavy chain humanised construct H700 SEQ ID:
28

ATGGGATGGAGCTGTATCATCCTCTTCTTGGTAGCAACAGCTACAGGTGTCC
ACTCCCAGGTGCAGCTGGTGCAGTCTGGGGCTGAGGTGAAGAAGCCTGGG
GCCTCAGTGAAGGTTTCCTGCAAGGCATCTGGATACACCTTCACCAGCTACT
GGATGCACTGGGTGCGACAGGCCCCTGGACAAGGGCTTGAGTGGATCGGA
AATATTAATCCTAGCAATGGTGGTACTAACTACAATGAGAAGTTCAAGAGCA
GAGCCACCCTGACCGTAGACAAATCCACGAGCACAGCCTACATGGAGCTGA
GCAGCCTGAGATCTGAGGACACGGCCGTGTATTACTGTGAACTGGGACAGG
GCTACTGGGGCCAGGGAACACTAGTCACAGTCTCCTCAGCCTCCACCAAGG
GCCCATCGGTCTTCCCCCTGGCACCCTCCTCCAAGAGCACCTCTGGGGGC
ACAGCGGCCCTGGGCTGCCTGGTCAAGGACTACTTCCCCGAACCGGTGAC
GGTGTCGTGGAACTCAGGCGCCCTGACCAGCGGCGTGCACACCTTCCCGG
CTGTCCTACAGTCCTCAGGACTCTACTCCCTCAGCAGCGTGGTGACCGTGC
CCTCCAGCAGCTTGGGCACCCAGACCTACATCTGCAACGTGAATCACAAGC
CCAGCAACACCAAGGTGGACAAGAAAGTTGAGCCCAAATCTTGTGACAAAA
CTCACACATGCCCACCGTGCCCAGCACCTGAACTCGCGGGGGCACCGTCA
GTCTTCCTCTTCCCCCCAAAACCCAAGGACACCCTCATGATCTCCCGGACC
CCTGAGGTCACATGCGTGGTGGTGGACGTGAGCCACGAAGACCCTGAGGT
CAAGTTCAACTGGTACGTGGACGGCGTGGAGGTGCATAATGCCAAGACAAA
GCCGCGGGAGGAGCAGTACAACAGCACGTACCGTGTGGTCAGCGTCCTCA
CCGTCCTGCACCAGGACTGGCTGAATGGCAAGGAGTACAAGTGCAAGGTCT
CCAACAAAGCCCTCCCAGCCCCCATCGAGAAAACCATCTCCAAAGCCAAAG
GGCAGCCCCGAGAACCACAGGTGTACACCCTGCCCCCATCCCGGGATGAG
CTGACCAAGAACCAGGTCAGCCTGACCTGCCTGGTCAAAGGCTTCTATCCC
AGCGACATCGCCGTGGAGTGGGAGAGCAATGGGCAGCCGGAGAACAACTA
CAAGACCACGCCTCCCGTGCTGGACTCCGACGGCTCCTTCTTCCTCTACAG
CAAGCTCACCGTGGACAAGAGCAGGTGGCAGCAGGGGAACGTCTTCTCAT
GCTCCGTGATGCATGAGGCTCTGCACAACCACTACACGCAGAAGAGCCTCT
CCCTGTCTCCGGGTAAATGA

SEQ ID 63: PN encoding 2A10 heavy chain humanised construct H14 SEQ ID:
29
ATGGGATGGAGCTGTATCATCCTCTTCTTGGTAGCAACAGCTACAGGTGTCC
ACTCCCAGGTGCAGCTGGTGCAGTCTGGGGCTGAGGTGGTCAAGCCTGGG
GCCTCAGTGAAGCTCTCCTGCAAGGCATCTGGATACACCTTCACCAGCTAC
TGGATGCACTGGGTGAAACAGGCCCCTGGACAAGGGCTTGAGTGGATCGG
AAATATTAATCCTAGCAATGGTGGTACTAACTACAATGAGAAGTTCAAGAGC
AGAGCCACCCTCACCAGGGACACGTCCACGAGCACAGCCTACATGGAGCT
GAGCAGCCTGAGATCTGAGGACACGGCCGTGTATTACTGTGAACTGGGACA
GGGCTACTGGGGCCAGGGAACACTAGTCACAGTCTCCTCAGCCTCCACCAA
GGGCCCATCGGTCTTCCCCCTGGCACCCTCCTCCAAGAGCACCTCTGGGG
GCACAGCGGCCCTGGGCTGCCTGGTCAAGGACTACTTCCCCGAACCGGTG
ACGGTGTCGTGGAACTCAGGCGCCCTGACCAGCGGCGTGCACACCTTCCC
GGCTGTCCTACAGTCCTCAGGACTCTACTCCCTCAGCAGCGTGGTGACCGT
GCCCTCCAGCAGCTTGGGCACCCAGACCTACATCTGCAACGTGAATCACAA
GCCCAGCAACACCAAGGTGGACAAGAAAGTTGAGCCCAAATCTTGTGACAA
AACTCACACATGCCCACCGTGCCCAGCACCTGAACTCGCGGGGGCACCGT
CAGTCTTCCTCTTCCCCCCAAAACCCAAGGACACCCTCATGATCTCCCGGA
CCCCTGAGGTCACATGCGTGGTGGTGGACGTGAGCCACGAAGACCCTGAG

GTCAAGTTCAACTGGTACGTGGACGGCGTGGAGGTGCATAATGCCAAGACA
AAGCCGCGGGAGGAGCAGTACAACAGCACGTACCGTGTGGTCAGCGTCCT
CACCGTCCTGCACCAGGACTGGCTGAATGGCAAGGAGTACAAGTGCAAGGT
CTCCAACAAAGCCCTCCCAGCCCCCATCGAGAAAACCATCTCCAAAGCCAA
AGGGCAGCCCCGAGAACCACAGGTGTACACCCTGCCCCCATCCCGGGATG
AGCTGACCAAGAACCAGGTCAGCCTGACCTGCCTGGTCAAAGGCTTCTATC
CCAGCGACATCGCCGTGGAGTGGGAGAGCAATGGGCAGCCGGAGAACAAC
TACAAGACCACGCCTCCCGTGCTGGACTCCGACGGCTCCTTCTTCCTCTAC
AGCAAGCTCACCGTGGACAAGAGCAGGTGGCAGCAGGGGAACGTCTTCTC
ATGCTCCGTGATGCATGAGGCTCTGCACAACCACTACACGCAGAAGAGCCT
CTCCCTGTCTCCGGGTAAATGA

SEQ ID 64: PN encoding 2A10 heavy chain humanised construct H15 SEQ ID:
30
ATGGGATGGAGCTGTATCATCCTCTTCTTGGTAGCAACAGCTACAGGTGTCC
ACTCCCAGGTGCAGCTGGTGCAGTCTGGGGCTGAGGTGGTCAAGCCTGGG
GCCTCAGTGAAGCTCTCCTGCAAGGCATCTGGATACACCTTCACCAGCTAC
TGGATGCACTGGGTGAAACAGCGACCTGGACAAGGGCTTGAGTGGATCGG
AAATATTAATCCTAGCAATGGTGGTACTAACTACAATGAGAAGTTCAAGAGC
AAAGCCACCCTCACCGTCGACACGTCCAGCAGCACAGCCTACATGGAGCTG
AGCAGCCTGAGATCTGAGGACAGCGCCGTGTATTACTGTGAACTGGGACAG
GGCTACTGGGGCCAGGGAACACTAGTCACAGTCTCCTCAGCCTCCACCAAG
GGCCCATCGGTCTTCCCCCTGGCACCCTCCTCCAAGAGCACCTCTGGGGG
CACAGCGGCCCTGGGCTGCCTGGTCAAGGACTACTTCCCCGAACCGGTGA
CGGTGTCGTGGAACTCAGGCGCCCTGACCAGCGGCGTGCACACCTTCCCG
GCTGTCCTACAGTCCTCAGGACTCTACTCCCTCAGCAGCGTGGTGACCGTG
CCCTCCAGCAGCTTGGGCACCCAGACCTACATCTGCAACGTGAATCACAAG
CCCAGCAACACCAAGGTGGACAAGAAAGTTGAGCCCAAATCTTGTGACAAA
ACTCACACATGCCCACCGTGCCCAGCACCTGAACTCGCGGGGGCACCGTC
AGTCTTCCTCTTCCCCCCAAAACCCAAGGACACCCTCATGATCTCCCGGAC
CCCTGAGGTCACATGCGTGGTGGTGGACGTGAGCCACGAAGACCCTGAGG
TCAAGTTCAACTGGTACGTGGACGGCGTGGAGGTGCATAATGCCAAGACAA
AGCCGCGGGAGGAGCAGTACAACAGCACGTACCGTGTGGTCAGCGTCCTC
ACCGTCCTGCACCAGGACTGGCTGAATGGCAAGGAGTACAAGTGCAAGGTC
TCCAACAAAGCCCTCCCAGCCCCCATCGAGAAAACCATCTCCAAAGCCAAA
GGGCAGCCCCGAGAACCACAGGTGTACACCCTGCCCCCATCCCGGGATGA
GCTGACCAAGAACCAGGTCAGCCTGACCTGCCTGGTCAAAGGCTTCTATCC
CAGCGACATCGCCGTGGAGTGGGAGAGCAATGGGCAGCCGGAGAACAACT
ACAAGACCACGCCTCCCGTGCTGGACTCCGACGGCTCCTTCTTCCTCTACA
GCAAGCTCACCGTGGACAAGAGCAGGTGGCAGCAGGGGAACGTCTTCTCA
TGCTCCGTGATGCATGAGGCTCTGCACAACCACTACACGCAGAAGAGCCTC
TCCCTGTCTCCGGGTAAATGA

SEQ ID 65: PN encoding 2A10 heavy chain humanised construct H16 SEQ ID:
31
ATGGGATGGAGCTGTATCATCCTCTTCTTGGTAGCAACAGCTACAGGTGTCC
ACTCCCAGGTGCAGCTGGTGCAGTCTGGGGCTGAGGTGAAGAAGCCTGGG

GCCTCAGTGAAGGTTTCCTGCAAGGCATCTGGATACACCTTCACCAGCTACT
GGATGCACTGGGTGAAACAGCGACCTGGACAAGGGCTTGAGTGGATCGGA
AATATTAATCCTAGCAATGGTGGTACTAACTACAATGAGAAGTTCAAGAGCA
AAGCCACCCTCACCGTCGACAAATCCACGAGCACAGCCTACATGGAGCTGA
GCAGCCTGAGATCTGAGGACACGGCCGTGTATTACTGTGAACTGGGACAGG
GCTACTGGGGCCAGGGAACACTAGTCACAGTCTCCTCAGCCTCCACCAAGG
GCCCATCGGTCTTCCCCCTGGCACCCTCCTCCAAGAGCACCTCTGGGGGC
ACAGCGGCCCTGGGCTGCCTGGTCAAGGACTACTTCCCCGAACCGGTGAC
GGTGTCGTGGAACTCAGGCGCCCTGACCAGCGGCGTGCACACCTTCCCGG
CTGTCCTACAGTCCTCAGGACTCTACTCCCTCAGCAGCGTGGTGACCGTGC
CCTCCAGCAGCTTGGGCACCCAGACCTACATCTGCAACGTGAATCACAAGC
CCAGCAACACCAAGGTGGACAAGAAAGTTGAGCCCAAATCTTGTGACAAAA
CTCACACATGCCCACCGTGCCCAGCACCTGAACTCGCGGGGGCACCGTCA
GTCTTCCTCTTCCCCCCAAAACCCAAGGACACCCTCATGATCTCCCGGACC
CCTGAGGTCACATGCGTGGTGGTGGACGTGAGCCACGAAGACCCTGAGGT
CAAGTTCAACTGGTACGTGGACGGCGTGGAGGTGCATAATGCCAAGACAAA
GCCGCGGGAGGAGCAGTACAACAGCACGTACCGTGTGGTCAGCGTCCTCA
CCGTCCTGCACCAGGACTGGCTGAATGGCAAGGAGTACAAGTGCAAGGTCT
CCAACAAAGCCCTCCCAGCCCCCATCGAGAAAACCATCTCCAAAGCCAAAG
GGCAGCCCCGAGAACCACAGGTGTACACCCTGCCCCCATCCCGGGATGAG
CTGACCAAGAACCAGGTCAGCCTGACCTGCCTGGTCAAAGGCTTCTATCCC
AGCGACATCGCCGTGGAGTGGGAGAGCAATGGGCAGCCGGAGAACAACTA
CAAGACCACGCCTCCCGTGCTGGACTCCGACGGCTCCTTCTTCCTCTACAG
CAAGCTCACCGTGGACAAGAGCAGGTGGCAGCAGGGGAACGTCTTCTCAT
GCTCCGTGATGCATGAGGCTCTGCACAACCACTACACGCAGAAGAGCCTCT
CCCTGTCTCCGGGTAAATGA

SEQ ID 66: PN encoding 2A10 heavy chain humanised construct H17 SEQ ID:
32
ATGGGATGGAGCTGTATCATCCTCTTCTTGGTAGCAACAGCTACAGGTGTCC
ACTCCCAGGTGCAGCTGGTGCAGTCTGGGGCTGAGGTGGTCAAGCCTGGG
GCCTCAGTGAAGCTCTCCTGCAAGGCATCTGGATACACCTTCACCAGCTAC
TGGATGCACTGGGTGAAACAGCGACCTGGACAAGGGCTTGAGTGGATCGG
AAATATTAATCCTAGCAATGGTGGTACTAACTACAATGAGAAGTTCAAGAGC
AAAGCCACCCTCACCGTCGACAAATCCACGAGCACAGCCTACATGGAGCTG
AGCAGCCTGAGATCTGAGGACACGGCCGTGTATTACTGTGAACTGGGACAG
GGCTACTGGGGCCAGGGAACACTAGTCACAGTCTCCTCAGCCTCCACCAAG
GGCCCATCGGTCTTCCCCCTGGCACCCTCCTCCAAGAGCACCTCTGGGGG
CACAGCGGCCCTGGGCTGCCTGGTCAAGGACTACTTCCCCGAACCGGTGA
CGGTGTCGTGGAACTCAGGCGCCCTGACCAGCGGCGTGCACACCTTCCCG
GCTGTCCTACAGTCCTCAGGACTCTACTCCCTCAGCAGCGTGGTGACCGTG
CCCTCCAGCAGCTTGGGCACCCAGACCTACATCTGCAACGTGAATCACAAG
CCCAGCAACACCAAGGTGGACAAGAAAGTTGAGCCCAAATCTTGTGACAAA
ACTCACACATGCCCACCGTGCCCAGCACCTGAACTCGCGGGGGCACCGTC
AGTCTTCCTCTTCCCCCCAAAACCCAAGGACACCCTCATGATCTCCCGGAC
CCCTGAGGTCACATGCGTGGTGGTGGACGTGAGCCACGAAGACCCTGAGG
TCAAGTTCAACTGGTACGTGGACGGCGTGGAGGTGCATAATGCCAAGACAA
AGCCGCGGGAGGAGCAGTACAACAGCACGTACCGTGTGGTCAGCGTCCTC

ACCGTCCTGCACCAGGACTGGCTGAATGGCAAGGAGTACAAGTGCAAGGTC
TCCAACAAAGCCCTCCCAGCCCCCATCGAGAAAACCATCTCCAAAGCCAAA
GGGCAGCCCCGAGAACCACAGGTGTACACCCTGCCCCCATCCCGGGATGA
GCTGACCAAGAACCAGGTCAGCCTGACCTGCCTGGTCAAAGGCTTCTATCC
CAGCGACATCGCCGTGGAGTGGGAGAGCAATGGGCAGCCGGAGAACAACT
ACAAGACCACGCCTCCCGTGCTGGACTCCGACGGCTCCTTCTTCCTCTACA
GCAAGCTCACCGTGGACAAGAGCAGGTGGCAGCAGGGGAACGTCTTCTCA
TGCTCCGTGATGCATGAGGCTCTGCACAACCACTACACGCAGAAGAGCCTC
TCCCTGTCTCCGGGTAAATGA

SEQ ID 67: PN encoding 2A10 heavy chain humanised construct H18 SEQ ID:
33
ATGGGATGGAGCTGTATCATCCTCTTCTTGGTAGCAACAGCTACAGGTGTCC
ACTCCCAGGTGCAGCTGGTGCAGTCTGGGGCTGAGGTGGTCAAGCCTGGG
GCCTCAGTGAAGCTCTCCTGCAAGGCATCTGGATACACCTTCACCAGCTAC
TGGATGCACTGGGTGAAACAGCGACCTGGACAAGGGCTTGAGTGGATCGG
AAATATTAATCCTAGCAATGGTGGTACTAACTACAATGAGAAGTTCAAGAGC
AAAGCCACCCTCACCGTCGACAAATCCAGCAGCACAGCCTACATGGAGCTG
AGCAGCCTGAGATCTGAGGACAGCGCCGTGTATTACTGTGAACTGGGACAG
GGCTACTGGGGCCAGGGAACACTAGTCACAGTCTCCTCAGCCTCCACCAAG
GGCCCATCGGTCTTCCCCCTGGCACCCTCCTCCAAGAGCACCTCTGGGGG
CACAGCGGCCCTGGGCTGCCTGGTCAAGGACTACTTCCCCGAACCGGTGA
CGGTGTCGTGGAACTCAGGCGCCCTGACCAGCGGCGTGCACACCTTCCCG
GCTGTCCTACAGTCCTCAGGACTCTACTCCCTCAGCAGCGTGGTGACCGTG
CCCTCCAGCAGCTTGGGCACCCAGACCTACATCTGCAACGTGAATCACAAG
CCCAGCAACACCAAGGTGGACAAGAAAGTTGAGCCCAAATCTTGTGACAAA
ACTCACACATGCCCACCGTGCCCAGCACCTGAACTCGCGGGGGCACCGTC
AGTCTTCCTCTTCCCCCCAAAACCCAAGGACACCCTCATGATCTCCCGGAC
CCCTGAGGTCACATGCGTGGTGGTGGACGTGAGCCACGAAGACCCTGAGG
TCAAGTTCAACTGGTACGTGGACGGCGTGGAGGTGCATAATGCCAAGACAA
AGCCGCGGGAGGAGCAGTACAACAGCACGTACCGTGTGGTCAGCGTCCTC
ACCGTCCTGCACCAGGACTGGCTGAATGGCAAGGAGTACAAGTGCAAGGTC
TCCAACAAAGCCCTCCCAGCCCCCATCGAGAAAACCATCTCCAAAGCCAAA
GGGCAGCCCCGAGAACCACAGGTGTACACCCTGCCCCCATCCCGGGATGA
GCTGACCAAGAACCAGGTCAGCCTGACCTGCCTGGTCAAAGGCTTCTATCC
CAGCGACATCGCCGTGGAGTGGGAGAGCAATGGGCAGCCGGAGAACAACT
ACAAGACCACGCCTCCCGTGCTGGACTCCGACGGCTCCTTCTTCCTCTACA
GCAAGCTCACCGTGGACAAGAGCAGGTGGCAGCAGGGGAACGTCTTCTCA
TGCTCCGTGATGCATGAGGCTCTGCACAACCACTACACGCAGAAGAGCCTC
TCCCTGTCTCCGGGTAAATGA

SEQ ID 68: PN encoding 2A10 light chain humanised construct L6 SEQ ID: 34
ATGGGATGGAGCTGTATCATCCTCTTCTTGGTAGCAACAGCTACAGGTGTCC
ACTCCGATATTGTGATGACTCAGTCTCCACTCTCCCTGCCCGTCACCCCTGG
AGAGCCGGCCTCCATCTCCTGCAGGTCTAGTAAGAGTCTCCTATATAAGGAT
GGGAAGACATACTTGAATTGGTTCCTGCAGCGACCAGGGCAGTCTCCACAG
CTCCTGATCTATTTGATGTCCACCCGTGCATCAGGGGTCCCTGACAGGTTCA

GTGGCAGTGGATCAGGCACAGATTTTACACTGAAAATCAGCAGAGTGGAGG
CTGAGGATGTTGGGGTTTATTACTGCCAACAACTTGTAGAGTATCCGCTCAC
GTTCGGCGGAGGGACCAAGGTGGAGATCAAACGTACGGTGGCTGCACCAT
CTGTCTTCATCTTCCCGCCATCTGATGAGCAGTTGAAATCTGGAACTGCCTC
TGTTGTGTGCCTGCTGAATAACTTCTATCCCAGAGAGGCCAAAGTACAGTGG
AAGGTGGACAACGCCCTCCAATCGGGTAACTCCCAGGAGAGTGTCACAGAG
CAGGACAGCAAGGACAGCACCTACAGCCTCAGCAGCACCCTGACGCTGAG
CAAAGCAGACTACGAGAAACACAAAGTCTACGCCTGCGAAGTCACCCATCA
GGGCCTGAGCTCGCCCGTCACAAAGAGCTTCAACAGGGGAGAGTGTTAG

SEQ ID 69: PN encoding 2A10 light chain humanised construct L13 SEQ ID: 35
ATGGGATGGAGCTGTATCATCCTCTTCTTGGTAGCAACAGCTACAGGTGTCC
ACTCCGATATTGTGATGACCCAGTCTCCACTCTCCCTGCCCGTCACCCTTGG
ACAGCCGGCCTCCATCTCCTGCAGGTCTAGTAAGAGTCTCCTATATAAGGAT
GGGAAGACATACTTGAATTGGTTTCAGCAGAGGCCAGGCCAATCTCCACAG
CTCCTAATTTATTTGATGTCCACCCGTGCATCTGGGGTCCCAGACAGATTCA
GCGGCGGTGGGTCAGGCACTGATTTCACACTGAAAATCAGCAGGGTGGAG
GCTGAGGATGTTGGGGTTTATTACTGCCAACAACTTGTAGAGTATCCGCTCA
CGTTTGGCCAGGGGACCAAGCTGGAGATCAAACGTACGGTGGCTGCACCA
TCTGTCTTCATCTTCCCGCCATCTGATGAGCAGTTGAAATCTGGAACTGCCT
CTGTTGTGTGCCTGCTGAATAACTTCTATCCCAGAGAGGCCAAAGTACAGTG
GAAGGTGGACAACGCCCTCCAATCGGGTAACTCCCAGGAGAGTGTCACAGA
GCAGGACAGCAAGGACAGCACCTACAGCCTCAGCAGCACCCTGACGCTGA
GCAAAGCAGACTACGAGAAACACAAAGTCTACGCCTGCGAAGTCACCCATC
AGGGCCTGAGCTCGCCCGTCACAAAGAGCTTCAACAGGGGAGAGTGTTAG

SEQ ID 70: PN encoding 2A10 light chain humanised construct L14 SEQ ID: 36
ATGGGATGGAGCTGTATCATCCTCTTCTTGGTAGCAACAGCTACAGGTGTCC
ACTCCGATATTGTGATGACCCAGTCTCCACTCTCCCTGCCCGTCACCCTTGG
ACAGCCGGCCTCCATCTCCTGCAGGTCTAGTAAGAGTCTCCTATATAAGGAT
GGGAAGACATACTTGAATTGGTTTCTCCAGAGGCCAGGCCAATCTCCACAG
CTCCTAATTTATTTGATGTCCACCCGTGCATCTGGGGTCCCAGACAGATTCA
GCGGCGGTGGGTCAGGCACTGATTTCACACTGAAAATCAGCAGGGTGGAG
GCTGAGGATGTTGGGGTTTATTACTGCCAACAACTTGTAGAGTATCCGCTCA
CGTTTGGCCAGGGGACCAAGCTGGAGATCAAACGTACGGTGGCTGCACCA
TCTGTCTTCATCTTCCCGCCATCTGATGAGCAGTTGAAATCTGGAACTGCCT
CTGTTGTGTGCCTGCTGAATAACTTCTATCCCAGAGAGGCCAAAGTACAGTG
GAAGGTGGACAACGCCCTCCAATCGGGTAACTCCCAGGAGAGTGTCACAGA
GCAGGACAGCAAGGACAGCACCTACAGCCTCAGCAGCACCCTGACGCTGA
GCAAAGCAGACTACGAGAAACACAAAGTCTACGCCTGCGAAGTCACCCATC
AGGGCCTGAGCTCGCCCGTCACAAAGAGCTTCAACAGGGGAGAGTGTTAG

SEQ ID 71: PN encoding 2A10 light chain humanised construct L15 SEQ ID: 37
ATGGGATGGAGCTGTATCATCCTCTTCTTGGTAGCAACAGCTACAGGTGTCC
ACTCCGATATTGTGATGACCCAGTCTCCACTCTCCCTGCCCGTCACCCTTGG
ACAGCCGGCCTCCATCTCCTGCAGGTCTAGTAAGAGTCTCCTATATAAGGAT
GGGAAGACATACTTGAATTGGTTTCTCCAGAGGCCAGGCCAATCTCCACAG

CTCCTAATTTATTTGATGTCCACCCGTGCATCTGGGGTCAGCGACAGATTCA
GCGGCGGTGGGTCAGGCACTGATTTCACACTGAAAATCAGCAGGGTGGAG
GCTGAGGATGTTGGGGTTTATTACTGCCAACAACTTGTAGAGTATCCGCTCA
CGTTTGGCCAGGGGACCAAGCTGGAGATCAAACGTACGGTGGCTGCACCA
TCTGTCTTCATCTTCCCGCCATCTGATGAGCAGTTGAAATCTGGAACTGCCT
CTGTTGTGTGCCTGCTGAATAACTTCTATCCCAGAGAGGCCAAAGTACAGTG
GAAGGTGGACAACGCCCTCCAATCGGGTAACTCCCAGGAGAGTGTCACAGA
GCAGGACAGCAAGGACAGCACCTACAGCCTCAGCAGCACCCTGACGCTGA
GCAAAGCAGACTACGAGAAACACAAAGTCTACGCCTGCGAAGTCACCCATC
AGGGCCTGAGCTCGCCCGTCACAAAGAGCTTCAACAGGGGAGAGTGTTAG

SEQ ID 72: PN encoding 2A10 light chain humanised construct L16 SEQ ID: 38
ATGGGATGGAGCTGTATCATCCTCTTCTTGGTAGCAACAGCTACAGGTGTCC
ACTCCGATATTGTGATGACCCAGTCTCCACTCTCCAACCCCGTCACCCTTGG
ACAGCCGGTCTCCATCTCCTGCAGGTCTAGTAAGAGTCTCCTATATAAGGAT
GGGAAGACATACTTGAATTGGTTTCTCCAGAGGCCAGGCCAATCTCCACAG
CTCCTAATTTATTTGATGTCCACCCGTGCATCTGGGGTCCCAGACAGATTCA
GCGGCGGTGGGTCAGGCACTGATTTCACACTGAAAATCAGCAGGGTGGAG
GCTGAGGATGTTGGGGTTTATTACTGCCAACAACTTGTAGAGTATCCGCTCA
CGTTTGGCCAGGGGACCAAGCTGGAGATCAAACGTACGGTGGCTGCACCA
TCTGTCTTCATCTTCCCGCCATCTGATGAGCAGTTGAAATCTGGAACTGCCT
CTGTTGTGTGCCTGCTGAATAACTTCTATCCCAGAGAGGCCAAAGTACAGTG
GAAGGTGGACAACGCCCTCCAATCGGGTAACTCCCAGGAGAGTGTCACAGA
GCAGGACAGCAAGGACAGCACCTACAGCCTCAGCAGCACCCTGACGCTGA
GCAAAGCAGACTACGAGAAACACAAAGTCTACGCCTGCGAAGTCACCCATC
AGGGCCTGAGCTCGCCCGTCACAAAGAGCTTCAACAGGGGAGAGTGTTAG

SEQ ID 73: PN encoding 2A10 light chain humanised construct L17 SEQ ID: 39
ATGGGATGGAGCTGTATCATCCTCTTCTTGGTAGCAACAGCTACAGGTGTCC
ACTCCGATATTGTGATGACCCAGGACCCACTCTCCCTGCCCGTCACCCTTG
GACAGCCGGCCTCCATCTCCTGCAGGTCTAGTAAGAGTCTCCTATATAAGG
ATGGGAAGACATACTTGAATTGGTTTCAGCAGAGGCCAGGCCAATCTCCAC
AGCTCCTAATTTATTTGATGTCCACCCGTGCATCTGGGGTCAGCGACAGATT
CAGCGGCAGCGGGTCAGGCACTGATTTCACACTGAAAATCAGCAGGGTGG
AGGCTGAGGATGTTGGGGTTTATTACTGCCAACAACTTGTAGAGTATCCGCT
CACGTTTGGCCAGGGGACCAAGCTGGAGATCAAACGTACGGTGGCTGCAC
CATCTGTCTTCATCTTCCCGCCATCTGATGAGCAGTTGAAATCTGGAACTGC
CTCTGTTGTGTGCCTGCTGAATAACTTCTATCCCAGAGAGGCCAAAGTACAG
TGGAAGGTGGACAACGCCCTCCAATCGGGTAACTCCCAGGAGAGTGTCACA
GAGCAGGACAGCAAGGACAGCACCTACAGCCTCAGCAGCACCCTGACGCT
GAGCAAAGCAGACTACGAGAAACACAAAGTCTACGCCTGCGAAGTCACCCA
TCAGGGCCTGAGCTCGCCCGTCACAAAGAGCTTCAACAGGGGAGAGTGTTA
G

SEQ ID 74: PN encoding 2A10 light chain humanised construct L18 SEQ ID: 40
ATGGGATGGAGCTGTATCATCCTCTTCTTGGTAGCAACAGCTACAGGTGTCC
ACTCCGATATTGTGATGACCCAGGACCCACTCTCCAACCCCGTCACCCTTG

GACAGCCGGTCTCCATCTCCTGCAGGTCTAGTAAGAGTCTCCTATATAAGGA
TGGGAAGACATACTTGAATTGGTTTCTCCAGAGGCCAGGCCAATCTCCACA
GCTCCTAATTTATTTGATGTCCACCCGTGCATCTGGGGTCAGCGACAGATTC
AGCGGCAGCGGGTCAGGCACTGATTTCACACTGAAAATCAGCAGGGTGGA
GGCTGAGGATGTTGGGGTTTATTACTGCCAACAACTTGTAGAGTATCCGCTC
ACGTTTGGCCAGGGGACCAAGCTGGAGATCAAACGTACGGTGGCTGCACC
ATCTGTCTTCATCTTCCCGCCATCTGATGAGCAGTTGAAATCTGGAACTGCC
TCTGTTGTGTGCCTGCTGAATAACTTCTATCCCAGAGAGGCCAAAGTACAGT
GGAAGGTGGACAACGCCCTCCAATCGGGTAACTCCCAGGAGAGTGTCACA
GAGCAGGACAGCAAGGACAGCACCTACAGCCTCAGCAGCACCCTGACGCT
GAGCAAAGCAGACTACGAGAAACACAAAGTCTACGCCTGCGAAGTCACCCA
TCAGGGCCTGAGCTCGCCCGTCACAAAGAGCTTCAACAGGGGAGAGTGTTA
G

SEQ ID 75: Campath leader sequence
MGWSCIILFLVATATGVHS

SEQ ID 76: Amino acids 586-785 of human NOGO A (NOGO-A56)
MQESLYPAAQLCPSFEESEATPSPVLPDIVMEAPLNSAVPSAGASVIQPSSSPL
EASSVNYESIKHEPENPPPYEEAMSVSLKKVSGIKEEIKEPENINAALQETEAPYI
SIACDLIKETKLSAEPAPDFSDYSEMAKVEQPVPDHSELVEDSSPDSEPVDLFS
DDSIPDVPQKQDETVMLVKESLTETSFESMIEYENKE

SEQ ID 77: 2A10 VH humanised construct H1
QVQLVQSGAEVKKPGASVKVSCKASGYTFTSYWMHWVRQAPGQGLEWMGNI
NPSNGGTNYNEKFKSRVTMTRDTSTSTVYMELSSLRSEDTAVYYCELGQGYW
GQGTLVTVSS

SEQ ID 78: 2A10 VL humanised construct L11
DIVITQSPLSLPVTLGQPASISCRSSKSLLYKDGKTYLNWFQQRPGQSPQLLIYL
MSTRASGVPDRFSGGGSGTDFTLKISRVEAEDVGVYYCQQLVEYPLTFGQGTK
LEIK

SEQ ID 79: 2A10 heavy chain humanised construct H1
MGWSCIILFLVATATGVHSQVQLVQSGAEVKKPGASVKVSCKASGYTFTSYWM
HWVRQAPGQGLEWMGNINPSNGGTNYNEKFKSRVTMTRDTSTSTVYMELSSL
RSEDTAVYYCELGQGYWGQGTLVTVSSASTKGPSVFPLAPSSKSTSGGTAAL
GCLVKDYFPEPVTVSWNSGALTSGVHTFPAVLQSSGLYSLSSVVTVPSSSLGT
QTYICNVNHKPSNTKVDKKVEPKSCDKTHTCPPCPAPELAGAPSVFLFPPKPKD
TLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKL
TVDKSRWQQGNVFSCSVMHEALHNHYTQKSLSLSPGK

SEQ ID 80: 2A10 light chain humanised construct L11

MGWSCIILFLVATATGVHSDIVITQSPLSLPVTLGQPASISCRSSKSLLYKDGKTY
LNWFQQRPGQSPQLLIYLMSTRASGVPDRFSGGGSGTDFTLKISRVEAEDVGV
YYCQQLVEYPLTFGQGTKLEIKRTVAAPSVFIFPPSDEQLKSGTASVVCLLNNFY
PREAKVQWKVDNALQSGNSQESVTEQDSKDSTYSLSSTLTLSKADYEKHKVYA
CEVTHQGLSSPVTKSFNRGEC

SEQ ID 81: PN encoding 2A10 VH humanised construct H1 SEQ ID: 77
CAGGTGCAGCTGGTGCAGTCTGGGGCTGAGGTGAAGAAGCCTGGGGCCTC
AGTGAAGGTTTCCTGCAAGGCATCTGGATACACCTTCACCAGCTACTGGAT
GCACTGGGTGCGACAGGCCCCTGGACAAGGGCTTGAGTGGATGGGAAATA
TTAATCCTAGCAATGGTGGTACTAACTACAATGAGAAGTTCAAGAGCAGAGT
CACCATGACCAGGGACACGTCCACGAGCACAGTCTACATGGAGCTGAGCA
GCCTGAGATCTGAGGACACGGCCGTGTATTACTGTGAACTGGGACAGGGCT
ACTGGGGCCAGGGAACACTAGTCACAGTCTCCTCA

SEQ ID 82: PN encoding 2A10 VL humanised construct L11 SEQ ID: 78
GATATTGTGATAACCCAGTCTCCACTCTCCCTGCCCGTCACCCTTGGACAGC
CGGCCTCCATCTCCTGCAGGTCTAGTAAGAGTCTCCTATATAAGGATGGGA
AGACATACTTGAATTGGTTTCAGCAGAGGCCAGGCCAATCTCCACAGCTCCT
AATTTATTTGATGTCCACCCGTGCATCTGGGGTCCCAGACAGATTCAGCGG
CGGTGGGTCAGGCACTGATTTCACACTGAAAATCAGCAGGGTGGAGGCTGA
GGATGTTGGGGTTTATTACTGCCAACAACTTGTAGAGTATCCGCTCACGTTT
GGCCAGGGGACCAAGCTGGAGATCAAA

SEQ ID 83: PN encoding 2A10 humanised heavy chain H1 SEQ ID: 79
ATGGGATGGAGCTGTATCATCCTCTTCTTGGTAGCAACAGCTACAGGTGTCC
ACTCCCAGGTGCAGCTGGTGCAGTCTGGGGCTGAGGTGAAGAAGCCTGGG
GCCTCAGTGAAGGTTTCCTGCAAGGCATCTGGATACACCTTCACCAGCTACT
GGATGCACTGGGTGCGACAGGCCCCTGGACAAGGGCTTGAGTGGATGGGA
AATATTAATCCTAGCAATGGTGGTACTAACTACAATGAGAAGTTCAAGAGCA
GAGTCACCATGACCAGGGACACGTCCACGAGCACAGTCTACATGGAGCTGA
GCAGCCTGAGATCTGAGGACACGGCCGTGTATTACTGTGAACTGGGACAGG
GCTACTGGGGCCAGGGAACACTAGTCACAGTCTCCTCAGCCTCCACCAAGG
GCCCATCGGTCTTCCCCCTGGCACCCTCCTCCAAGAGCACCTCTGGGGGC
ACAGCGGCCCTGGGCTGCCTGGTCAAGGACTACTTCCCCGAACCGGTGAC
GGTGTCGTGGAACTCAGGCGCCCTGACCAGCGGCGTGCACACCTTCCCGG
CTGTCCTACAGTCCTCAGGACTCTACTCCCTCAGCAGCGTGGTGACCGTGC
CCTCCAGCAGCTTGGGCACCCAGACCTACATCTGCAACGTGAATCACAAGC
CCAGCAACACCAAGGTGGACAAGAAAGTTGAGCCCAAATCTTGTGACAAAA
CTCACACATGCCCACCGTGCCCAGCACCTGAACTCGCGGGGGCACCGTCA
GTCTTCCTCTTCCCCCCAAAACCCAAGGACACCCTCATGATCTCCCGGACC
CCTGAGGTCACATGCGTGGTGGTGGACGTGAGCCACGAAGACCCTGAGGT
CAAGTTCAACTGGTACGTGGACGGCGTGGAGGTGCATAATGCCAAGACAAA
GCCGCGGGAGGAGCAGTACAACAGCACGTACCGTGTGGTCAGCGTCCTCA
CCGTCCTGCACCAGGACTGGCTGAATGGCAAGGAGTACAAGTGCAAGGTCT
CCAACAAAGCCCTCCCAGCCCCCATCGAGAAAACCATCTCCAAAGCCAAAG
GGCAGCCCCGAGAACCACAGGTGTACACCCTGCCCCCATCCCGGGATGAG

CTGACCAAGAACCAGGTCAGCCTGACCTGCCTGGTCAAAGGCTTCTATCCC
AGCGACATCGCCGTGGAGTGGGAGAGCAATGGGCAGCCGGAGAACAACTA
CAAGACCACGCCTCCCGTGCTGGACTCCGACGGCTCCTTCTTCCTCTACAG
CAAGCTCACCGTGGACAAGAGCAGGTGGCAGCAGGGGAACGTCTTCTCAT
GCTCCGTGATGCATGAGGCTCTGCACAACCACTACACGCAGAAGAGCCTCT
CCCTGTCTCCGGGTAAATGA

SEQ ID 84: PN encoding 2A10 humanised light chain construct L11 SEQ ID: 90
ATGGGATGGAGCTGTATCATCCTCTTCTTGGTAGCAACAGCTACAGGTGTCC
ACTCCGATATTGTGATAACCCAGTCTCCACTCTCCCTGCCCGTCACCCTTGG
ACAGCCGGCCTCCATCTCCTGCAGGTCTAGTAAGAGTCTCCTATATAAGGAT
GGGAAGACATACTTGAATTGGTTTCAGCAGAGGCCAGGCCAATCTCCACAG
CTCCTAATTTATTTGATGTCCACCCGTGCATCTGGGGTCCCAGACAGATTCA
GCGGCGGTGGGTCAGGCACTGATTTCACACTGAAAATCAGCAGGGTGGAG
GCTGAGGATGTTGGGGTTTATTACTGCCAACAACTTGTAGAGTATCCGCTCA
CGTTTGGCCAGGGGACCAAGCTGGAGATCAAACGTACGGTGGCTGCACCA
TCTGTCTTCATCTTCCCGCCATCTGATGAGCAGTTGAAATCTGGAACTGCCT
CTGTTGTGTGCCTGCTGAATAACTTCTATCCCAGAGAGGCCAAAGTACAGTG
GAAGGTGGACAACGCCCTCCAATCGGGTAACTCCCAGGAGAGTGTCACAGA
GCAGGACAGCAAGGACAGCACCTACAGCCTCAGCAGCACCCTGACGCTGA
GCAAAGCAGACTACGAGAAACACAAAGTCTACGCCTGCGAAGTCACCCATC
AGGGCCTGAGCTCGCCCGTCACAAAGAGCTTCAACAGGGGAGAGTGTTAG

SEQ ID 85: 2A10 VH humanised construct H19
QVQLVQSGAEVKKPGASVKVSCKASGYTFTSYWMHWVRQRPGQGLEWIGNIN
PSNGGTNYNEKFKSRATMTRDTSTSTAYMELSSLRSEDTAVYYCELGQGYWG
QGTLVTVSS

SEQ ID 86: 2A10 VH humanised construct H20
QVQLVQSGAEVKKPGASVKVSCKASGYTFTSYWMHWVRQAPGQGLEWIGNIN
PSNGGTNYNEKFKSKATMTRDTSTSTAYMELSSLRSEDTAVYYCELGQGYWG
QGTLVTVSS

SEQ ID 87: 2A10 VH humanised construct H21
QVQLVQSGAEVKKPGASVKVSCKASGYTFTSYWMHWVRQRPGQGLEWIGNIN
PSNGGTNYNEKFKSKATMTRDTSTSTAYMELSSLRSEDTAVYYCELGQGYWG
QGTLVTVSS

SEQ ID 88: 2A10 VH humanised construct H22
QVQLVQSGAEVKKPGASVKVSCKASGYTFTSYWMHWVKQRPGQGLEWIGNIN
PSNGGTNYNEKFKSKATMTRDTSTSTAYMELSSLRSEDTAVYYCELGQGYWG
QGTLVTVSS

SEQ ID 89: 2A10 VH humanised construct H23

QVQLVQSGAEVKKPGASVKVSCKASGYTFTSYWMHWVKQRPGQGLEWIGNIN
PSNGGTNYNEKFKSKATMTVDTSTSTAYMELSSLRSEDTAVYYCELGQGYWG
QGTLVTVSS

SEQ ID 90: 2A10 VH humanised construct H24
QVQLVQSGAEVKKPGASVKVSCKASGYTFTSYWMHWVKQRPGQGLEWIGNIN
PSNGGTNYNEKFKSKATLTVDTSTSTAYMELSSLRSEDTAVYYCELGQGYWG
QGTLVTVSS

SEQ ID 91: 2A10 VH humanised construct H25
QVQLVQSGAEVKKPGASVKVSCKASGYTFTSYWMHWVRQRPGQGLEWMGNI
NPSNGGTNYNEKFKSRVTMTRDTSTSTAYMELSSLRSEDTAVYYCELGQGYW
GQGTLVTVSS

SEQ ID 92: 2A10 heavy chain humanised construct H19
MGWSCIILFLVATATGVHSQVQLVQSGAEVKKPGASVKVSCKASGYTFTSYWM
HWVRQRPGQGLEWIGNINPSNGGTNYNEKFKSRATMTRDTSTSTAYMELSSL
RSEDTAVYYCELGQGYWGQGTLVTVSSASTKGPSVFPLAPSSKSTSGGTAAL
GCLVKDYFPEPVTVSWNSGALTSGVHTFPAVLQSSGLYSLSSVVTVPSSSLGT
QTYICNVNHKPSNTKVDKKVEPKSCDKTHTCPPCPAPELAGAPSVFLFPPKPKD
TLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKL
TVDKSRWQQGNVFSCSVMHEALHNHYTQKSLSLSPGK

SEQ ID 93: 2A10 heavy chain humanised construct H20
MGWSCIILFLVATATGVHSQVQLVQSGAEVKKPGASVKVSCKASGYTFTSYWM
HWVRQAPGQGLEWIGNINPSNGGTNYNEKFKSKATMTRDTSTSTAYMELSSLR
SEDTAVYYCELGQGYWGQGTLVTVSSASTKGPSVFPLAPSSKSTSGGTAALG
CLVKDYFPEPVTVSWNSGALTSGVHTFPAVLQSSGLYSLSSVVTVPSSSLGTQ
TYICNVNHKPSNTKVDKKVEPKSCDKTHTCPPCPAPELAGAPSVFLFPPKPKDT
LMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLT
VDKSRWQQGNVFSCSVMHEALHNHYTQKSLSLSPGK

SEQ ID 94: 2A10 heavy chain humanised construct H21
MGWSCIILFLVATATGVHSQVQLVQSGAEVKKPGASVKVSCKASGYTFTSYWM
HWVRQRPGQGLEWIGNINPSNGGTNYNEKFKSKATMTRDTSTSTAYMELSSL
RSEDTAVYYCELGQGYWGQGTLVTVSSASTKGPSVFPLAPSSKSTSGGTAAL
GCLVKDYFPEPVTVSWNSGALTSGVHTFPAVLQSSGLYSLSSVVTVPSSSLGT
QTYICNVNHKPSNTKVDKKVEPKSCDKTHTCPPCPAPELAGAPSVFLFPPKPKD
TLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKL
TVDKSRWQQGNVFSCSVMHEALHNHYTQKSLSLSPGK

SEQ ID 95: 2A10 heavy chain humanised construct H22
MGWSCIILFLVATATGVHSQVQLVQSGAEVKKPGASVKVSCKASGYTFTSYWM
HWVKQRPGQGLEWIGNINPSNGGTNYNEKFKSKATMTRDTSTSTAYMELSSLR
SEDTAVYYCELGQGYWGQGTLVTVSSASTKGPSVFPLAPSSKSTSGGTAALG
CLVKDYFPEPVTVSWNSGALTSGVHTFPAVLQSSGLYSLSSVVTVPSSSLGTQ
TYICNVNHKPSNTKVDKKVEPKSCDKTHTCPPCPAPELAGAPSVFLFPPKPKDT
LMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLT
VDKSRWQQGNVFSCSVMHEALHNHYTQKSLSLSPGK

SEQ ID 96: 2A10 heavy chain humanised construct H23
MGWSCIILFLVATATGVHSQVQLVQSGAEVKKPGASVKVSCKASGYTFTSYWM
HWVKQRPGQGLEWIGNINPSNGGTNYNEKFKSKATMTVDTSTSTAYMELSSLR
SEDTAVYYCELGQGYWGQGTLVTVSSASTKGPSVFPLAPSSKSTSGGTAALG
CLVKDYFPEPVTVSWNSGALTSGVHTFPAVLQSSGLYSLSSVVTVPSSSLGTQ
TYICNVNHKPSNTKVDKKVEPKSCDKTHTCPPCPAPELAGAPSVFLFPPKPKDT
LMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLT
VDKSRWQQGNVFSCSVMHEALHNHYTQKSLSLSPGK

SEQ ID 97: 2A10 heavy chain humanised construct H24
MGWSCIILFLVATATGVHSQVQLVQSGAEVKKPGASVKVSCKASGYTFTSYWM
HWVKQRPGQGLEWIGNINPSNGGTNYNEKFKSKATLTVDTSTSTAYMELSSLR
SEDTAVYYCELGQGYWGQGTLVTVSSASTKGPSVFPLAPSSKSTSGGTAALG
CLVKDYFPEPVTVSWNSGALTSGVHTFPAVLQSSGLYSLSSVVTVPSSSLGTQ
TYICNVNHKPSNTKVDKKVEPKSCDKTHTCPPCPAPELAGAPSVFLFPPKPKDT
LMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLT
VDKSRWQQGNVFSCSVMHEALHNHYTQKSLSLSPGK

SEQ ID 98: 2A10 heavy chain humanised construct H25
MGWSCIILFLVATATGVHSQVQLVQSGAEVKKPGASVKVSCKASGYTFTSYWM
HWVRQRPGQGLEWMGNINPSNGGTNYNEKFKSRVTMTRDTSTSTAYMELSSL
RSEDTAVYYCELGQGYWGQGTLVTVSSASTKGPSVFPLAPSSKSTSGGTAAL
GCLVKDYFPEPVTVSWNSGALTSGVHTFPAVLQSSGLYSLSSVVTVPSSSLGT
QTYICNVNHKPSNTKVDKKVEPKSCDKTHTCPPCPAPELAGAPSVFLFPPKPKD
TLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKL
TVDKSRWQQGNVFSCSVMHEALHNHYTQKSLSLSPGK

SEQ ID 99: PN encoding 2A10 VH humanised construct H19 SEQ ID: 85

CAGGTGCAGCTGGTGCAGTCTGGGGCTGAGGTGAAGAAGCCTGGGGCCTC
AGTGAAGGTTTCCTGCAAGGCATCTGGATACACCTTCACCAGCTACTGGAT
GCACTGGGTGCGACAGAGGCCTGGACAAGGGCTTGAGTGGATCGGAAATA
TTAATCCTAGCAATGGTGGTACTAACTACAATGAGAAGTTCAAGAGCAGAGC
CACCATGACCAGGGACACGTCCACGAGCACAGCCTACATGGAGCTGAGCA
GCCTGAGATCTGAGGACACGGCCGTGTATTACTGTGAACTGGGACAGGGCT
ACTGGGGCCAGGGAACACTAGTCACAGTCTCCTCA

SEQ ID 100: PN encoding 2A10 VH humanised construct H20 SEQ ID: 86
CAGGTGCAGCTGGTGCAGTCTGGGGCTGAGGTGAAGAAGCCTGGGGCCTC
AGTGAAGGTTTCCTGCAAGGCATCTGGATACACCTTCACCAGCTACTGGAT
GCACTGGGTGCGACAGGCCCCTGGACAAGGGCTTGAGTGGATCGGAAATA
TTAATCCTAGCAATGGTGGTACTAACTACAATGAGAAGTTCAAGAGCAAGGC
CACCATGACCAGGGACACGTCCACGAGCACAGCCTACATGGAGCTGAGCA
GCCTGAGATCTGAGGACACGGCCGTGTATTACTGTGAACTGGGACAGGGCT
ACTGGGGCCAGGGAACACTAGTCACAGTCTCCTCA

SEQ ID 101: PN encoding 2A10 VH humanised construct H21 SEQ ID: 87
CAGGTGCAGCTGGTGCAGTCTGGGGCTGAGGTGAAGAAGCCTGGGGCCTC
AGTGAAGGTTTCCTGCAAGGCATCTGGATACACCTTCACCAGCTACTGGAT
GCACTGGGTGCGACAGAGGCCTGGACAAGGGCTTGAGTGGATCGGAAATA
TTAATCCTAGCAATGGTGGTACTAACTACAATGAGAAGTTCAAGAGCAAGGC
CACCATGACCAGGGACACGTCCACGAGCACAGCCTACATGGAGCTGAGCA
GCCTGAGATCTGAGGACACGGCCGTGTATTACTGTGAACTGGGACAGGGCT
ACTGGGGCCAGGGAACACTAGTCACAGTCTCCTCA

SEQ ID 102: PN encoding 2A10 VH humanised construct H22 SEQ ID: 88
CAGGTGCAGCTGGTGCAGTCTGGGGCTGAGGTGAAGAAGCCTGGGGCCTC
AGTGAAGGTTTCCTGCAAGGCATCTGGATACACCTTCACCAGCTACTGGAT
GCACTGGGTGAAGCAGAGGCCTGGACAAGGGCTTGAGTGGATCGGAAATA
TTAATCCTAGCAATGGTGGTACTAACTACAATGAGAAGTTCAAGAGCAAGGC
CACCATGACCAGGGACACGTCCACGAGCACAGCCTACATGGAGCTGAGCA
GCCTGAGATCTGAGGACACGGCCGTGTATTACTGTGAACTGGGACAGGGCT
ACTGGGGCCAGGGAACACTAGTCACAGTCTCCTCA

SEQ ID 103: PN encoding 2A10 VH humanised construct H23 SEQ ID: 89
CAGGTGCAGCTGGTGCAGTCTGGGGCTGAGGTGAAGAAGCCTGGGGCCTC
AGTGAAGGTTTCCTGCAAGGCATCTGGATACACCTTCACCAGCTACTGGAT
GCACTGGGTGAAGCAGAGGCCTGGACAAGGGCTTGAGTGGATCGGAAATA
TTAATCCTAGCAATGGTGGTACTAACTACAATGAGAAGTTCAAGAGCAAGGC
CACCATGACCGTAGACACGTCCACGAGCACAGCCTACATGGAGCTGAGCAG
CCTGAGATCTGAGGACACGGCCGTGTATTACTGTGAACTGGGACAGGGCTA
CTGGGGCCAGGGAACACTAGTCACAGTCTCCTCA

SEQ ID 104: PN encoding 2A10 VH humanised construct H24 SEQ ID: 90

CAGGTGCAGCTGGTGCAGTCTGGGGCTGAGGTGAAGAAGCCTGGGGCCTC
AGTGAAGGTTTCCTGCAAGGCATCTGGATACACCTTCACCAGCTACTGGAT
GCACTGGGTGAAGCAGAGGCCTGGACAAGGGCTTGAGTGGATCGGAAATA
TTAATCCTAGCAATGGTGGTACTAACTACAATGAGAAGTTCAAGAGCAAGGC
CACCCTGACCGTAGACACGTCCACGAGCACAGCCTACATGGAGCTGAGCA
GCCTGAGATCTGAGGACACGGCCGTGTATTACTGTGAACTGGGACAGGGCT
ACTGGGGCCAGGGAACACTAGTCACAGTCTCCTCA

SEQ ID 105: PN encoding 2A10 VH humanised construct H25 SEQ ID: 91
CAGGTGCAGCTGGTGCAGTCTGGGGCTGAGGTGAAGAAGCCTGGGGCCTC
AGTGAAGGTTTCCTGCAAGGCATCTGGATACACCTTCACCAGCTACTGGAT
GCACTGGGTGCGACAGAGGCCTGGACAAGGGCTTGAGTGGATGGGAAATA
TTAATCCTAGCAATGGTGGTACTAACTACAATGAGAAGTTCAAGAGCAGAGT
CACCATGACCAGGGACACGTCCACGAGCACAGCCTACATGGAGCTGAGCA
GCCTGAGATCTGAGGACACGGCCGTGTATTACTGTGAACTGGGACAGGGCT
ACTGGGGCCAGGGAACACTAGTCACAGTCTCCTCA

SEQ ID 106: PN encoding 2A10 heavy chain humanised construct H19 SEQ ID:
92
ATGGGATGGAGCTGTATCATCCTCTTCTTGGTAGCAACAGCTACAGGTGTCC
ACTCCCAGGTGCAGCTGGTGCAGTCTGGGGCTGAGGTGAAGAAGCCTGGG
GCCTCAGTGAAGGTTTCCTGCAAGGCATCTGGATACACCTTCACCAGCTACT
GGATGCACTGGGTGCGACAGAGGCCTGGACAAGGGCTTGAGTGGATCGGA
AATATTAATCCTAGCAATGGTGGTACTAACTACAATGAGAAGTTCAAGAGCA
GAGCCACCATGACCAGGGACACGTCCACGAGCACAGCCTACATGGAGCTG
AGCAGCCTGAGATCTGAGGACACGGCCGTGTATTACTGTGAACTGGGACAG
GGCTACTGGGGCCAGGGAACACTAGTCACAGTCTCCTCAGCCTCCACCAAG
GGCCCATCGGTCTTCCCCCTGGCACCCTCCTCCAAGAGCACCTCTGGGGG
CACAGCGGCCCTGGGCTGCCTGGTCAAGGACTACTTCCCCGAACCGGTGA
CGGTGTCGTGGAACTCAGGCGCCCTGACCAGCGGCGTGCACACCTTCCCG
GCTGTCCTACAGTCCTCAGGACTCTACTCCCTCAGCAGCGTGGTGACCGTG
CCCTCCAGCAGCTTGGGCACCCAGACCTACATCTGCAACGTGAATCACAAG
CCCAGCAACACCAAGGTGGACAAGAAAGTTGAGCCCAAATCTTGTGACAAA
ACTCACACATGCCCACCGTGCCCAGCACCTGAACTCGCGGGGGCACCGTC
AGTCTTCCTCTTCCCCCCAAAACCCAAGGACACCCTCATGATCTCCCGGAC
CCCTGAGGTCACATGCGTGGTGGTGGACGTGAGCCACGAAGACCCTGAGG
TCAAGTTCAACTGGTACGTGGACGGCGTGGAGGTGCATAATGCCAAGACAA
AGCCGCGGGAGGAGCAGTACAACAGCACGTACCGTGTGGTCAGCGTCCTC
ACCGTCCTGCACCAGGACTGGCTGAATGGCAAGGAGTACAAGTGCAAGGTC
TCCAACAAAGCCCTCCCAGCCCCCATCGAGAAAACCATCTCCAAAGCCAAA
GGGCAGCCCCGAGAACCACAGGTGTACACCCTGCCCCCATCCCGGGATGA
GCTGACCAAGAACCAGGTCAGCCTGACCTGCCTGGTCAAAGGCTTCTATCC
CAGCGACATCGCCGTGGAGTGGGAGAGCAATGGGCAGCCGGAGAACAACT
ACAAGACCACGCCTCCCGTGCTGGACTCCGACGGCTCCTTCTTCCTCTACA
GCAAGCTCACCGTGGACAAGAGCAGGTGGCAGCAGGGGAACGTCTTCTCA
TGCTCCGTGATGCATGAGGCTCTGCACAACCACTACACGCAGAAGAGCCTC
TCCCTGTCTCCGGGTAAATGA

SEQ ID 107: PN encoding 2A10 heavy chain humanised construct H20 SEQ ID:
93
ATGGGATGGAGCTGTATCATCCTCTTCTTGGTAGCAACAGCTACAGGTGTCC
ACTCCCAGGTGCAGCTGGTGCAGTCTGGGGCTGAGGTGAAGAAGCCTGGG
GCCTCAGTGAAGGTTTCCTGCAAGGCATCTGGATACACCTTCACCAGCTACT
GGATGCACTGGGTGCGACAGGCCCCTGGACAAGGGCTTGAGTGGATCGGA
AATATTAATCCTAGCAATGGTGGTACTAACTACAATGAGAAGTTCAAGAGCA
AGGCCACCATGACCAGGGACACGTCCACGAGCACAGCCTACATGGAGCTG
AGCAGCCTGAGATCTGAGGACACGGCCGTGTATTACTGTGAACTGGGACAG
GGCTACTGGGGCCAGGGAACACTAGTCACAGTCTCCTCAGCCTCCACCAAG
GGCCCATCGGTCTTCCCCCTGGCACCCTCCTCCAAGAGCACCTCTGGGGG
CACAGCGGCCCTGGGCTGCCTGGTCAAGGACTACTTCCCCGAACCGGTGA
CGGTGTCGTGGAACTCAGGCGCCCTGACCAGCGGCGTGCACACCTTCCCG
GCTGTCCTACAGTCCTCAGGACTCTACTCCCTCAGCAGCGTGGTGACCGTG
CCCTCCAGCAGCTTGGGCACCCAGACCTACATCTGCAACGTGAATCACAAG
CCCAGCAACACCAAGGTGGACAAGAAAGTTGAGCCCAAATCTTGTGACAAA
ACTCACACATGCCCACCGTGCCCAGCACCTGAACTCGCGGGGGCACCGTC
AGTCTTCCTCTTCCCCCCAAAACCCAAGGACACCCTCATGATCTCCCGGAC
CCCTGAGGTCACATGCGTGGTGGTGGACGTGAGCCACGAAGACCCTGAGG
TCAAGTTCAACTGGTACGTGGACGGCGTGGAGGTGCATAATGCCAAGACAA
AGCCGCGGGAGGAGCAGTACAACAGCACGTACCGTGTGGTCAGCGTCCTC
ACCGTCCTGCACCAGGACTGGCTGAATGGCAAGGAGTACAAGTGCAAGGTC
TCCAACAAAGCCCTCCCAGCCCCCATCGAGAAAACCATCTCCAAAGCCAAA
GGGCAGCCCCGAGAACCACAGGTGTACACCCTGCCCCCATCCCGGGATGA
GCTGACCAAGAACCAGGTCAGCCTGACCTGCCTGGTCAAAGGCTTCTATCC
CAGCGACATCGCCGTGGAGTGGGAGAGCAATGGGCAGCCGGAGAACAACT
ACAAGACCACGCCTCCCGTGCTGGACTCCGACGGCTCCTTCTTCCTCTACA
GCAAGCTCACCGTGGACAAGAGCAGGTGGCAGCAGGGGAACGTCTTCTCA
TGCTCCGTGATGCATGAGGCTCTGCACAACCACTACACGCAGAAGAGCCTC
TCCCTGTCTCCGGGTAAATGA

SEQ ID 108: PN encoding 2A10 heavy chain humanised construct H21 SEQ ID:
94
ATGGGATGGAGCTGTATCATCCTCTTCTTGGTAGCAACAGCTACAGGTGTCC
ACTCCCAGGTGCAGCTGGTGCAGTCTGGGGCTGAGGTGAAGAAGCCTGGG
GCCTCAGTGAAGGTTTCCTGCAAGGCATCTGGATACACCTTCACCAGCTACT
GGATGCACTGGGTGCGACAGAGGCCTGGACAAGGGCTTGAGTGGATCGGA
AATATTAATCCTAGCAATGGTGGTACTAACTACAATGAGAAGTTCAAGAGCA
AGGCCACCATGACCAGGGACACGTCCACGAGCACAGCCTACATGGAGCTG
AGCAGCCTGAGATCTGAGGACACGGCCGTGTATTACTGTGAACTGGGACAG
GGCTACTGGGGCCAGGGAACACTAGTCACAGTCTCCTCAGCCTCCACCAAG
GGCCCATCGGTCTTCCCCCTGGCACCCTCCTCCAAGAGCACCTCTGGGGG
CACAGCGGCCCTGGGCTGCCTGGTCAAGGACTACTTCCCCGAACCGGTGA
CGGTGTCGTGGAACTCAGGCGCCCTGACCAGCGGCGTGCACACCTTCCCG
GCTGTCCTACAGTCCTCAGGACTCTACTCCCTCAGCAGCGTGGTGACCGTG
CCCTCCAGCAGCTTGGGCACCCAGACCTACATCTGCAACGTGAATCACAAG

CCCAGCAACACCAAGGTGGACAAGAAAGTTGAGCCCAAATCTTGTGACAAA
ACTCACACATGCCCACCGTGCCCAGCACCTGAACTCGCGGGGGCACCGTC
AGTCTTCCTCTTCCCCCCAAAACCCAAGGACACCCTCATGATCTCCCGGAC
CCCTGAGGTCACATGCGTGGTGGTGGACGTGAGCCACGAAGACCCTGAGG
TCAAGTTCAACTGGTACGTGGACGGCGTGGAGGTGCATAATGCCAAGACAA
AGCCGCGGGAGGAGCAGTACAACAGCACGTACCGTGTGGTCAGCGTCCTC
ACCGTCCTGCACCAGGACTGGCTGAATGGCAAGGAGTACAAGTGCAAGGTC
TCCAACAAAGCCCTCCCAGCCCCCATCGAGAAAACCATCTCCAAAGCCAAA
GGGCAGCCCCGAGAACCACAGGTGTACACCCTGCCCCCATCCCGGGATGA
GCTGACCAAGAACCAGGTCAGCCTGACCTGCCTGGTCAAAGGCTTCTATCC
CAGCGACATCGCCGTGGAGTGGGAGAGCAATGGGCAGCCGGAGAACAACT
ACAAGACCACGCCTCCCGTGCTGGACTCCGACGGCTCCTTCTTCCTCTACA
GCAAGCTCACCGTGGACAAGAGCAGGTGGCAGCAGGGGAACGTCTTCTCA
TGCTCCGTGATGCATGAGGCTCTGCACAACCACTACACGCAGAAGAGCCTC
TCCCTGTCTCCGGGTAAATGA

SEQ ID 109: PN encoding 2A10 heavy chain humanised construct H22 SEQ ID:
95
ATGGGATGGAGCTGTATCATCCTCTTCTTGGTAGCAACAGCTACAGGTGTCC
ACTCCCAGGTGCAGCTGGTGCAGTCTGGGGCTGAGGTGAAGAAGCCTGGG
GCCTCAGTGAAGGTTTCCTGCAAGGCATCTGGATACACCTTCACCAGCTACT
GGATGCACTGGGTGAAGCAGAGGCCTGGACAAGGGCTTGAGTGGATCGGA
AATATTAATCCTAGCAATGGTGGTACTAACTACAATGAGAAGTTCAAGAGCA
AGGCCACCATGACCAGGGACACGTCCACGAGCACAGCCTACATGGAGCTG
AGCAGCCTGAGATCTGAGGACACGGCCGTGTATTACTGTGAACTGGGACAG
GGCTACTGGGGCCAGGGAACACTAGTCACAGTCTCCTCAGCCTCCACCAAG
GGCCCATCGGTCTTCCCCCTGGCACCCTCCTCCAAGAGCACCTCTGGGGG
CACAGCGGCCCTGGGCTGCCTGGTCAAGGACTACTTCCCCGAACCGGTGA
CGGTGTCGTGGAACTCAGGCGCCCTGACCAGCGGCGTGCACACCTTCCCG
GCTGTCCTACAGTCCTCAGGACTCTACTCCCTCAGCAGCGTGGTGACCGTG
CCCTCCAGCAGCTTGGGCACCCAGACCTACATCTGCAACGTGAATCACAAG
CCCAGCAACACCAAGGTGGACAAGAAAGTTGAGCCCAAATCTTGTGACAAA
ACTCACACATGCCCACCGTGCCCAGCACCTGAACTCGCGGGGGCACCGTC
AGTCTTCCTCTTCCCCCCAAAACCCAAGGACACCCTCATGATCTCCCGGAC
CCCTGAGGTCACATGCGTGGTGGTGGACGTGAGCCACGAAGACCCTGAGG
TCAAGTTCAACTGGTACGTGGACGGCGTGGAGGTGCATAATGCCAAGACAA
AGCCGCGGGAGGAGCAGTACAACAGCACGTACCGTGTGGTCAGCGTCCTC
ACCGTCCTGCACCAGGACTGGCTGAATGGCAAGGAGTACAAGTGCAAGGTC
TCCAACAAAGCCCTCCCAGCCCCCATCGAGAAAACCATCTCCAAAGCCAAA
GGGCAGCCCCGAGAACCACAGGTGTACACCCTGCCCCCATCCCGGGATGA
GCTGACCAAGAACCAGGTCAGCCTGACCTGCCTGGTCAAAGGCTTCTATCC
CAGCGACATCGCCGTGGAGTGGGAGAGCAATGGGCAGCCGGAGAACAACT
ACAAGACCACGCCTCCCGTGCTGGACTCCGACGGCTCCTTCTTCCTCTACA
GCAAGCTCACCGTGGACAAGAGCAGGTGGCAGCAGGGGAACGTCTTCTCA
TGCTCCGTGATGCATGAGGCTCTGCACAACCACTACACGCAGAAGAGCCTC
TCCCTGTCTCCGGGTAAATGA

SEQ ID 110: PN encoding 2A10 heavy chain humanised construct H23 SEQ ID:
96
ATGGGATGGAGCTGTATCATCCTCTTCTTGGTAGCAACAGCTACAGGTGTCC
ACTCCCAGGTGCAGCTGGTGCAGTCTGGGGCTGAGGTGAAGAAGCCTGGG
GCCTCAGTGAAGGTTTCCTGCAAGGCATCTGGATACACCTTCACCAGCTACT
GGATGCACTGGGTGAAGCAGAGGCCTGGACAAGGGCTTGAGTGGATCGGA
AATATTAATCCTAGCAATGGTGGTACTAACTACAATGAGAAGTTCAAGAGCA
AGGCCACCATGACCGTAGACACGTCCACGAGCACAGCCTACATGGAGCTGA
GCAGCCTGAGATCTGAGGACACGGCCGTGTATTACTGTGAACTGGGACAGG
GCTACTGGGGCCAGGGAACACTAGTCACAGTCTCCTCAGCCTCCACCAAGG
GCCCATCGGTCTTCCCCCTGGCACCCTCCTCCAAGAGCACCTCTGGGGGC
ACAGCGGCCCTGGGCTGCCTGGTCAAGGACTACTTCCCCGAACCGGTGAC
GGTGTCGTGGAACTCAGGCGCCCTGACCAGCGGCGTGCACACCTTCCCGG
CTGTCCTACAGTCCTCAGGACTCTACTCCCTCAGCAGCGTGGTGACCGTGC
CCTCCAGCAGCTTGGGCACCCAGACCTACATCTGCAACGTGAATCACAAGC
CCAGCAACACCAAGGTGGACAAGAAAGTTGAGCCCAAATCTTGTGACAAAA
CTCACACATGCCCACCGTGCCCAGCACCTGAACTCGCGGGGGCACCGTCA
GTCTTCCTCTTCCCCCCAAAACCCAAGGACACCCTCATGATCTCCCGGACC
CCTGAGGTCACATGCGTGGTGGTGGACGTGAGCCACGAAGACCCTGAGGT
CAAGTTCAACTGGTACGTGGACGGCGTGGAGGTGCATAATGCCAAGACAAA
GCCGCGGGAGGAGCAGTACAACAGCACGTACCGTGTGGTCAGCGTCCTCA
CCGTCCTGCACCAGGACTGGCTGAATGGCAAGGAGTACAAGTGCAAGGTCT
CCAACAAAGCCCTCCCAGCCCCCATCGAGAAAACCATCTCCAAAGCCAAAG
GGCAGCCCCGAGAACCACAGGTGTACACCCTGCCCCCATCCCGGGATGAG
CTGACCAAGAACCAGGTCAGCCTGACCTGCCTGGTCAAAGGCTTCTATCCC
AGCGACATCGCCGTGGAGTGGGAGAGCAATGGGCAGCCGGAGAACAACTA
CAAGACCACGCCTCCCGTGCTGGACTCCGACGGCTCCTTCTTCCTCTACAG
CAAGCTCACCGTGGACAAGAGCAGGTGGCAGCAGGGGAACGTCTTCTCAT
GCTCCGTGATGCATGAGGCTCTGCACAACCACTACACGCAGAAGAGCCTCT
CCCTGTCTCCGGGTAAATGA

SEQ ID 111: PN encoding 2A10 heavy chain humanised construct H24 SEQ ID:
97
ATGGGATGGAGCTGTATCATCCTCTTCTTGGTAGCAACAGCTACAGGTGTCC
ACTCCCAGGTGCAGCTGGTGCAGTCTGGGGCTGAGGTGAAGAAGCCTGGG
GCCTCAGTGAAGGTTTCCTGCAAGGCATCTGGATACACCTTCACCAGCTACT
GGATGCACTGGGTGAAGCAGAGGCCTGGACAAGGGCTTGAGTGGATCGGA
AATATTAATCCTAGCAATGGTGGTACTAACTACAATGAGAAGTTCAAGAGCA
AGGCCACCCTGACCGTAGACACGTCCACGAGCACAGCCTACATGGAGCTG
AGCAGCCTGAGATCTGAGGACACGGCCGTGTATTACTGTGAACTGGGACAG
GGCTACTGGGGCCAGGGAACACTAGTCACAGTCTCCTCAGCCTCCACCAAG
GGCCCATCGGTCTTCCCCCTGGCACCCTCCTCCAAGAGCACCTCTGGGGG
CACAGCGGCCCTGGGCTGCCTGGTCAAGGACTACTTCCCCGAACCGGTGA
CGGTGTCGTGGAACTCAGGCGCCCTGACCAGCGGCGTGCACACCTTCCCG
GCTGTCCTACAGTCCTCAGGACTCTACTCCCTCAGCAGCGTGGTGACCGTG
CCCTCCAGCAGCTTGGGCACCCAGACCTACATCTGCAACGTGAATCACAAG
CCCAGCAACACCAAGGTGGACAAGAAAGTTGAGCCCAAATCTTGTGACAAA

ACTCACACATGCCCACCGTGCCCAGCACCTGAACTCGCGGGGGCACCGTC
AGTCTTCCTCTTCCCCCCAAAACCCAAGGACACCCTCATGATCTCCCGGAC
CCCTGAGGTCACATGCGTGGTGGTGGACGTGAGCCACGAAGACCCTGAGG
TCAAGTTCAACTGGTACGTGGACGGCGTGGAGGTGCATAATGCCAAGACAA
AGCCGCGGGAGGAGCAGTACAACAGCACGTACCGTGTGGTCAGCGTCCTC
ACCGTCCTGCACCAGGACTGGCTGAATGGCAAGGAGTACAAGTGCAAGGTC
TCCAACAAAGCCCTCCCAGCCCCCATCGAGAAAACCATCTCCAAAGCCAAA
GGGCAGCCCCGAGAACCACAGGTGTACACCCTGCCCCCATCCCGGGATGA
GCTGACCAAGAACCAGGTCAGCCTGACCTGCCTGGTCAAAGGCTTCTATCC
CAGCGACATCGCCGTGGAGTGGGAGAGCAATGGGCAGCCGGAGAACAACT
ACAAGACCACGCCTCCCGTGCTGGACTCCGACGGCTCCTTCTTCCTCTACA
GCAAGCTCACCGTGGACAAGAGCAGGTGGCAGCAGGGGAACGTCTTCTCA
TGCTCCGTGATGCATGAGGCTCTGCACAACCACTACACGCAGAAGAGCCTC
TCCCTGTCTCCGGGTAAATGA

SEQ ID 112: PN encoding 2A10 heavy chain humanised construct H25 SEQ ID:
98
ATGGGATGGAGCTGTATCATCCTCTTCTTGGTAGCAACAGCTACAGGTGTCC
ACTCCCAGGTGCAGCTGGTGCAGTCTGGGGCTGAGGTGAAGAAGCCTGGG
GCCTCAGTGAAGGTTTCCTGCAAGGCATCTGGATACACCTTCACCAGCTACT
GGATGCACTGGGTGCGACAGAGGCCTGGACAAGGGCTTGAGTGGATGGGA
AATATTAATCCTAGCAATGGTGGTACTAACTACAATGAGAAGTTCAAGAGCA
GAGTCACCATGACCAGGGACACGTCCACGAGCACAGCCTACATGGAGCTG
AGCAGCCTGAGATCTGAGGACACGGCCGTGTATTACTGTGAACTGGGACAG
GGCTACTGGGGCCAGGGAACACTAGTCACAGTCTCCTCAGCCTCCACCAAG
GGCCCATCGGTCTTCCCCCTGGCACCCTCCTCCAAGAGCACCTCTGGGGG
CACAGCGGCCCTGGGCTGCCTGGTCAAGGACTACTTCCCCGAACCGGTGA
CGGTGTCGTGGAACTCAGGCGCCCTGACCAGCGGCGTGCACACCTTCCCG
GCTGTCCTACAGTCCTCAGGACTCTACTCCCTCAGCAGCGTGGTGACCGTG
CCCTCCAGCAGCTTGGGCACCCAGACCTACATCTGCAACGTGAATCACAAG
CCCAGCAACACCAAGGTGGACAAGAAAGTTGAGCCCAAATCTTGTGACAAA
ACTCACACATGCCCACCGTGCCCAGCACCTGAACTCGCGGGGGCACCGTC
AGTCTTCCTCTTCCCCCCAAAACCCAAGGACACCCTCATGATCTCCCGGAC
CCCTGAGGTCACATGCGTGGTGGTGGACGTGAGCCACGAAGACCCTGAGG
TCAAGTTCAACTGGTACGTGGACGGCGTGGAGGTGCATAATGCCAAGACAA
AGCCGCGGGAGGAGCAGTACAACAGCACGTACCGTGTGGTCAGCGTCCTC
ACCGTCCTGCACCAGGACTGGCTGAATGGCAAGGAGTACAAGTGCAAGGTC
TCCAACAAAGCCCTCCCAGCCCCCATCGAGAAAACCATCTCCAAAGCCAAA
GGGCAGCCCCGAGAACCACAGGTGTACACCCTGCCCCCATCCCGGGATGA
GCTGACCAAGAACCAGGTCAGCCTGACCTGCCTGGTCAAAGGCTTCTATCC
CAGCGACATCGCCGTGGAGTGGGAGAGCAATGGGCAGCCGGAGAACAACT
ACAAGACCACGCCTCCCGTGCTGGACTCCGACGGCTCCTTCTTCCTCTACA
GCAAGCTCACCGTGGACAAGAGCAGGTGGCAGCAGGGGAACGTCTTCTCA
TGCTCCGTGATGCATGAGGCTCTGCACAACCACTACACGCAGAAGAGCCTC
TCCCTGTCTCCGGGTAAATGA

SEQ ID NO.113 – Amino acid sequence of Marmoset NOGO-A fragment
VQDSLCPVAQLCPSFEESEATPSPVLPDIVMEAPLNSAVPSAGASAVQPSSSPL
EASSVNFESVKHEPENPPPYEEAMNVSRKKVSGIKEEIKEPESINAAVQETEAP
YISIACDLIKETKLSAEPTPDFSSYSEMAKVEQPLPDHSELVEDSSPDSEPVDLF
SDDSIPDVPQKQDEAVILVKETLTETSFESMIEHENK

## Claims

1. A humanised monoclonal antibody which binds to and neutralises human NOGO, the antibody comprising a heavy chain variable region having an amino acid sequence of SEQ ID NO:86, SEQ ID NO:88, SEQ ID NO:89 or SEQ ID NO:90, and a light chain variable region having an amino acid sequence of SEQ ID NO:23 or SEQ ID NO:25, or a variant thereof comprising an analog of a complementarity determining region (CDR) of said antibody, wherein said variant retains the binding specificity and neutralising ability of the antibody from which it was derived.

2. A monoclonal antibody according to claim 1, comprising VH and VL regions selected from the following list: H20L16 (SEQ ID 86 + SEQ ID 23), H22L16 (SEQ ID 88 + SEQ ID 23), H23L16 (SEQ ID 89 + SEQ ID 23), H24L16 (SEQ ID 90 + SEQ ID 23), H20L18 (SEQ ID 86 + SEQ ID 25), H22L18 (SEQ ID 88 + SEQ ID 25), H23L18 (SEQ ID 89 + SEQ ID 25), and H24L18 (SEQ ID 90 + SEQ ID 25).

3. A host cell co-transfected with first and second vectors encoding heavy and light chains, respectively, of an antibody according to claim 1 or 2.

4. A host cell transfected with a vector encoding heavy and light chains of an antibody according to claim 1 or 2.

5. A method of producing an antibody according to claim 1 or 2, comprising the step of culturing a host cell according to claim 3 or 4, and recovering the antibody thereby produced.

6. An antibody according to claim 1 or 2 produced by the method of claim 5.

7. A pharmaceutical composition comprising an anti-NOGO antibody or functional fragment thereof according to claim 1, 2 or 6, together with a pharmaceutically acceptable diluent or carrier.

8. An antibody according to claim 1, 2 or 6 for use in the treatment or prophylaxis of a human patient.

## Patentansprüche

1. Humanisierter monoklonaler Antikörper, welcher an menschliches NOGO bindet und es neutralisiert, der Antikörper umfasst eine variable Region der schweren Kette mit einer Aminosäuresequenz der SEQ ID NO: 86, SEQ ID NO: 88, SEQ ID NO: 89 oder SEQ ID NO: 90 und eine variable Region der leichten Kette mit einer Aminosäuresequenz der SEQ ID NO: 23 oder SEQ ID NO: 25 oder eine Variante davon, die ein Ananloges einer Komplementaritäts-bestimmenden Region (CDR) des Antikörpers umfasst, wobei die Variante die Bindungsspezifität und neutralisierende Fähigkeit des Antikörpers behält, von dem sie abgeleitet wurde.

2. Monoklonaler Antikörper gemäss Anspruch 1, umfassend VH- und VL-Regionen, die aus der folgenden Liste ausgewählt sind: H20L16 (SEQ ID NO: 86 + SEQ ID NO: 23), H22L16 (SEQ ID NO: 88 + SEQ ID NO: 23), H23L16 (SEQ ID NO: 89 + SEQ ID NO: 23), H24L16 (SEQ ID NO: 90 + SEQ ID NO: 23), H20L18 (SEQ ID NO: 86 + SEQ ID NO: 25), H22L18 (SEQ ID NO: 88 + SEQ ID NO: 25), H23L18 (SEQ ID NO: 89 + SEQ ID NO: 25) und H24L18 (SEQ ID NO: 90 + SEQ ID NO: 25).

3. Wirtszelle, co-transfiziert mit ersten und zweiten Vektoren, die schwere bzw. leichte Ketten eines Antikörpers gemäss Anspruch 1 oder 2 codieren.

4. Wirtszelle, transfiziert mit einem Vektor, der schwere und leichte Ketten eines Antikörpers gemäss Anspruch 1 oder 2 codiert.

5. Verfahren zur Herstellung eines Antikörpers gemäss Anspruch 1 oder 2, umfassend den Schritt der Kultivierung einer Wirtszelle gemäss Anspruch 3 oder 4 und die Gewinnung des dadurch hergestellten Antikörpers.

6. Antikörper gemäss Anspruch 1 oder 2, hergestellt durch das Verfahren gemäss Anspruch 5.

7. Pharmazeutische Zusammensetzung, umfassend einen Anti-NOGO-Antikörper oder ein funktionsfähiges Fragment davon gemäss Anspruch 1, 2 oder 6 zusammen mit einem pharmazeutisch annehmbaren Verdünnungsmittel oder Träger.

8. Antikörper gemäss Anspruch 1, 2 oder 6 zur Verwendung in der Behandlung oder Prophylaxe eines menschlichen Patienten.

## Revendications

1. Anticorps monoclonal humanisé qui se lie au NOGO humain et le neutralise, l'anticorps comprenant une zone variable de chaîne lourde ayant une séquence d'acide aminé de SEQ. ID N°86, SEQ. ID N°88, SEQ. ID N°89 ou SEQ. ID N°90, et une zone variable de chaîne légère, ayant une séquence d'acide aminé de SEQ. ID N°23 ou SEQ. ID N°25, ou une variante correspondante comprenant un analogue d'une zone de détermination de complémentarité (CDR) dudit anticorps, dans lequel ladite variante maintient la spécificité de liaison et la capacité de neutralisation de l'anticorps dont elle découle.

2. Anticorps monoclonal selon la revendication 1, comprenant des zones VH et VL choisies dans la liste suivante : H20L16 (SEQ. ID 86 + SEQ. ID 23), H22L16 (SEQ. ID 88 + SEQ. ID 23), H23L16 (SEQ. ID 89 + SEQ. ID 23), H24L16 (SEQ. ID 90 + SEQ. ID 23), H20L18 (SEQ. ID 86 + SEQ. ID 25), H22L18 (SEQ.ID 88 + SEQ. ID 25), H23L18 (SEQ. ID 89 + SEQ. ID 25) et H24L18 (SEQ. ID 90 + SEQ.ID25).

3. Cellule hôte co-transfectée avec un premier et un second vecteurs codant les chaînes lourde et légère, respectivement, d'un anticorps selon la revendication 1 ou 2.

4. Cellule hôte transfectée avec un vecteur codant les chaînes lourde et légère d'un anticorps selon la revendication 1 ou 2.

5. Procédé de production d'un anticorps selon la revendication 1 ou 2, comprenant l'étape consistant à cultiver une cellule hôte selon la revendication 3 ou 4, et à récupérer l'anticorps ainsi produit.

6. Anticorps selon la revendication 1 ou 2 produit par le procédé selon la revendication 5.

7. Composition pharmaceutique comprenant un anticorps anti-NOGO ou un fragment fonctionnel correspondant, selon les revendications 1, 2 ou 6 avec un diluant ou excipient pharmaceutiquement acceptable.

8. Anticorps selon la revendication 1, 2 ou 6 à utiliser dans le traitement ou la prophylaxie d'un patient humain.
